# EUROPEAN PATENT APPLICATION

(11) **EP 4 375 750 A1**
(43) Date of publication of application: **29.05.2024**
(21) Application number: 22845862.6
(22) Date of filing: 15.07.2022
(51) Int. Cl.: G03F 7/11, C07D 487/04, C08G 61/12

(54) **FILM-FORMING MATERIAL FOR SEMICONDUCTOR, MEMBER-FORMING MATERIAL FOR SEMICONDUCTOR, PROCESS MEMBER-FORMING MATERIAL FOR SEMICONDUCTOR, UNDERLAYER FILM-FORMING MATERIAL, UNDERLAYER FILM, AND SEMICONDUCTOR DEVICE**

(30) Priority: 20.07.2021 JP 2021119825
(71) Applicant: ADEKA CORPORATION, Arakawa-ku Tokyo 116-8554 (JP)
(72) Inventor: SAITO, Hiromasa, Tokyo 116-8554 (JP); MURAMATSU, Yousuke, Tokyo 116-8554 (JP); SUZUKI, Mizuki, Tokyo 116-8554 (JP); IRISAWA, Masatomi, Tokyo 116-8554 (JP)
(74) Representative: Forstmeyer, Dietmar
(86) International application number: PCT/JP2022/027788
(87) International publication number: WO 2023/002928

(57) **Abstract**

An objective of the invention is to provide a material for forming a film for a semiconductor, the material being capable of providing a film having excellent heat resistance and solvent resistance. The invention relates to a material for forming a film for a semiconductor, the material containing: a compound represented by general formula (I) below and having in its molecule at least one reactive group, or a polymer including, as a monomer, a compound represented by general formula (I) below and having in its molecule at least one reactive group; and a solvent. In the formula, A represents a hydrocarbon ring having 6 carbon atoms, X¹ and X² each represent, for example, an aryl group having 6 to 30 carbon atoms and optionally substituted by a reactive group or a group having a reactive group, etc., R¹, R², R³, R⁴, R⁶, R⁷, R⁸, and R⁹ each represent, for example, a hydrogen atom, a hydrocarbon group having 1 to 20 carbon atoms and optionally substituted by a reactive group or a group having a reactive group, etc., and R⁵ and R¹⁰ each represent, for example, a hydrogen atom, a hydrocarbon group having 1 to 20 carbon atoms and optionally substituted by a reactive group, etc.

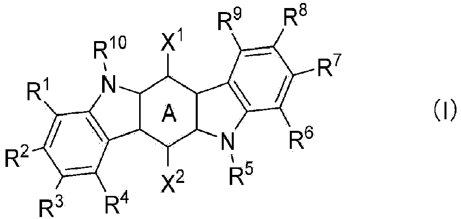

## Description

### Technical Field

The present invention relates to a material for forming a film for a semiconductor, the material containing a compound having a specific backbone.

### Background Art

In recent years, integrated circuits have achieved greater capacity, higher levels of integration and higher speed. Accordingly, further enhancements in properties, such as high heat resistance and high durability, are demanded of materials used for forming members for semiconductors, such as encapsulants, insulating films, multilayer substrate materials, barrier films, insulating films for through-silicon vias, etc. Patent Literature 1 discloses an oxazine compound having an aromatic ring structure and a plurality of specific carbon-carbon triple bond structures, as a compound having excellent heat resistance and low thermal expansivity, as well as tight adherence, excellent moisture resistance and solder resistance.

Further, semiconductor patterns are becoming even finer, and this is leading to further reduction in the thickness of photoresist layers in view of such issues as rectangular-shape retainability of photoresist patterns and collapse avoidance. However, reduction in film thickness may lead to insufficient etching resistance of photoresist patterns, which makes it difficult to sufficiently etch substrates being processed. Thus, with the aim of obtaining a favorable pattern shape, a multilayer resist material constituted by, for example, a photoresist, an intermediate film, and an underlayer film is often used in a semiconductor-manufacturing process.

Below is an example of an overview of a semiconductor-manufacturing process using a multilayer resist material.

A typical example employing a multilayer resist material is a three-layer resist method involving two layers below a photoresist layer. More specifically, the method is as follows.

First, an underlayer film-forming material is applied to a substrate to be processed, such as a silicon wafer, and an underlayer film is formed by heating. An intermediate film is formed on the underlayer film, and then, a photoresist is applied thereto, which is then subjected to exposure and development, to form a pattern. The formed pattern is employed as a mask, and under appropriate dry-etching conditions, etching is conducted in the order of the intermediate film and the underlayer film. Then, the obtained underlayer film pattern is employed as a mask, and under appropriate dry-etching conditions, the substrate to be processed is subjected to etching. The remaining mask is subjected to ashing, to obtain a substrate having the intended structure.

The underlayer film-forming material needs to have heat resistance and solvent resistance such that it does not deform due to heat or solvent at the time of forming the intermediate film and the photoresist layer, and it also needs to have etching resistance such that transferring by etching can be conducted accurately. Further, the material needs to have embeddability with respect to the substrate's projections and depressions and also have planarizing properties so that film formation is conducted in a planarized manner.

Known examples of underlayer film materials include vinyl derivatives having an aromatic hydrocarbon ring (Patent Literature 2) and aromatic compounds having a carbon-carbon triple bond structure (Patent Literature 3).

### Citation List

### Patent Literature

Patent Literature 1: JP 2018-002612A
Patent Literature 2: JP 2018-140972A
Patent Literature 3: US 2020/0142309A1

### Summary of Invention

### Technical Problem

An objective of the present invention is to provide a material for forming a film for semiconductors which is capable of providing a film having excellent heat resistance and solvent resistance.

### Solution to Problem

As a result of diligent research, Inventors have found that a material for forming a film for semiconductors, which contains a solvent and a compound having a specific backbone and having at least one reactive group in its molecule, or a polymer including the compound as a monomer, is capable of providing a film having excellent heat resistance and solvent resistance, thus accomplishing the present invention.

That is, the present invention relates to a material for forming a film for a semiconductor, the material containing: a compound represented by general formula (I) below and having in its molecule at least one reactive group (also referred to hereinafter as "Compound (I)") or a polymer including Compound (I) as a monomer (also referred to hereinafter as "Polymer (I)"); and a solvent. 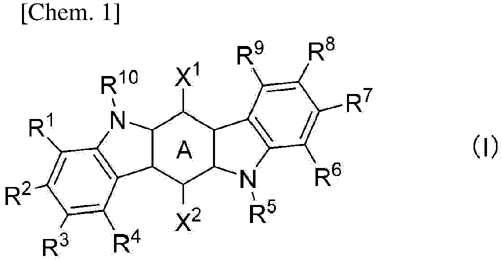
wherein, in the formula, A represents a hydrocarbon ring having 6 carbon atoms;
X¹ and X² each independently represent an aryl group having 6 to 30 carbon atoms and optionally substituted by a reactive group or a group having a reactive group, a heterocyclic group having 2 to 30 carbon atoms and optionally substituted by a reactive group or a group having a reactive group, or a heterocycle-containing group having 3 to 30 carbon atoms and optionally substituted by a reactive group or a group having a reactive group;
R¹, R², R³, R⁴, R⁶, R⁷, R⁸, and R⁹ each independently represent a hydrogen atom, a halogen atom, a reactive group, a nitro group, a hydrocarbon group having 1 to 20 carbon atoms and optionally substituted by a reactive group, a heterocyclic group having 2 to 10 carbon atoms and optionally substituted by a reactive group, a heterocycle-containing group having 3 to 30 carbon atoms and optionally substituted by a reactive group, a group in which at least one methylene group in the aforementioned hydrocarbon group having 1 to 20 carbon atoms has been substituted by a divalent group selected from {Group A} below, or a group in which at least one methylene group in the aforementioned heterocycle-containing group having 3 to 30 carbon atoms has been substituted by a divalent group selected from {Group A} below; and
R⁵ and R¹⁰ each independently represent a hydrogen atom, a hydrocarbon group having 1 to 20 carbon atoms and optionally substituted by a reactive group, a heterocyclic group having 2 to 10 carbon atoms and optionally substituted by a reactive group, a heterocycle-containing group having 3 to 30 carbon atoms and optionally substituted by a reactive group, a group in which at least one methylene group in the aforementioned hydrocarbon group having 1 to 20 carbon atoms has been substituted by a divalent group selected from {Group A} below, or a group in which at least one methylene group in the aforementioned heterocycle-containing group having 3 to 30 carbon atoms has been substituted by a divalent group selected from {Group A} below:

   {Group A}: -O-, -CO-, -COO-, -OCO-, -NR¹¹-, -NR¹²CO-, -S-,
wherein R¹¹ and R¹² each independently represent a hydrogen atom or a hydrocarbon group having 1 to 20 carbon atoms.

### Advantageous Effects of Invention

A material for forming a film for semiconductors according to the present invention is capable of providing a film for semiconductors that has excellent heat resistance and solvent resistance.

A "material for forming a film for a semiconductor" is a material necessary for forming a film for a semiconductor, and includes: a "material for forming a member for a semiconductor", which is necessary for forming a member for a semiconductor; and a "material for forming a member for a semiconductor process", which is necessary for forming a member for a semiconductor process.

The aforementioned "material for forming a member for a semiconductor" encompasses materials suitable for various uses, with examples including insulating film forming materials, barrier film forming materials, encapsulant forming materials, gap filler forming materials, mounting films, mounting adhesives, circuit connection materials, etc.

The aforementioned "material for forming a member for a semiconductor process" encompasses materials suitable for various uses, with examples including underlayer film-forming materials, photoresist forming materials, antireflective film forming materials, intermediate film forming materials, etc.

The aforementioned "film for a semiconductor" refers to a film to be used for manufacturing a semiconductor, and more specifically, is a collective term encompassing a solid layer obtained by removing the solvent by distillation after application of the material for forming a film for a semiconductor, as well as a cured product obtained by curing the solid layer by a polymerization reaction etc.

The aforementioned "film for a semiconductor" includes a "member for a semiconductor" and a "member for a semiconductor process". A "member for a semiconductor" refers to a member that remains on a semiconductor device as a permanent film. A "member for a semiconductor process" refers to a member that is used as a sacrificial film in a process for manufacturing a semiconductor, but does not remain on a semiconductor device.

Examples of the aforementioned "member for a semiconductor" may include insulating films, barrier films, encapsulants, gap fillers, mounting films, mounting adhesives, circuit connection materials, etc.

Examples of the aforementioned "member for a semiconductor process" may include photoresists, intermediate films, underlayer films, antireflective films, etc.

A photoresist, an intermediate film, and an underlayer film are a "member for a semiconductor process" used with an objective to obtain a favorable pattern, and can be used as a multilayer resist in which an underlayer film, an intermediate film, and a photoresist are layered in this order on a substrate to be processed, such as a silicon wafer.

The aforementioned "semiconductor device" refers to an electronic component including a semiconductor, with examples including: discrete components (individual semiconductors) such as transistors, diodes, etc., wherein a single element has a single function; integrated circuits (ICs) wherein elements with a plurality of functions are mounted on a single chip; CPUs, including memories, microprocessors (MPUs), logic ICs, etc.; and the like.

### Description of Embodiments

The aforementioned Compound (I) to be used in the present invention is a compound having a specific backbone, and has at least one reactive group in its molecule.

The "reactive group" in the Compound (I) refers to a group having a property capable of forming a covalent bond with another reactive group. Herein, "another reactive group" may be the same type of reactive group or a different type of reactive group. In the present invention, "reactive group" refers to a carbon-carbon double bond, a carbon-carbon triple bond, a nitrile group, an epoxy group, an isocyanate group, a hydroxy group, an amino group, and a thiol group. Stated differently, the reactive group in the general formula (I) is a vinyl group, an ethynyl group, a nitrile group, an epoxy group, an isocyanate group, a hydroxy group, an amino group, or a thiol group. Note that the nitrile group is included as a reactive group because it is capable of forming a triazine ring through a trimerization reaction. As for the reactive group, a carbon-carbon triple bond, a carbon-carbon double bond, a hydroxy group, and an epoxy group are preferred; a carbon-carbon triple bond, a carbon-carbon double bond, and a phenolic hydroxy group are more preferred; and a carbon-carbon triple bond and a phenolic hydroxy group are particularly preferred. This is because, by employing the aforementioned group(s) as the reactive group, the obtained film will have excellent heat resistance.

A "group having a reactive group" in the general formula (I) refers to a group in which at least one hydrogen atom of a group adoptable in general formula (I) has been substituted by the aforementioned reactive group, and examples may include: alkenyl groups having 2 to 10 carbon atoms, such as an allyl group, etc.; alkenyloxy groups having 2 to 10 carbon atoms, such as a vinyloxy group, an allyloxy group, etc.; alkynyl groups having 2 to 10 carbon atoms, such as a propargyl group, etc.; alkynyloxy groups having 2 to 10 carbon atoms, such as a propargyloxy group, etc.; an acryloyl group, a methacryloyl group, a glycidyl group, a glycidyloxy group, an oxetanyl group, etc.

Examples of the hydrocarbon ring having 6 carbon atoms represented by A in the general formula (I) may include a benzene ring, a cyclohexadiene ring, a cyclohexene ring, and a cyclohexane ring.

The aryl group having 6 to 30 carbon atoms, as represented by X¹ and X² in the general formula (I), may have a monocyclic structure or a fused-ring structure, or may be formed by linking two aromatic hydrocarbon rings. Examples of fused-ring-structure aryl groups having 6 to 30 carbon atoms may include hydrocarbon-type aromatic fused ring groups having 7 to 30 carbon atoms, which are structured by fusing two or more aromatic hydrocarbon rings.

Examples of monocyclic-structure aryl groups having 6 to 30 carbon atoms may include phenyl, tolyl, xylyl, ethylphenyl, 2,4,6-trimethylphenyl, etc. Examples of hydrocarbon-type aromatic fused ring groups having 7 to 30 carbon atoms may include naphthyl, anthracenyl, phenanthryl, pyrenyl, fluorenyl, indenofluorenyl, etc.

Aryl groups formed by linking two aromatic hydrocarbon rings may be obtained by linking two monocyclic-structure aromatic hydrocarbon rings, or may be obtained by linking a monocyclic-structure aromatic hydrocarbon ring and a fused-ring-structure aromatic hydrocarbon ring, or may be obtained by linking a fused-ring-structure aromatic hydrocarbon ring and a fused-ring-structure aromatic hydrocarbon ring.

Examples of linking groups for linking two aromatic hydrocarbon rings may include a single bond, a sulfide group (-S-), a carbonyl group, etc.

Examples of aryl groups formed by linking two monocyclic-structure aromatic hydrocarbon rings may include biphenyl, diphenyl sulfide, benzoylphenyl, etc.

The aryl group having 6 to 30 carbon atoms may be optionally substituted by a reactive group or a group having a reactive group. An "aryl group having 6 to 30 carbon atoms and substituted by a reactive group or a group having a reactive group" refers to a group having 6 to 30 carbon atoms wherein at least one hydrogen atom in the aryl group has been substituted by a reactive group or a group having a reactive group. The aryl group having 6 to 30 carbon atoms may also have other substituent(s). Examples of other substituents may include a halogen atom, a nitro group, etc.

The "heterocyclic group having 2 to 30 carbon atoms", as represented by X¹ and X² in the general formula (I), refers to a group in which one hydrogen atom has been eliminated from a heterocyclic compound, but the heterocyclic group does not encompass an epoxy group because this is classified as a reactive group. The heterocyclic group may have a monocyclic structure or a fused-ring structure. Examples of fused-ring-structure heterocyclic groups having 2 to 30 carbon atoms may include heterocycle-containing fused ring groups having 3 to 30 carbon atoms in which a heterocycle is fused with a heterocycle or a hydrocarbon ring. Concrete examples of heterocyclic groups may include pyridyl, quinolyl, thiazolyl, tetrahydrofuranyl, dioxolanyl, tetrahydropyranyl, methylthiophenyl, hexylthiophenyl, benzothiophenyl, pyrrolyl, pyrrolidinyl, imidazolyl, imidazolidinyl, imidazolinyl, pyrazolyl, pyrazolidinyl, piperidinyl, piperazinyl, pyrimidinyl, furyl, thienyl, benzoxazol-2-yl, thiazolyl, isothiazolyl, oxazolyl, isooxazolyl, morpholinyl, etc.

The heterocyclic group having 2 to 30 carbon atoms may be optionally substituted by a reactive group or a group having a reactive group. A "heterocyclic group having 2 to 30 carbon atoms and substituted by a reactive group or a group having a reactive group" refers to a group having 2 to 30 carbon atoms wherein at least one hydrogen atom in the heterocyclic group has been substituted by a reactive group or a group having a reactive group. The heterocyclic group having 2 to 30 carbon atoms may also have other substituent(s). Examples of other substituents may include a halogen atom, a nitro group, etc.

The "heterocycle-containing group having 3 to 30 carbon atoms", as represented by X¹ and X² in the general formula (I), refers to a group having 3 to 30 carbon atoms wherein at least one hydrogen atom in a hydrocarbon group has been substituted by a heterocyclic group. Examples of the heterocyclic group may include groups given as examples of the aforementioned heterocyclic group having 2 to 30 carbon atoms. Examples of the hydrocarbon group may include hydrocarbon groups having 1 to 20 carbon atoms. The hydrocarbon groups having 1 to 20 carbon atoms will be described further below.

The heterocycle-containing group having 3 to 30 carbon atoms may be optionally substituted by a reactive group or a group having a reactive group. A "heterocycle-containing group having 3 to 30 carbon atoms and substituted by a reactive group or a group having a reactive group" refers to a group having 3 to 30 carbon atoms wherein at least one hydrogen atom in the heterocycle-containing group has been substituted by a reactive group or a group having a reactive group. The heterocycle-containing group having 3 to 30 carbon atoms may also have other substituent(s). Examples of other substituents may include a halogen atom, a nitro group, etc.

The "hydrocarbon group having 1 to 20 carbon atoms", as represented by R¹, R², R³, R⁴, R⁵, R⁶, R⁷, R⁸, R⁹ and R¹⁰ (referred to hereinafter as "R¹ etc.") in the general formula (I), is a group constituted by carbon atoms and hydrogen atoms and having 1 to 20 carbon atoms. Examples of the hydrocarbon groups having 1 to 20 carbon atoms may include aliphatic hydrocarbon groups having 1 to 20 carbon atoms and aromatic hydrocarbon ring-containing groups having 6 to 20 carbon atoms. Note, however, that reactive groups or groups having a reactive group, i.e., a vinyl group, an ethynyl group, and groups having these groups, are not encompassed in the aforementioned hydrocarbon groups.

Examples of the aliphatic hydrocarbon groups having 1 to 20 carbon atoms may include alkyl groups having 1 to 20 carbon atoms, cycloalkyl groups having 3 to 20 carbon atoms, cycloalkylalkyl groups having 4 to 20 carbon atoms, etc.

The alkyl group having 1 to 20 carbon atoms may be linear or branched. Examples of linear alkyl groups may include methyl, ethyl, propyl, butyl, iso-amyl, tert-amyl, hexyl, heptyl, and octyl. Examples of branched alkyl groups may include iso-propyl, sec-butyl, tert-butyl, iso-butyl, iso-pentyl, tert-pentyl, 2-hexyl, 3-hexyl, 2-heptyl, 3-heptyl, iso-heptyl, tert-heptyl, iso-octyl, tert-octyl, 2-ethylhexyl, nonyl, isononyl, decyl, dodecyl, tridecyl, tetradecyl, pentadecyl, hexadecyl, heptadecyl, octadecyl, etc.

Examples of the cycloalkyl groups having 3 to 20 carbon atoms may include saturated monocyclic alkyl groups having 3 to 20 carbon atoms, saturated polycyclic alkyl groups having 3 to 20 carbon atoms, and groups having 4 to 20 carbon atoms wherein at least one hydrogen atom in the ring of these groups has been substituted by an alkyl group. Examples of the aforementioned saturated monocyclic alkyl groups may include cyclopropyl, cyclobutyl, cyclopentyl, cyclohexyl, cycloheptyl, cyclooctyl, cyclononyl, cyclodecyl, etc. Examples of the aforementioned saturated polycyclic alkyl groups may include adamantyl, decahydronaphthyl, octahydropentalene, bicyclo[1.1.1]pentanyl, etc. Examples of alkyl groups substituting the hydrogen atom(s) in the ring of the saturated monocyclic or saturated polycyclic alkyl group may include groups given as examples of the aforementioned alkyl groups having 1 to 20 carbon atoms. Examples of groups in which at least one hydrogen atom in the ring of a saturated polycyclic alkyl group has been substituted by an alkyl group may include bornyl, etc.

The "cycloalkylalkyl group having 4 to 20 carbon atoms" refers to a group having 4 to 20 carbon atoms wherein a hydrogen atom in the alkyl group has been substituted by a cycloalkyl group. The cycloalkyl group in the cycloalkylalkyl group may be monocyclic or polycyclic. Examples of the cycloalkylalkyl groups having 4 to 20 carbon atoms wherein the cycloalkyl group is monocyclic may include cyclopropylmethyl, 2-cyclobutylethyl, 3-cyclopentylpropyl, 4-cyclohexylbutyl, cycloheptylmethyl, cyclooctylmethyl, 2-cyclononylethyl, 2-cyclodecylethyl, etc. Examples of the cycloalkylalkyl groups having 4 to 20 carbon atoms wherein the cycloalkyl group is polycyclic may include 3-3-adamantylpropyl, decahydronaphthylpropyl, etc.

The aromatic hydrocarbon ring-containing groups having 6 to 20 carbon atoms are hydrocarbon groups that include an aromatic hydrocarbon ring but do not include a heterocycle, and may include an aliphatic hydrocarbon group. Examples of the aromatic hydrocarbon ring-containing groups may include aryl groups having 6 to 20 carbon atoms and arylalkyl groups having 7 to 20 carbon atoms.

Examples of the aryl groups having 6 to 20 carbon atoms may include groups given as examples of the aryl groups having 6 to 30 carbon atoms as represented by X¹ and X².

The "arylalkyl group having 7 to 20 carbon atoms" refers to a group wherein at least one hydrogen atom in the alkyl group has been substituted by an aryl group. Examples of the arylalkyl groups having 7 to 20 carbon atoms may include benzyl, fluorenyl, indenyl, 9-fluorenylmethyl, α-methylbenzyl, α,α-dimethylbenzyl, phenylethyl, naphthylpropyl group, etc.

The hydrocarbon group having 1 to 20 carbon atoms represented by R¹ etc. in the general formula (I) may also have a substituent. Examples of the substituent may include a halogen atom, a nitro group, etc. In cases where a substituent is included, the number of carbon atoms refers to the number of carbon atoms in the entire group.

R¹ etc. in the general formula (I) may be a group in which at least one methylene group in the aforementioned hydrocarbon group having 1 to 20 carbon atoms has been substituted by a divalent group selected from the aforementioned {Group A}, or a group in which at least one methylene group in the aforementioned heterocycle-containing group having 3 to 30 carbon atoms has been substituted by a divalent group selected from the aforementioned {Group A}. The number of carbon atoms in these groups represents the number of carbon atoms in the hydrocarbon group and the heterocycle-containing group before substitution of the methylene group.

The hydrocarbon group having 1 to 20 carbon atoms as represented by R¹¹ and R¹² in the general formula (I) is the same as the hydrocarbon group having 1 to 20 carbon atoms as represented by R¹ etc. in the general formula (I).

In the present invention, compounds in which A in the general formula (I) is a benzene ring, a cyclohexadiene ring, or a cyclohexane ring are preferable, because such compounds have excellent solubility to a solvent and can obtain a film having excellent heat resistance and solvent resistance. More specifically, it is preferable that the Compound (I) is a compound represented by the following general formula (Ia), (Ib), or (Ic).

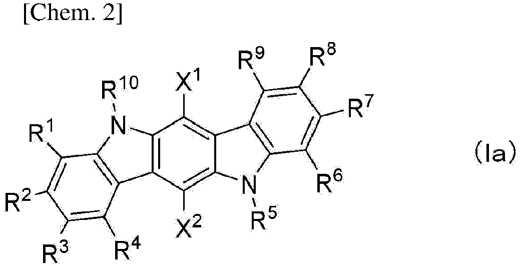

The symbols in the formula are the same as those in the general formula (I).

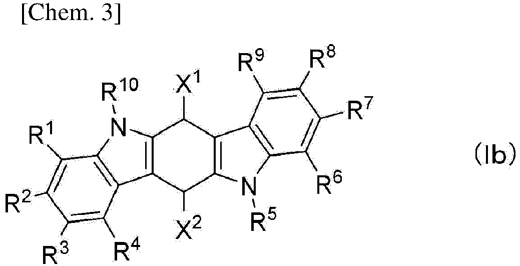

The symbols in the formula are the same as those in the general formula (I).

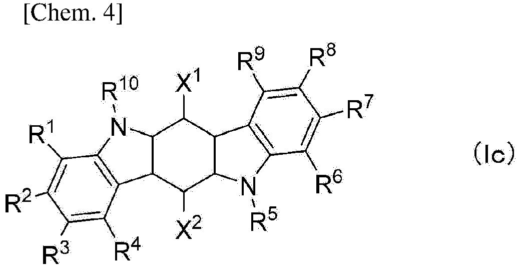

The symbols in the formula are the same as those in the general formula (I).

Preferable among the above are compounds represented by the general formula (Ia), in which A in the general formula (I) is a benzene ring, because the obtained film has even better heat resistance and solvent resistance.

Compounds wherein X¹ and X² in the general formula (I) are a monocyclic-structure aryl group having 6 to 30 carbon atoms, a hydrocarbon-type aromatic fused ring group having 7 to 30 carbon atoms, or a heterocycle-containing fused ring group having 3 to 30 carbon atoms, which may be optionally substituted by a reactive group or a group having a reactive group, are preferable, because such compounds can obtain a film with even better heat resistance. As for the monocyclic-structure aryl group having 6 to 30 carbon atoms, a phenyl group is particularly preferable because of high solubility to a solvent.

The "hydrocarbon-type aromatic fused ring group having 7 to 30 carbon atoms" is a fused ring containing an aromatic ring constituted only by carbon atoms and hydrogen atoms, wherein the atoms constituting the ring structure of the fused ring are solely carbon atoms. Examples of the hydrocarbon-type aromatic fused ring having 7 to 30 carbon atoms may include a naphthyl group, an anthracenyl group, a pyrenyl group, a tetracenyl group, a triphenylenyl group, an azulenyl group, a phenanthrenyl group, a tetracenyl group, a perylenyl group, a fluorenyl group, etc. A naphthyl group and a fluorenyl group are preferable because of high solubility to a solvent.

The "heterocycle-containing fused ring group having 3 to 30 carbon atoms" is a fused ring containing a heterocycle and having 3 to 30 carbon atoms. Examples of the heterocycle-containing fused ring group having 3 to 30 carbon atoms may include an indolyl group, a carbazolyl group, a benzofuranyl group, a benzothiophenyl group, a benzopyrazoyl group, a julolidinyl group, a benzoquinolinyl group, etc. An indolyl group, a carbazolyl group, and a benzothiophenyl group are preferable because of high solubility to a solvent.

In the present invention, compounds wherein X¹ and X² in the general formula (I) are each independently a group that may be optionally substituted by a reactive group or a group having a reactive group are preferable, because it is possible to obtain a film having excellent heat resistance. Further, compounds wherein X¹ and X² are each independently substituted by a reactive group or a group having a reactive group are more preferable, and compounds including an aryl group having 6 to 30 carbon atoms and substituted by a reactive group or a group having a reactive group are particularly preferable.

In cases where X¹ and X² are an aryl group having 6 to 30 carbon atoms and substituted by a reactive group or a group having a reactive group, the aryl group may preferably be a phenyl group, a biphenyl group, a naphthyl group, an anthracenyl group, a pyrenyl group, or a fluorenyl group, because it is possible to obtain a film having excellent heat resistance.

In cases where X¹ or X² are an aryl group having 6 to 30 carbon atoms and substituted by a reactive group or a group having a reactive group, the number of the reactive group or the group having a reactive group is preferably one or two, respectively.

In cases where X¹ and X² each have a reactive group or a group having a reactive group, it is preferable that the reactive group is a carbon-carbon triple bond, because it is possible to obtain a film having excellent heat resistance. As for groups having a carbon-carbon triple bond, alkynyl groups having 2 to 10 carbon atoms and alkynyloxy groups having 2 to 10 carbon atoms are preferable, and a propargyl group and a propargyloxy group are more preferable.

In cases where X¹ and X² each have a reactive group, it is also preferable that the reactive group is a hydroxy group, because it is possible to obtain a film having excellent heat resistance. Further, compounds, wherein X¹ and X² are an aryl group having 6 to 30 carbon atoms and substituted by a hydroxy group, i.e., an aryl group having 6 to 30 carbon atoms and having a phenolic hydroxy group, are more preferable.

X¹ and X² may be the same group, or may be different groups from one another, but from the viewpoint of compound synthesis, it is preferable that they are the same group.

In the present invention, it is also preferable that R⁵ and R¹⁰ in the general formula (I) are a hydrocarbon group having 1 to 20 carbon atoms and substituted by a reactive group. In cases where R⁵ and R¹⁰ are a group having a reactive group, it is preferable that the reactive group is a carbon-carbon triple bond or a carbon-carbon double bond, and more preferably a carbon-carbon triple bond, because it is possible to obtain a film having excellent heat resistance. Examples of hydrocarbon groups having 1 to 20 carbon atoms and substituted by a carbon-carbon triple bond preferably include alkynyl groups having 3 to 10 carbon atoms, and more preferably a propargyl group.

Further, from the viewpoint of compound synthesis, it is preferable that R¹, R², R³, R⁴, R⁶, R⁷, R⁸, and R⁹ are a hydrogen atom.

It is preferable that the compound of the present invention has three or more reactive groups, and particularly preferably from four to six reactive groups, in its molecule because it is possible to obtain a film having excellent heat resistance.

Examples of cases where three or more reactive groups are included in the molecule may include cases where each of X¹ and X² includes two reactive groups, cases where each of X¹ and X² includes two reactive groups and R⁵ and R¹⁰ also include a reactive group, cases where each of X¹ and X² includes one reactive group and R⁵ and R¹⁰ also include a reactive group, and so forth.

In the present invention, it is preferable that X¹ and X² in the general formula (I) are a group having a reactive group. It is also preferable that X¹, X², R⁵, and R¹⁰ in the general formula (I) are a group having a reactive group.

Concrete examples of the aforementioned Compound (I) may include the following compounds (1) to (215).

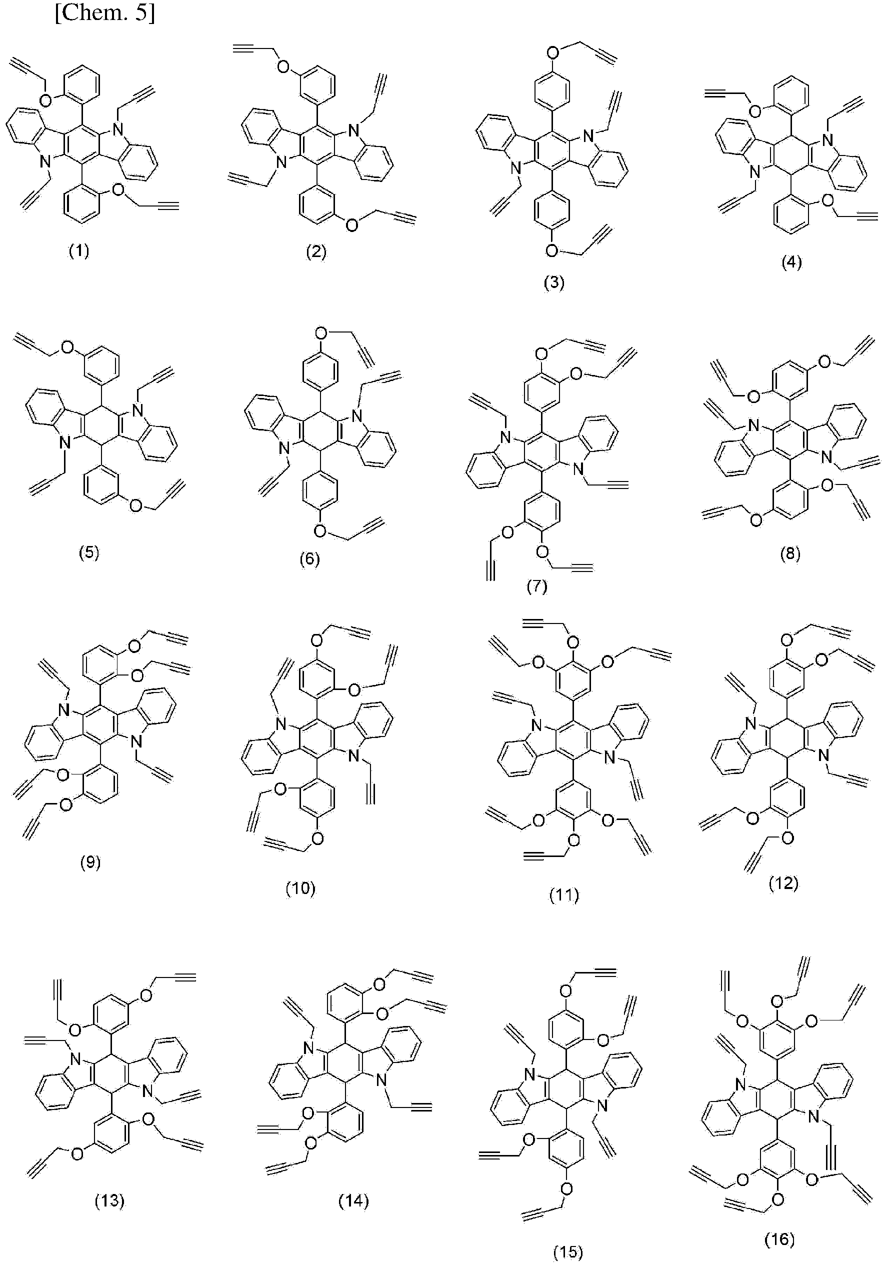

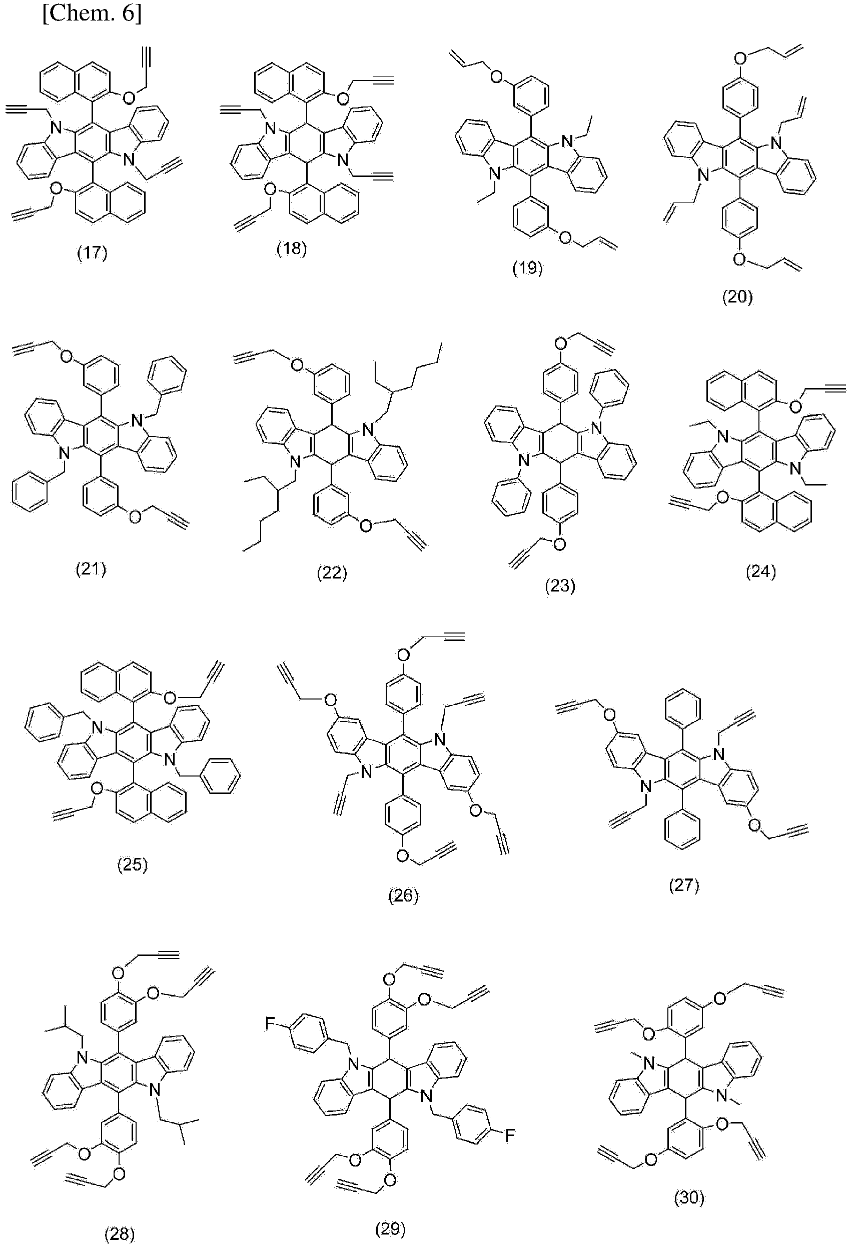

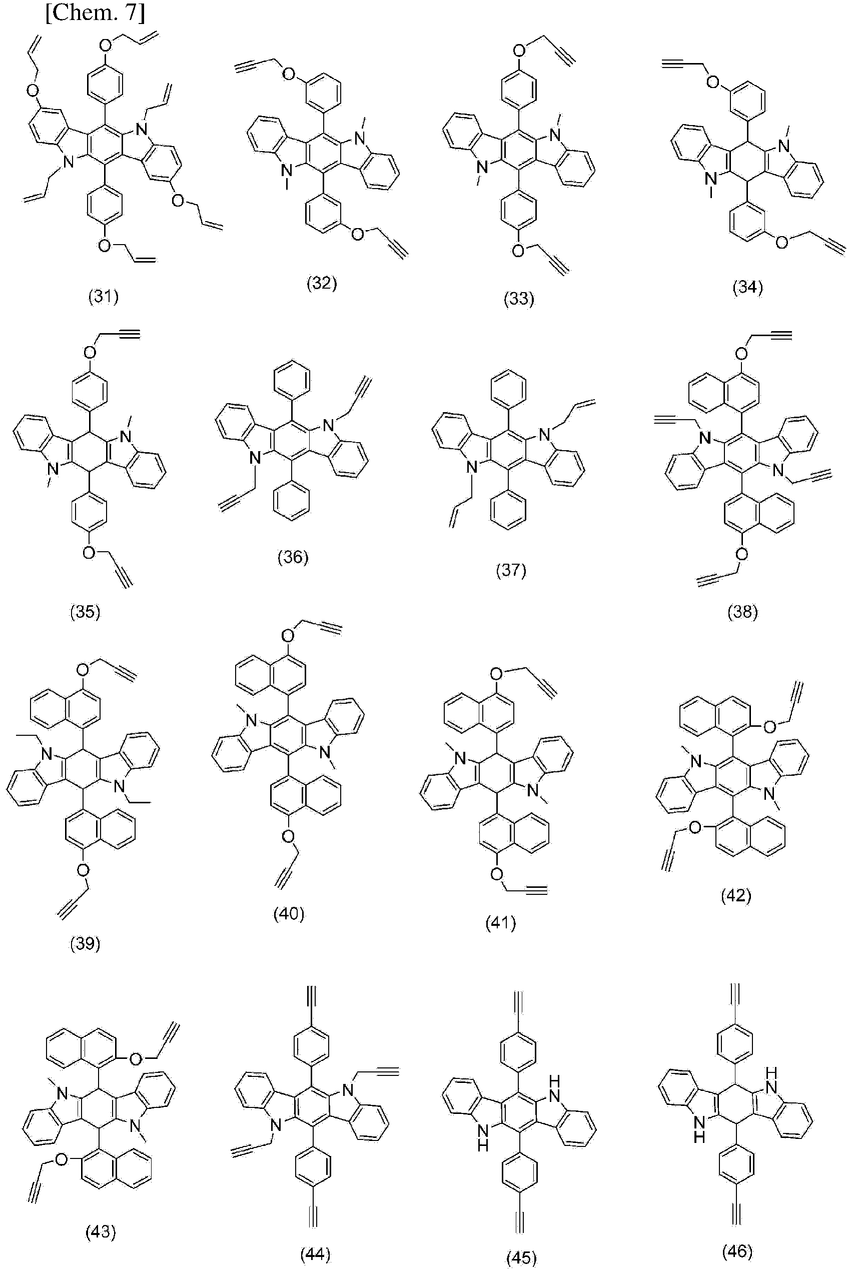

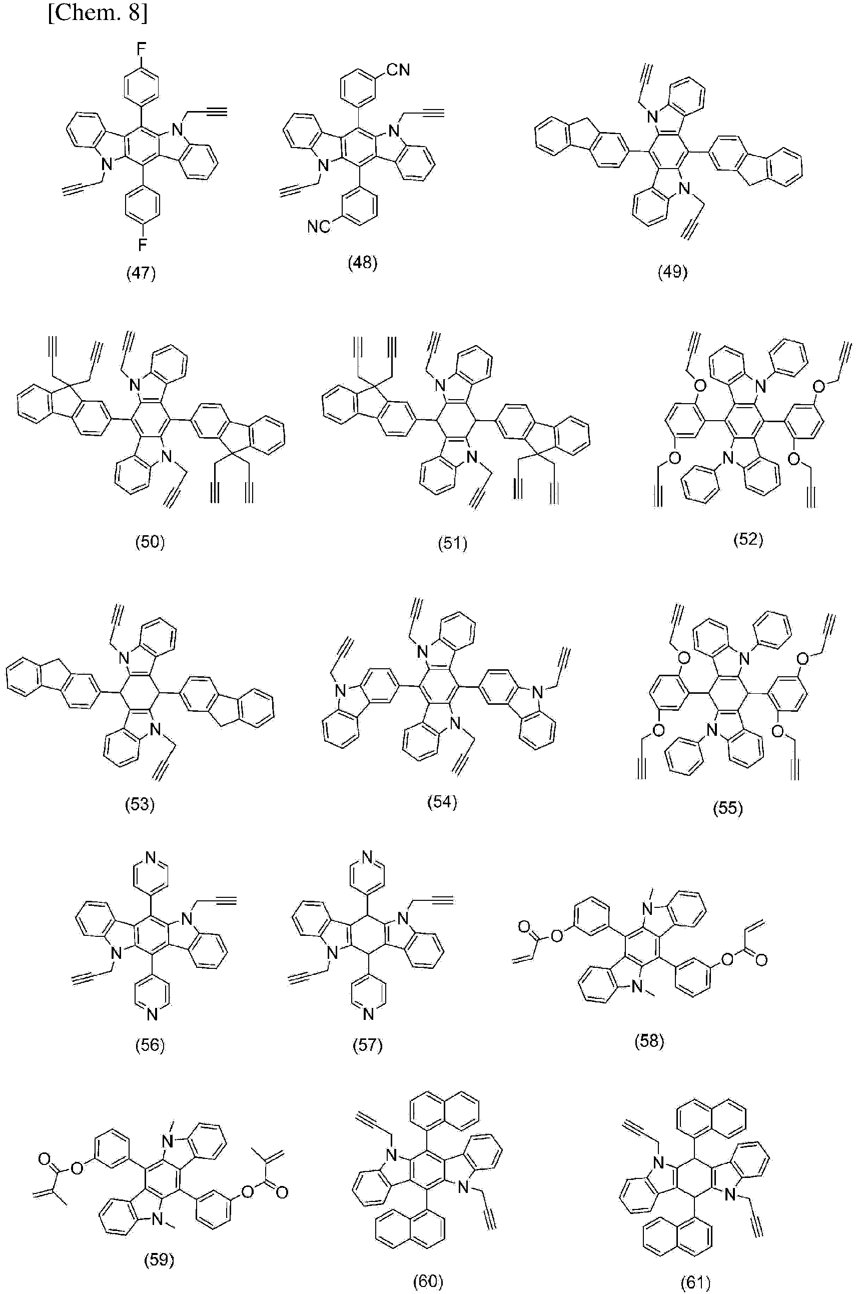

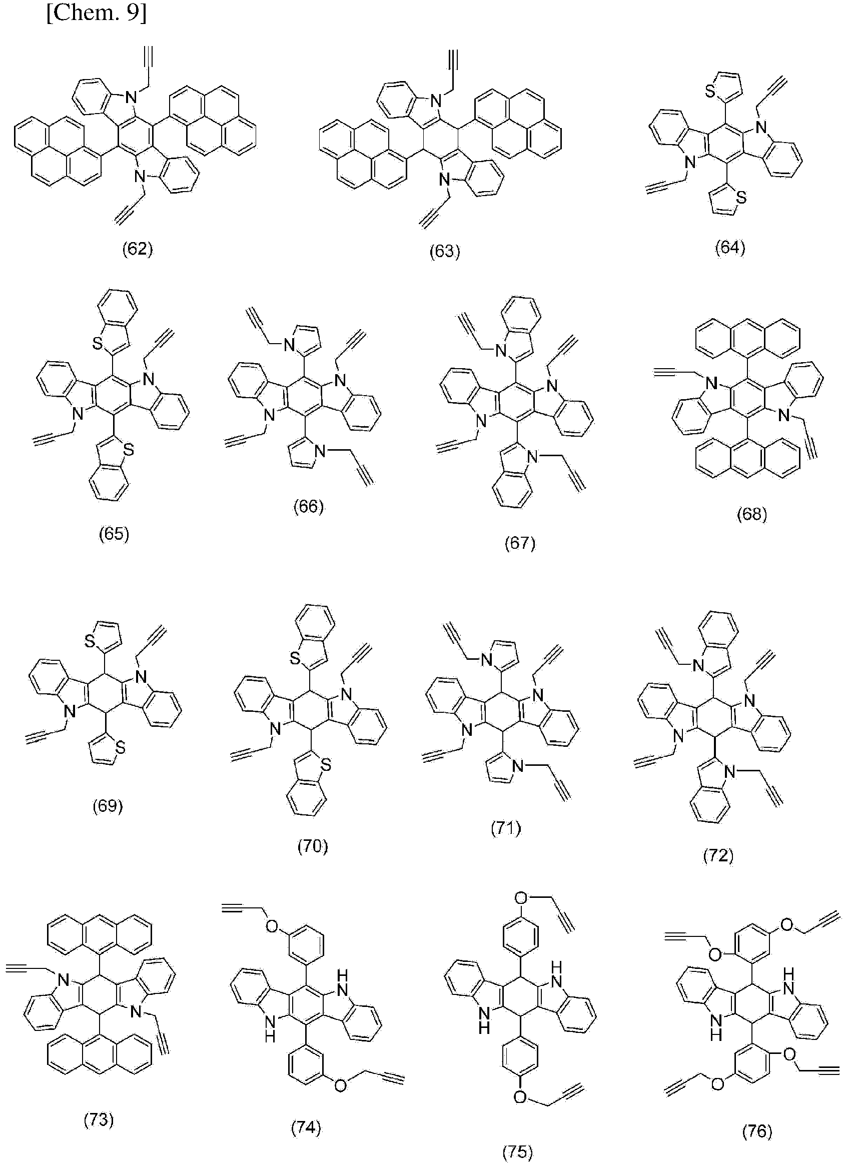

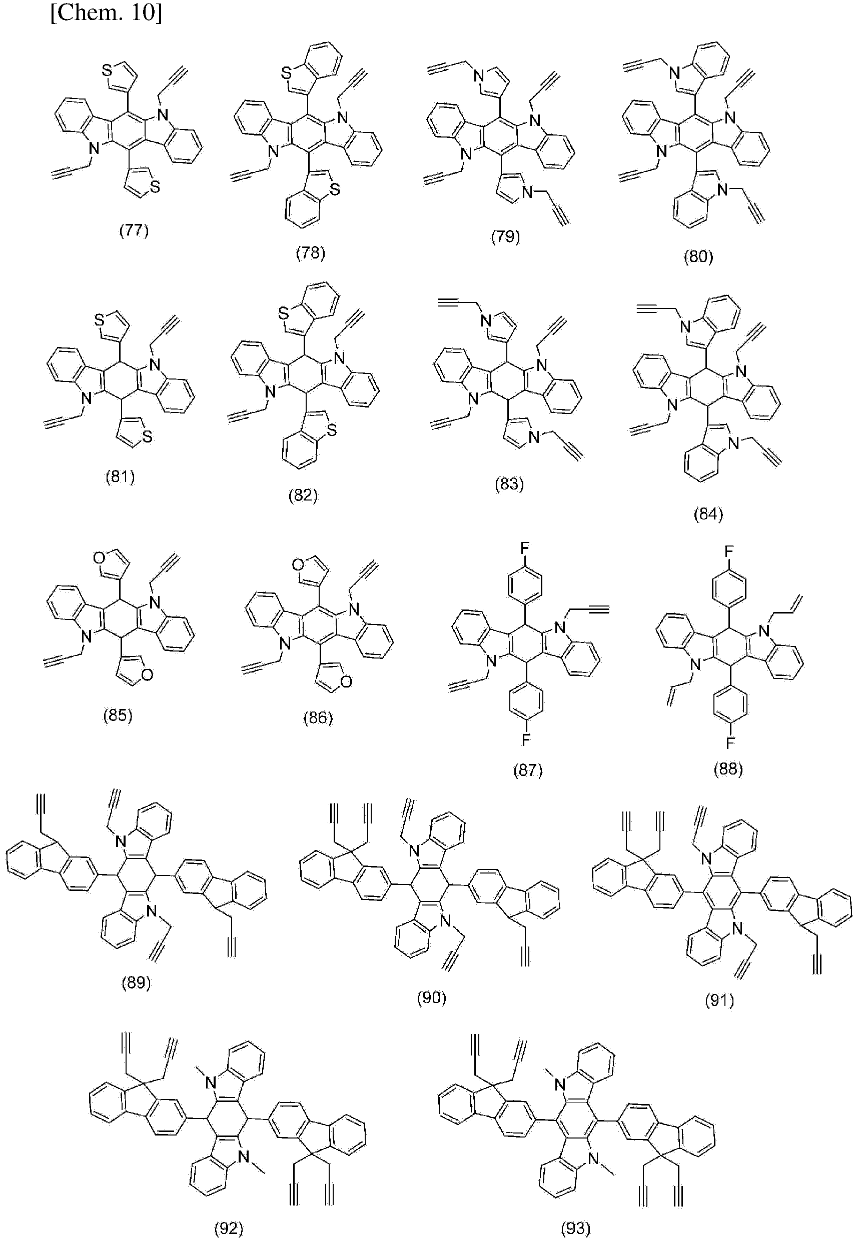

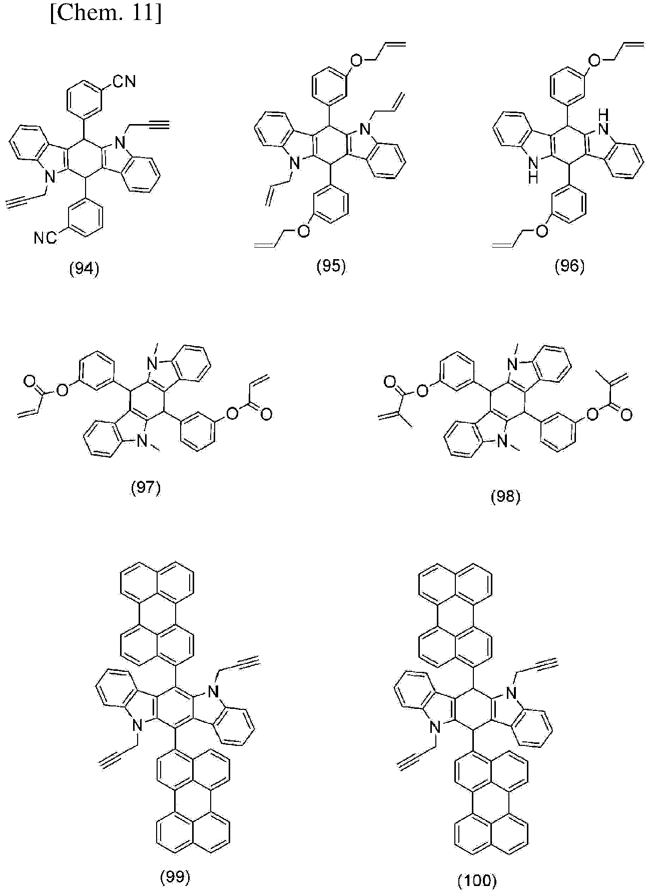

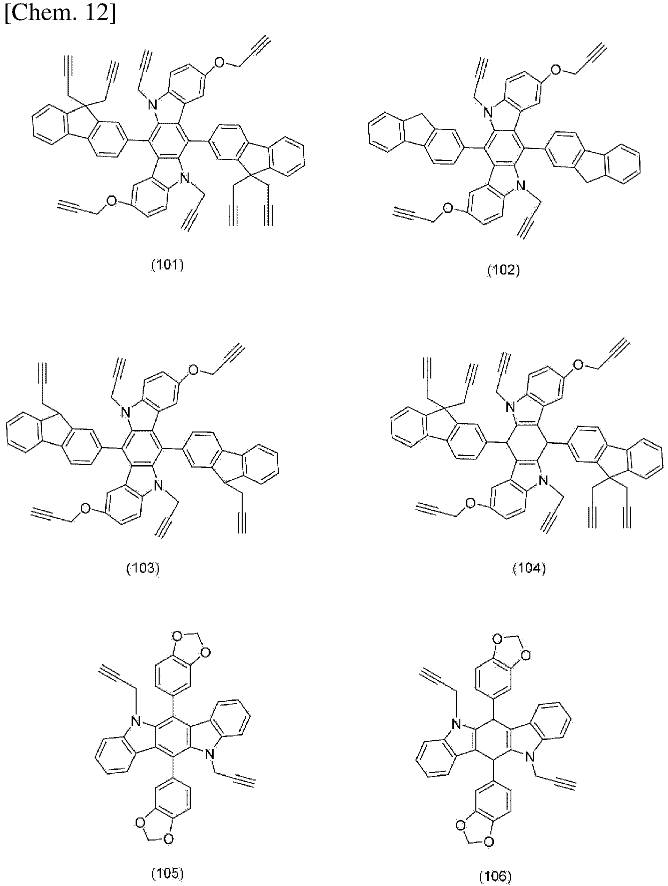

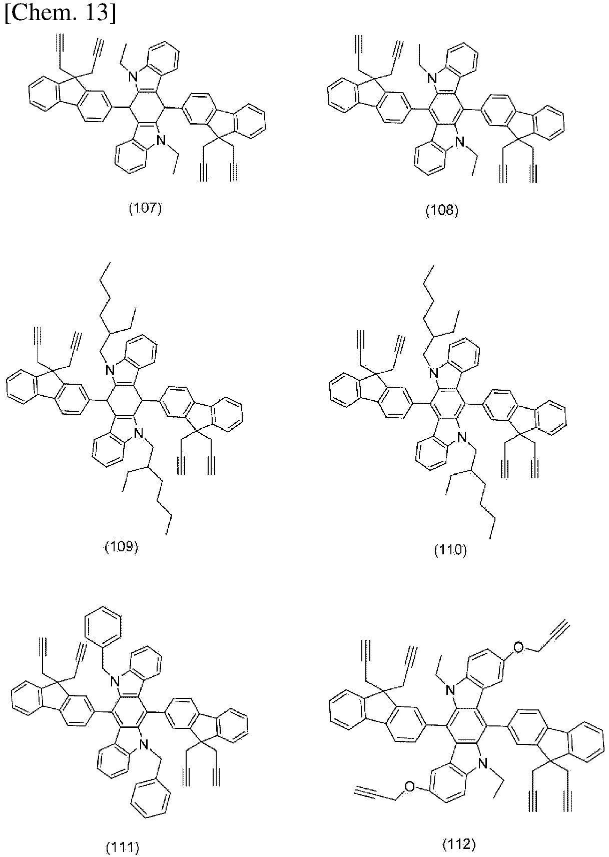

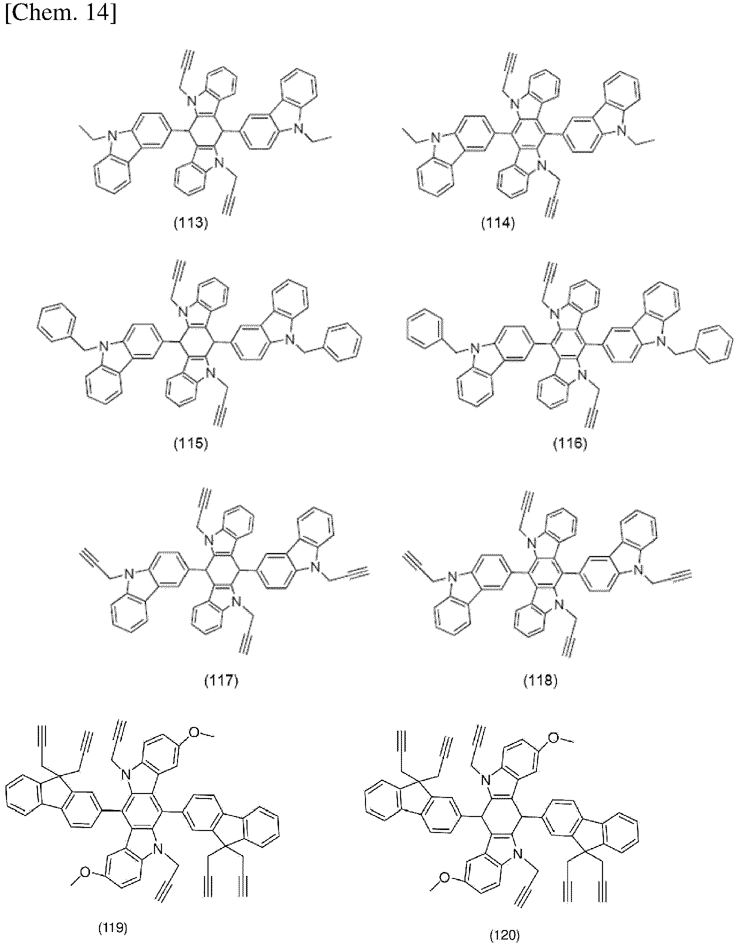

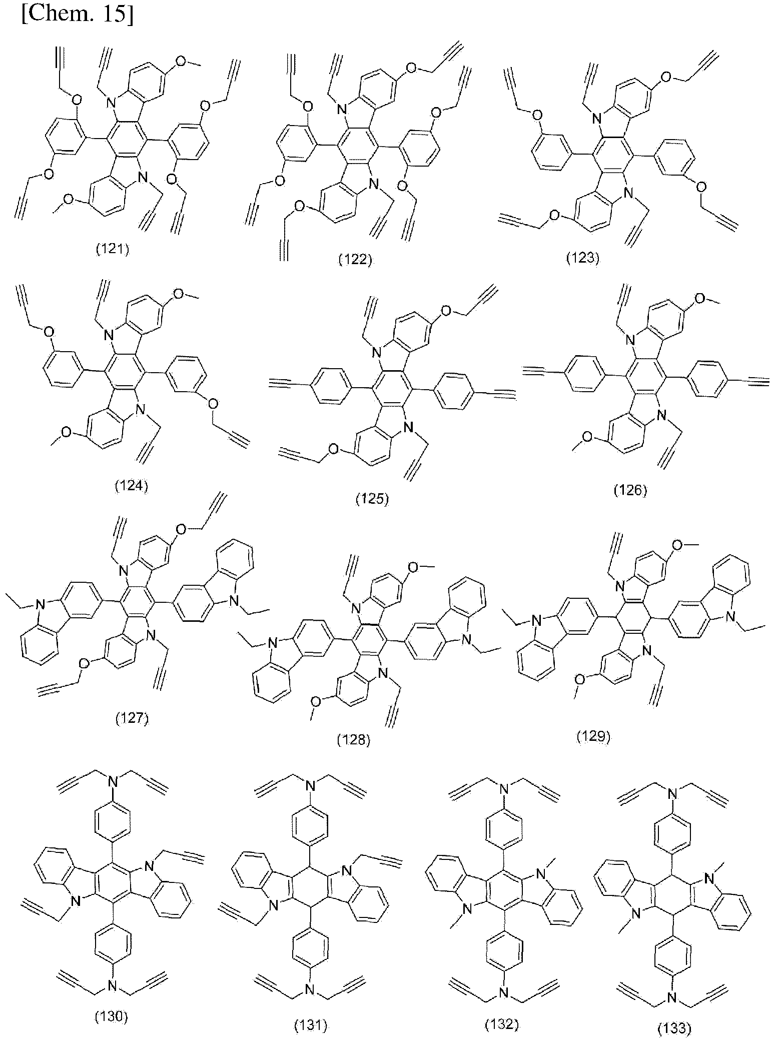

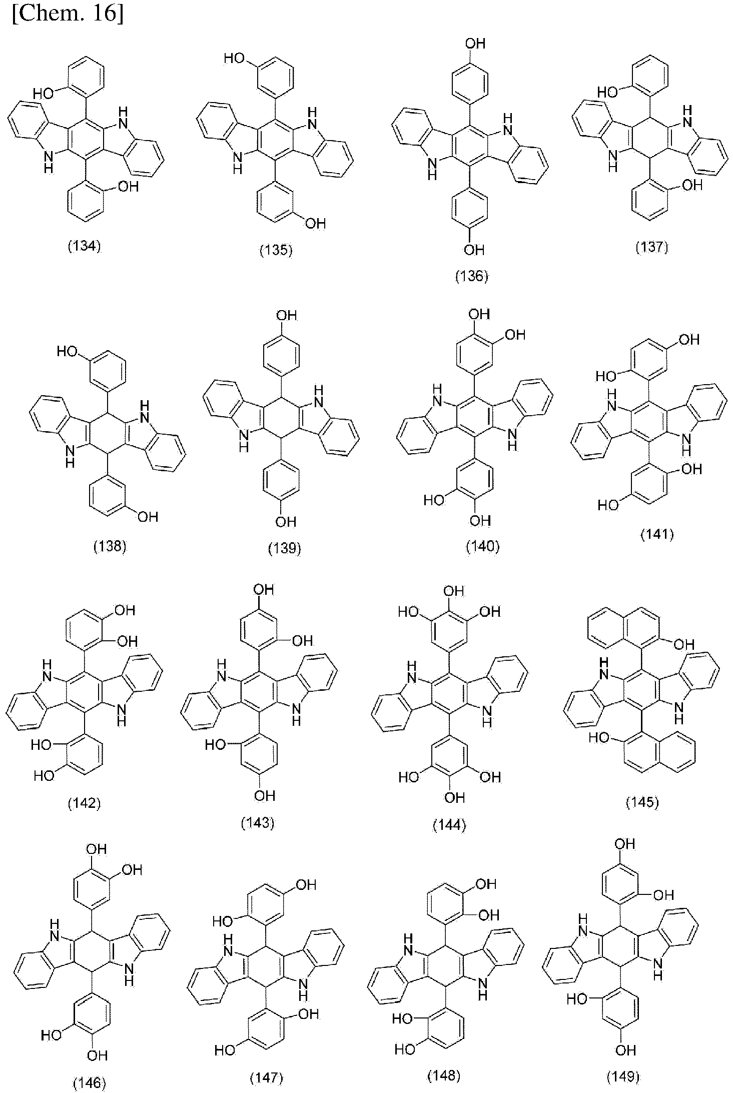

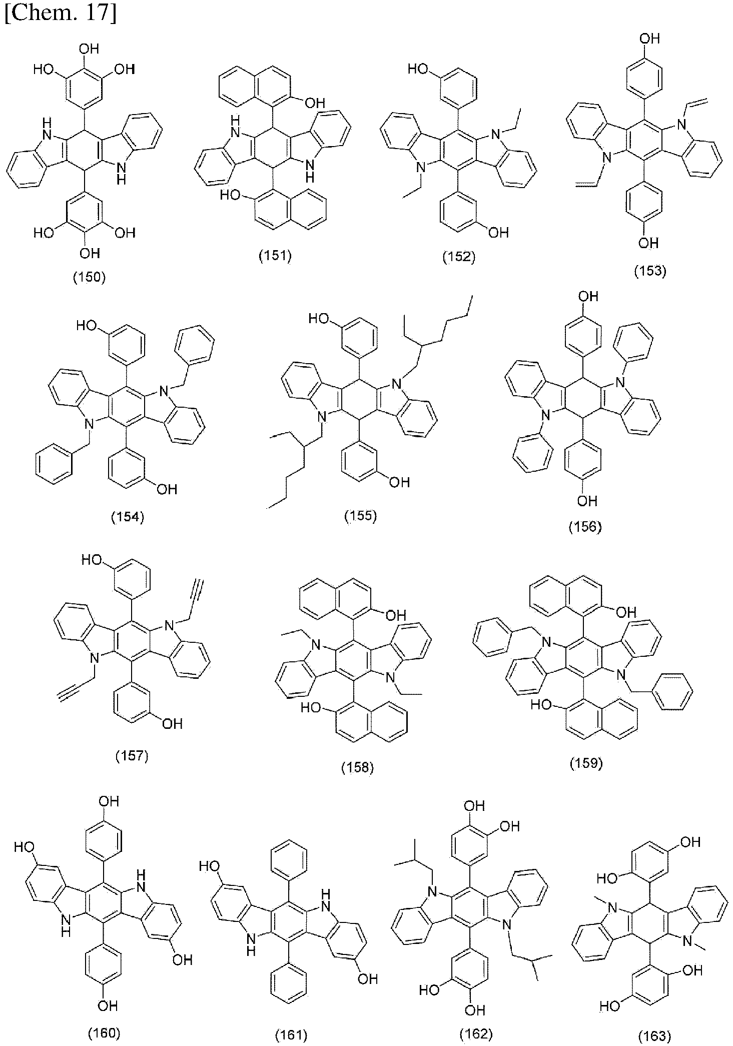

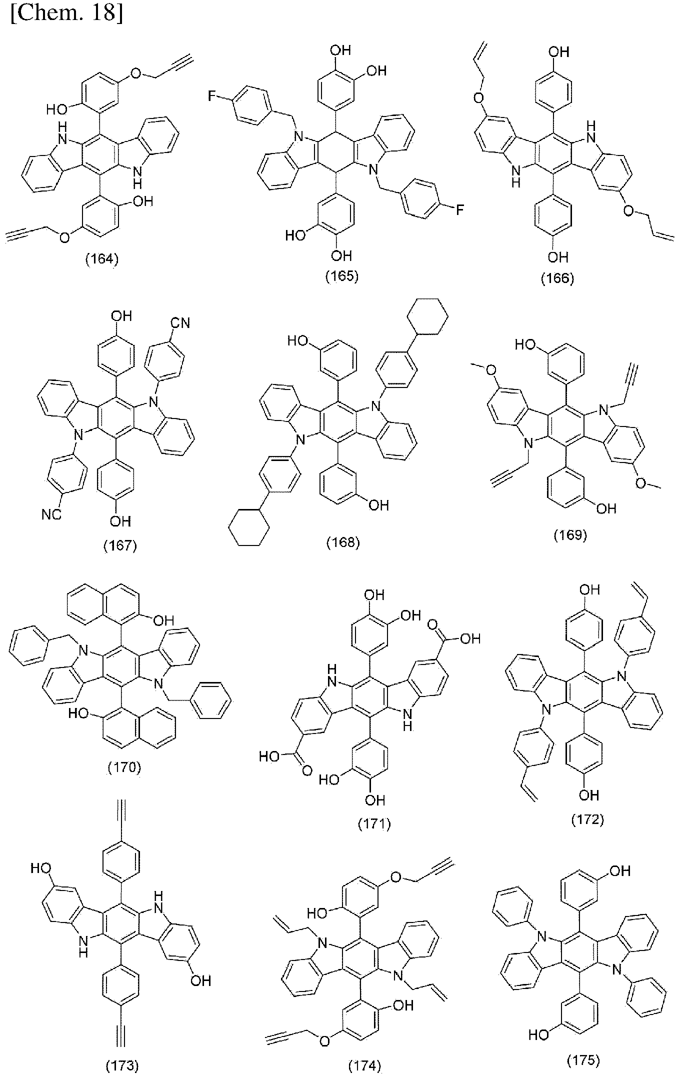

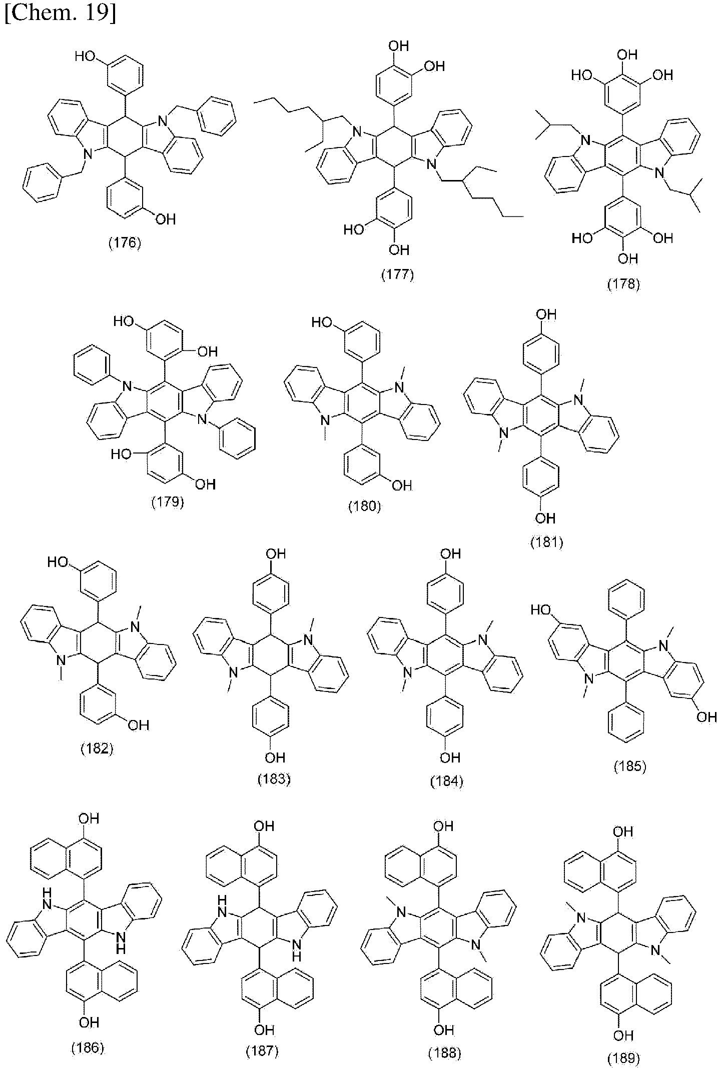

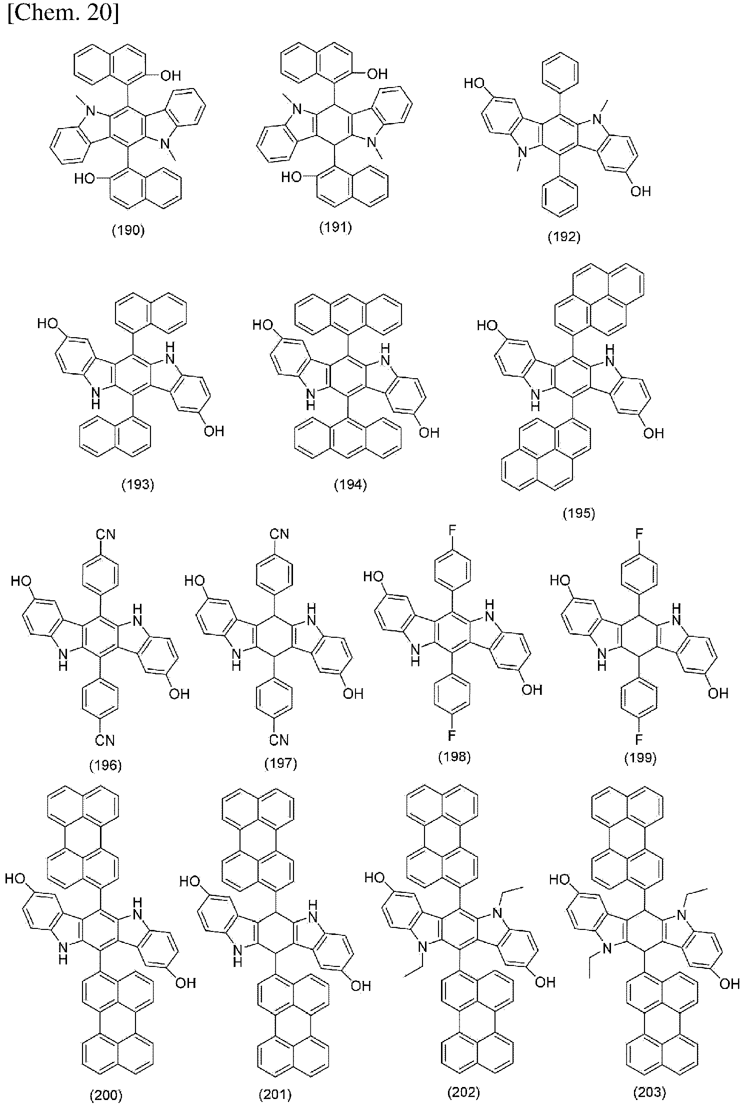

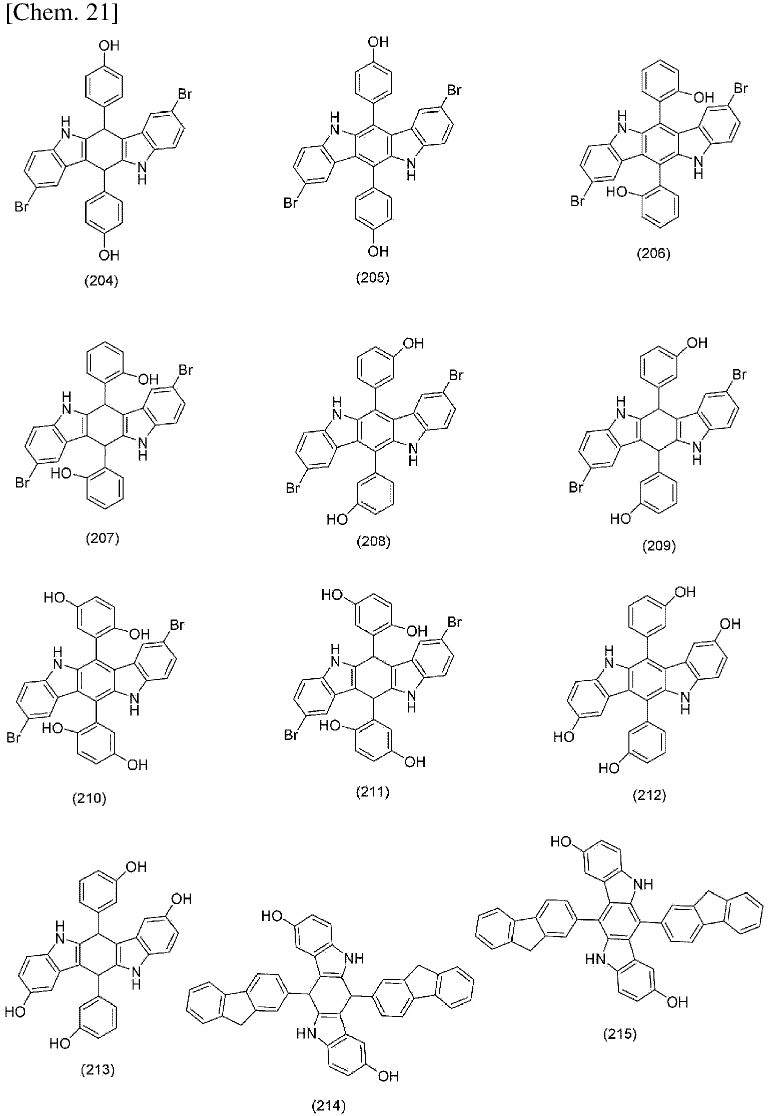

The aforementioned Compound (I) can be produced according to known methods. More specifically, by condensation of indole and an aldehyde compound by using an acid catalyst, it is possible to produce a tetrahydroindolocarbazole compound, which corresponds to a compound wherein A in the general formula (I) is a cyclohexadiene ring. Then, by oxidizing the tetrahydroindolocarbazole compound with an oxidizer, such as iodine, chloranil, etc., it is possible to produce a dihydroindolocarbazole compound, which corresponds to a compound wherein A in the general formula (I) is a benzene ring. Then, by introducing a reactive group to the amino group(s) in these indolocarbazole compounds, it is possible to produce the aforementioned Compound (I). For example, a compound wherein X¹ and X² in the general formula (I) are each a phenyl group, R⁵ and R¹⁰ are each a reactive group R, and R¹, R², R³, R⁴, R⁶, R⁷, R⁸, and R⁹ are each a hydrogen atom, can be produced as follows.

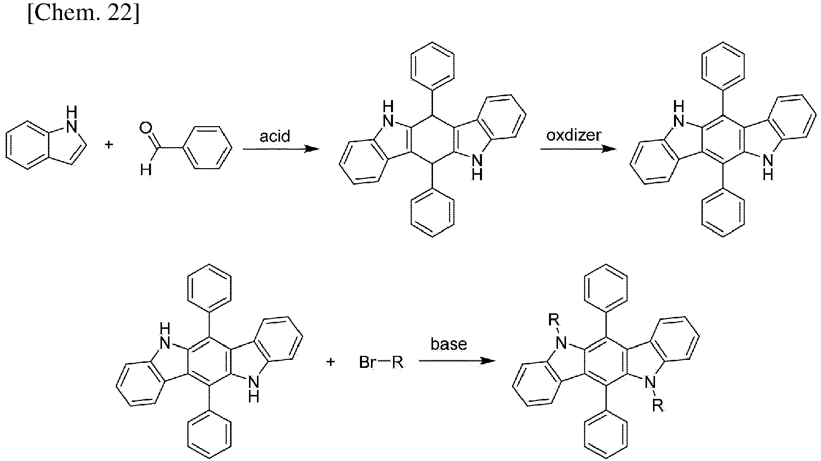

Further, for example, by using an aldehyde compound having a hydroxy group, it is possible to produce a compound having a phenolic hydroxy group, wherein X¹ and X² in the general formula (I) are each a hydroxyphenyl group. Furthermore, by introducing a reactive group R to each hydroxy group and amino group in this compound, it is possible to produce a compound having three or more reactive groups R in the molecule thereof.

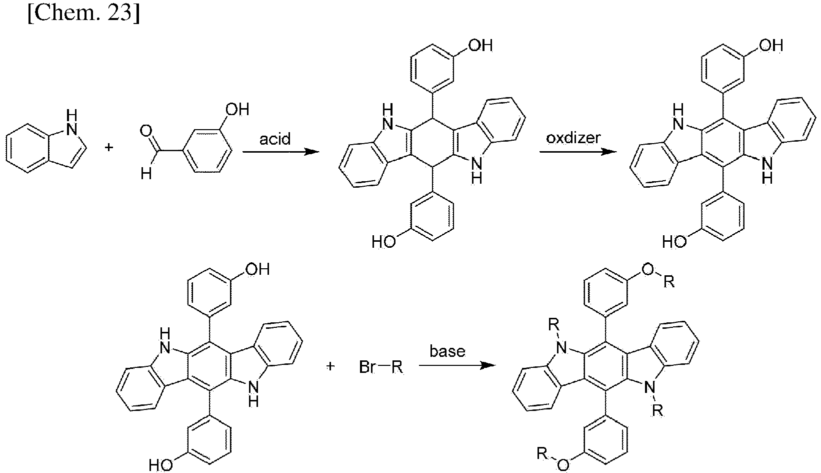

The aforementioned Polymer (I) to be used in the present invention may be a homopolymer of the aforementioned Compound (I), or may be a copolymer with another monomer.

In the present invention, it is preferable that the Polymer (I) is a polymer (also referred to hereinafter as "Polymer (II)") including a structural unit (also referred to hereinafter as "Structural Unit (II)") represented by general formula (II) below and having at least one reactive group.

In the present invention, it is preferable that X¹ and X² in the general formula (II) are a group having a reactive group. It is also preferable that X¹, X², R⁵, and R¹⁰ in the general formula (II) are a group having a reactive group.

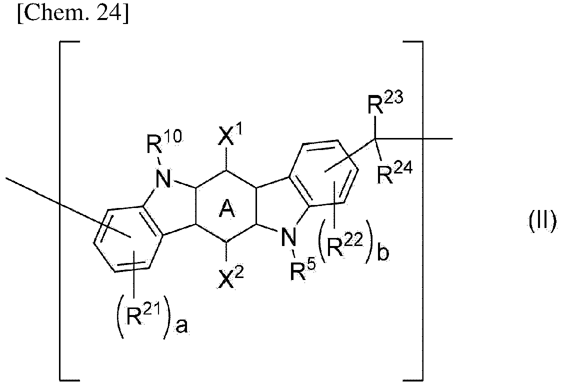

In the formula, A, X¹, X², R⁵, and R¹⁰ are the same as those in the aforementioned general formula (I);
R²¹ and R²² each independently represent a halogen atom, a reactive group, a nitro group, a cyano group, a hydrocarbon group having 1 to 20 carbon atoms and optionally substituted by a reactive group, a heterocyclic group having 2 to 10 carbon atoms and optionally substituted by a reactive group, a heterocycle-containing group having 3 to 30 carbon atoms and optionally substituted by a reactive group, a group in which at least one methylene group in said hydrocarbon group having 1 to 20 carbon atoms has been substituted by a divalent group selected from {Group B} below, or a group in which at least one methylene group in said heterocycle-containing group having 3 to 30 carbon atoms has been substituted by a divalent group selected from {Group B} below;
R²³ and R²⁴ each independently represent a hydrogen atom, a halogen atom, a hydroxy group, a nitro group, a cyano group, a hydrocarbon group having 1 to 20 carbon atoms and optionally having a substituent, a heterocyclic group having 2 to 10 carbon atoms and optionally having a substituent, a heterocycle-containing group having 3 to 30 carbon atoms and optionally having a substituent, a group in which at least one methylene group in said hydrocarbon group having 1 to 20 carbon atoms has been substituted by a divalent group selected from {Group B} below, or a group in which at least one methylene group in said heterocycle-containing group having 3 to 30 carbon atoms has been substituted by a divalent group selected from {Group B} below;
R²³ and R²⁴ may be bonded to one another directly or via a methylene group, -O-, or -S-, to form a ring;
a represents an integer of from 0 to 3; and
b represents an integer of from 0 to 3.

   {Group B }: -O-, -CO-, -COO-, -OCO-, -NR²⁵-, -NR²⁶CO-, -S-,
wherein R²⁵ and R²⁶ each independently represent a hydrogen atom or a hydrocarbon group having 1 to 20 carbon atoms.

Examples of the hydrocarbon groups having 1 to 20 carbon atoms as represented by R²¹, R²², R²³, R²⁴, R²⁵, and R²⁶ in the general formula (II) may include groups given as examples of the hydrocarbon groups having 1 to 20 carbon atoms as represented by R¹ etc. in the general formula (I).

Examples of the heterocyclic groups having 2 to 10 carbon atoms and the heterocycle-containing groups having 3 to 30 carbon atoms, as represented by R²¹, R²² , R²³, and R²⁴ in the general formula (II), may include groups given as examples of the heterocyclic groups having 2 to 10 carbon atoms and the heterocycle-containing groups having 3 to 30 carbon atoms as represented by R¹ etc. in the general formula (I).

In the present invention, polymers in which A in the general formula (II) is a benzene ring, a cyclohexadiene ring, or a cyclohexane ring are preferable, because it is possible to obtain a film having excellent heat resistance. More specifically, it is preferable that the Structural Unit (II) is a structural unit represented by the following general formula (IIa), (IIb), or (IIc), and having at least one reactive group.

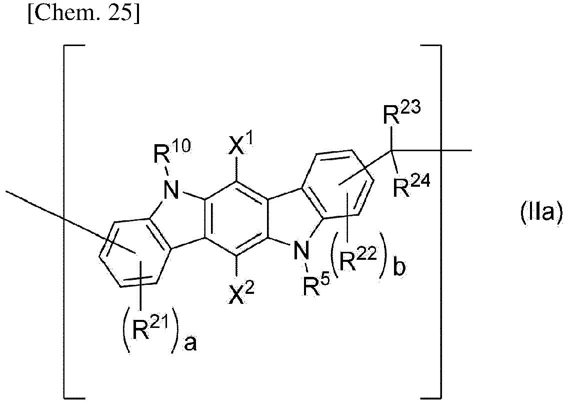

The symbols in the formula are the same as those in the general formula (II).

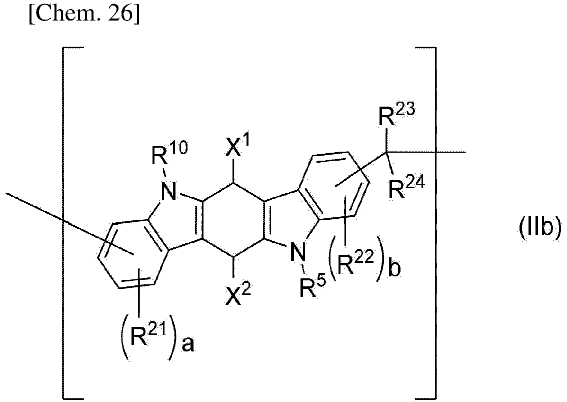

The symbols in the formula are the same as those in the general formula (II).

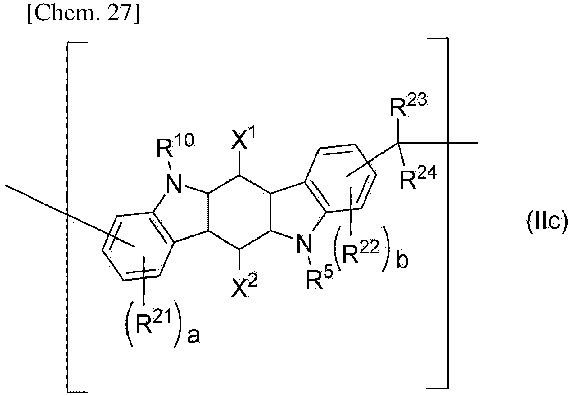

The symbols in the formula are the same as those in the general formula (II).

In the Structural Unit (II), preferred embodiments of A, X¹, X², R⁵, and R¹⁰ in the general formula (II) may be the same as those described for the aforementioned Compound (I).

It is preferable that a and b in the general formula (II) are 0, because it is possible to obtain a film having excellent heat resistance. The expression "a and b are 0" is synonymous with the expression that R¹, R², R³, R⁴, R⁶, R⁷, R⁸, and R⁹ in the general formula (I) are a hydrogen atom.

In the present invention, it is preferable that the weight-average molecular weight of the polymer is 500 or greater and less than 30,000, because such a polymer has excellent solubility to a solvent and can obtain a film having excellent heat resistance. It is more preferable that the weight-average molecular weight is 700 or greater and less than 15,000, even more preferably 1,000 or greater and less than 10,000, because of excellent solubility to a solvent and excellent film formability.

In the present invention, "weight-average molecular weight" refers to the molecular weight in terms of polystyrene as measured by gel permeation chromatography (GPC).

For example, the weight-average molecular weight (Mw) can be measured by using a GPC (LC-2000 plus series) from JASCO Corporation, wherein tetrahydrofuran is employed as an eluent, the polystyrene standards for creating a calibration curve have Mw 1,110,000, 707,000, 397,000, 189,000, 98,900, 37,200, 15,700, 9,490, 5,430, 3,120, 1,010, and 589 (TSKgel Standard Polystyrene from Tosoh Corporation), and KF-804, KF-803, and KF-802 (from Showa Denko K.K.) are employed as measurement columns.

Concrete examples of the aforementioned Structural Unit (II) may include the following structural units (u1) to (u101).

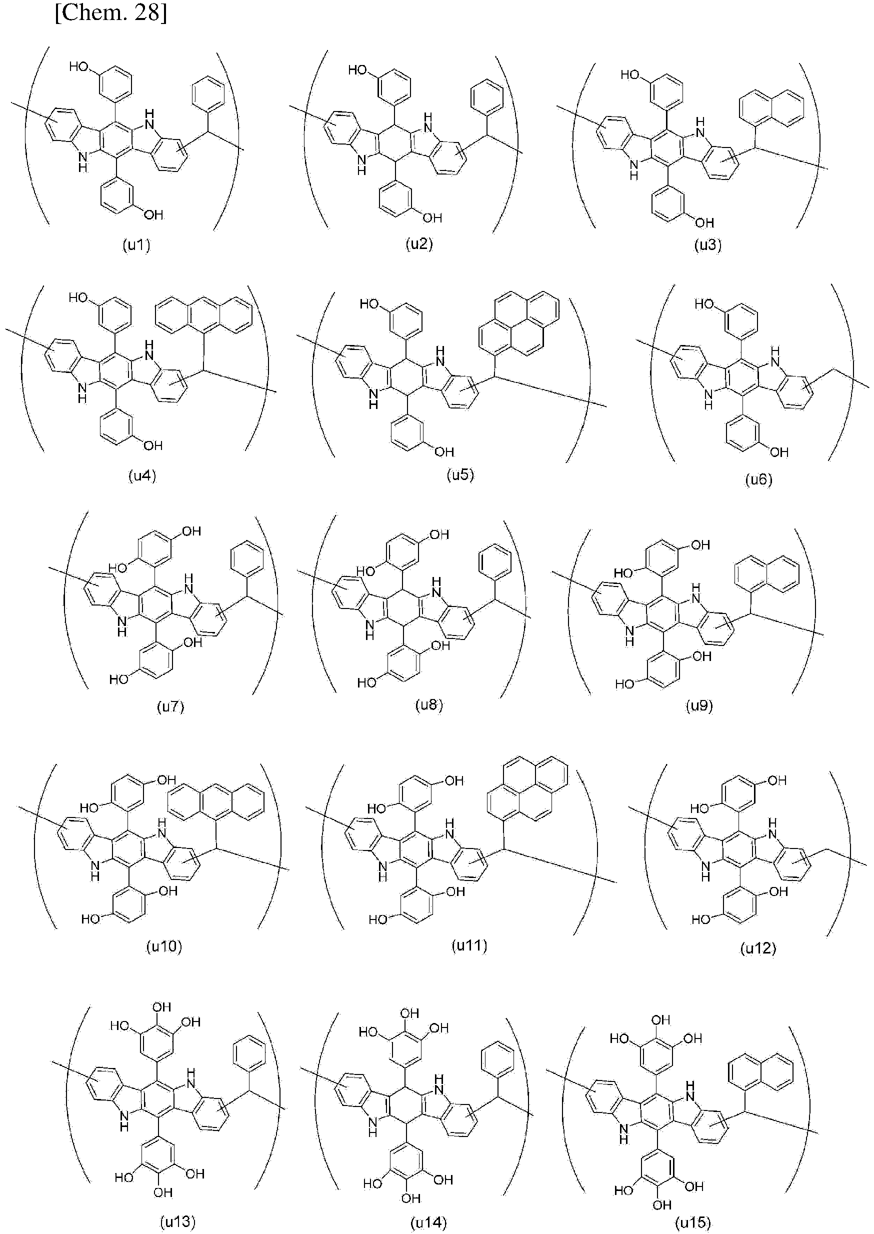

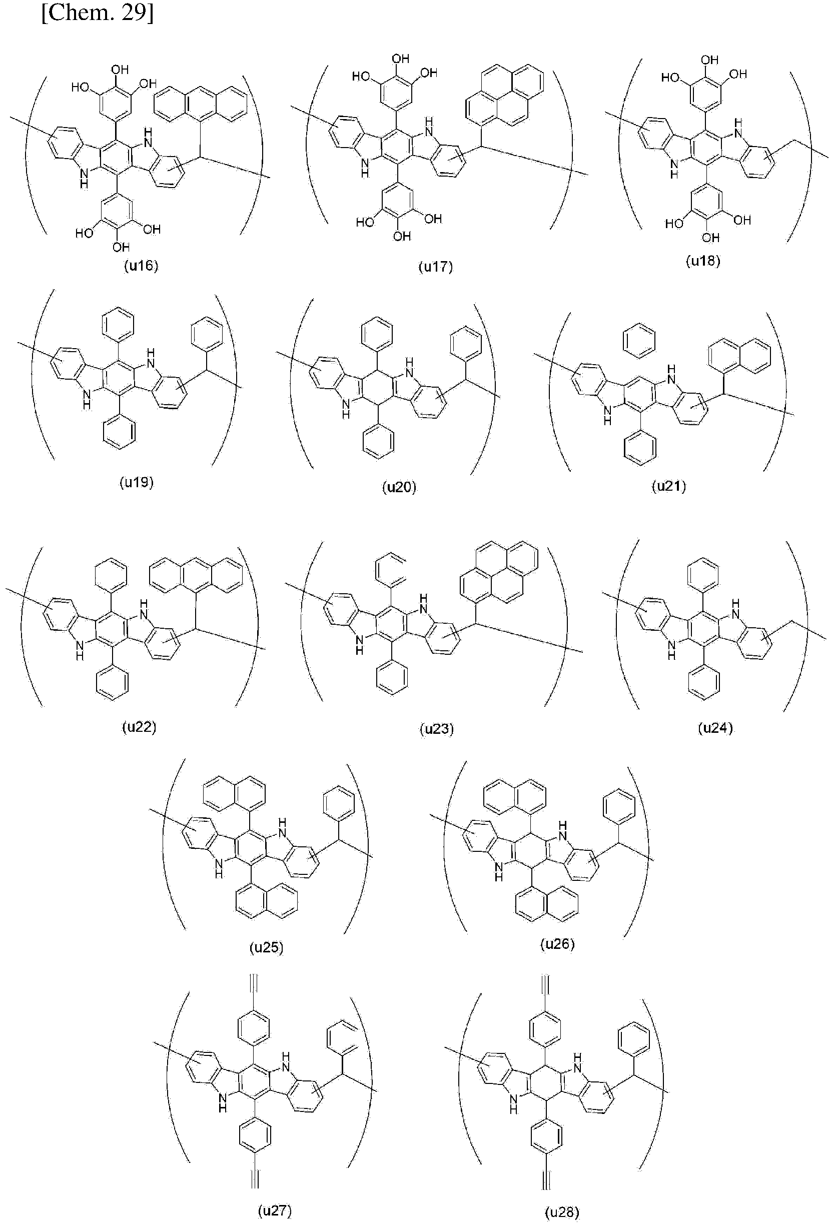

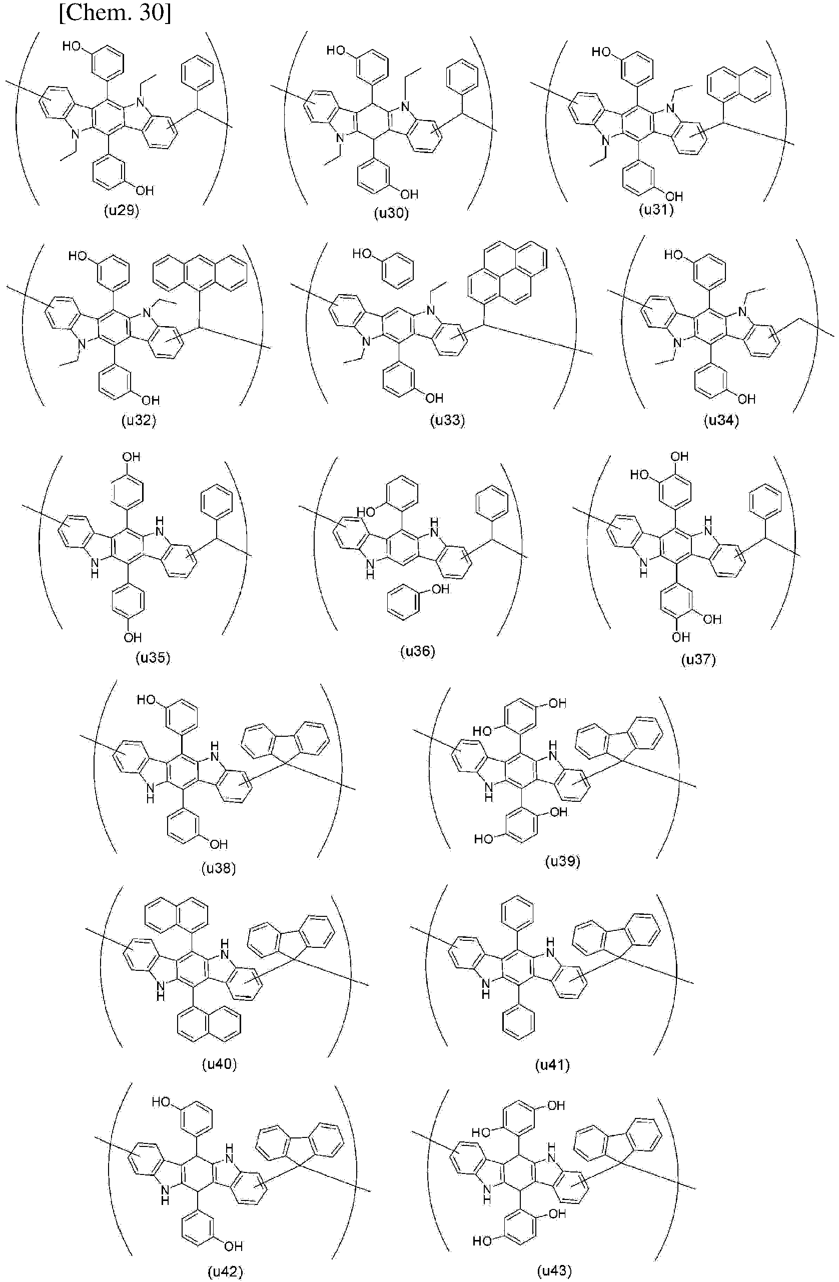

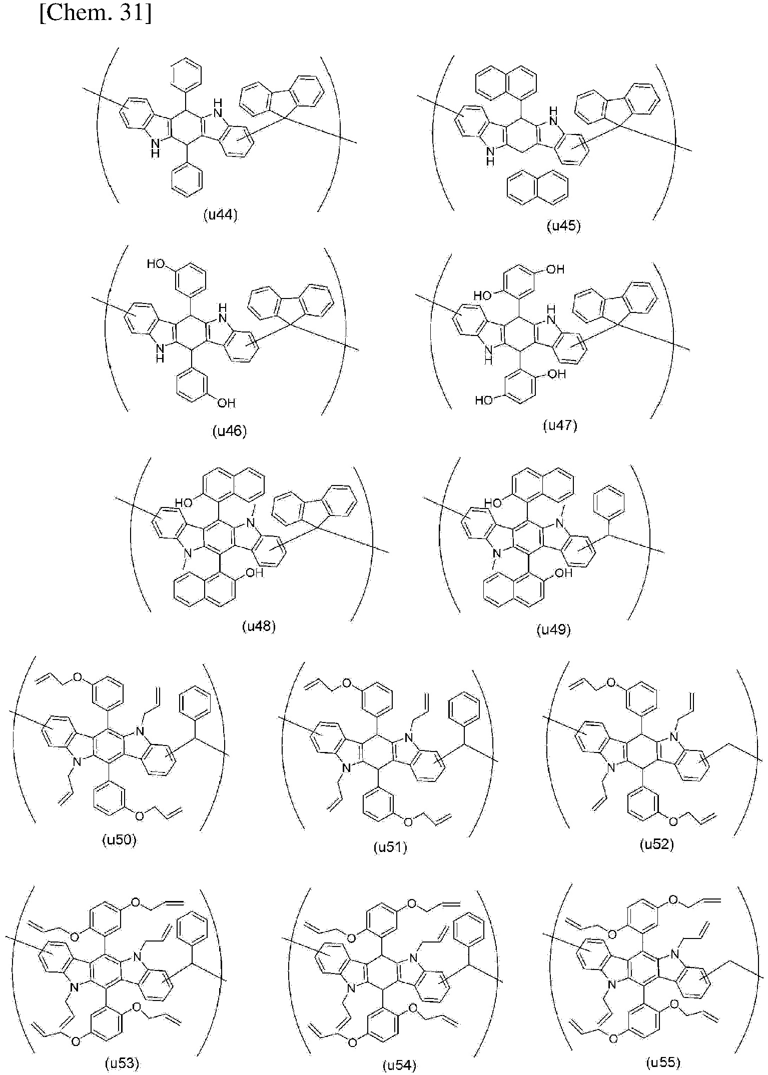

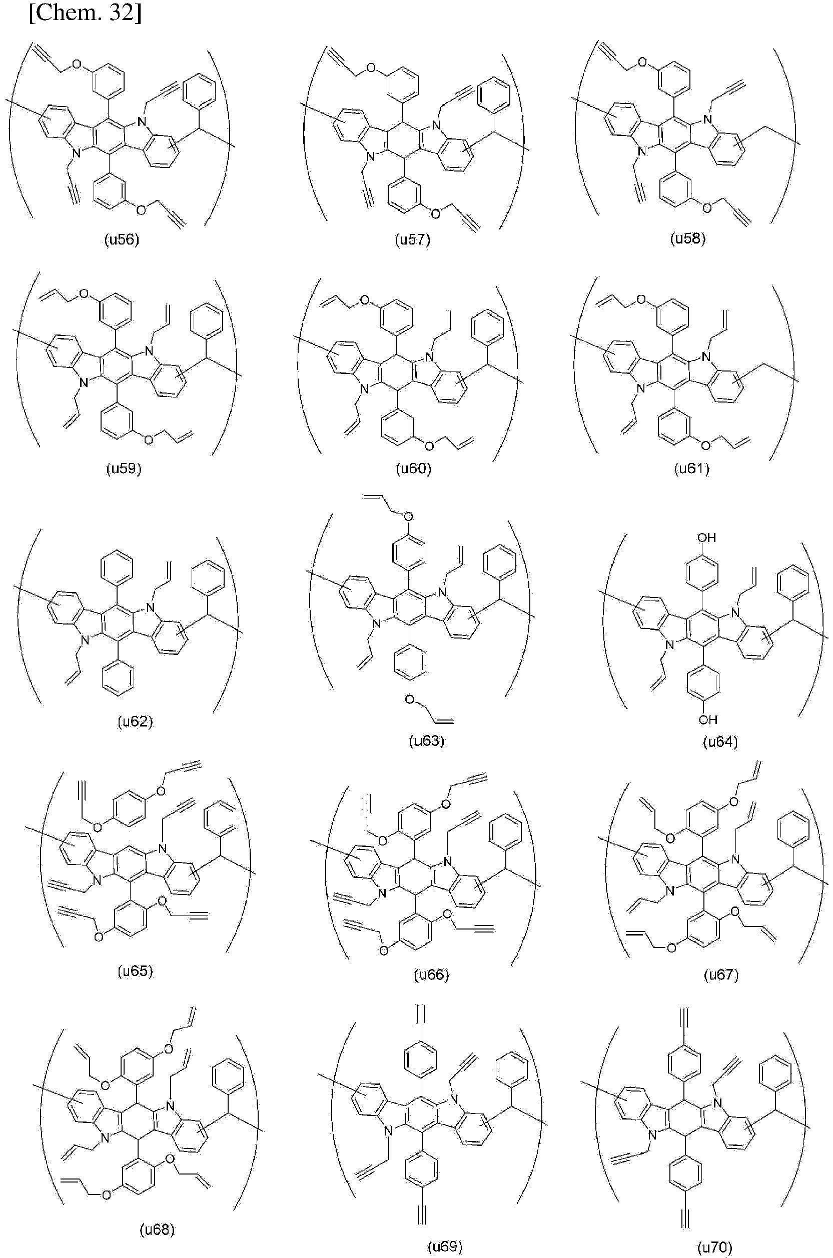

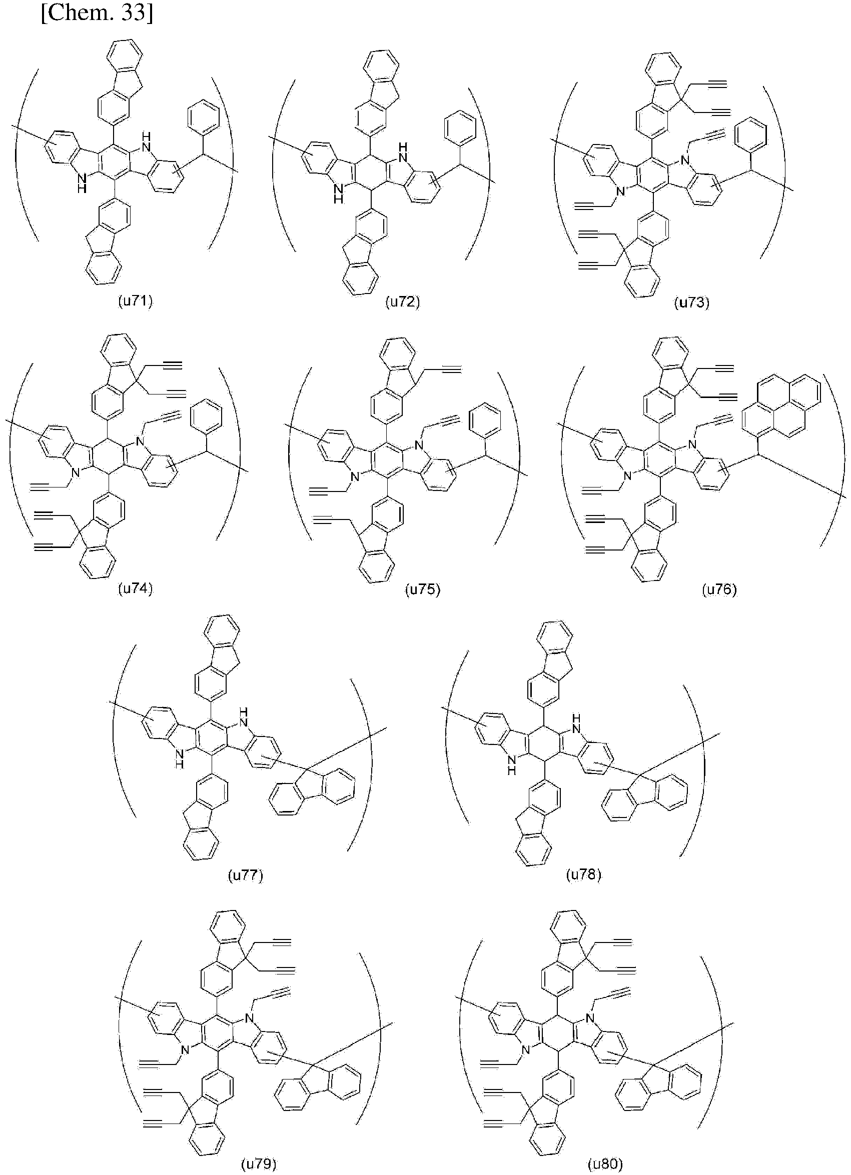

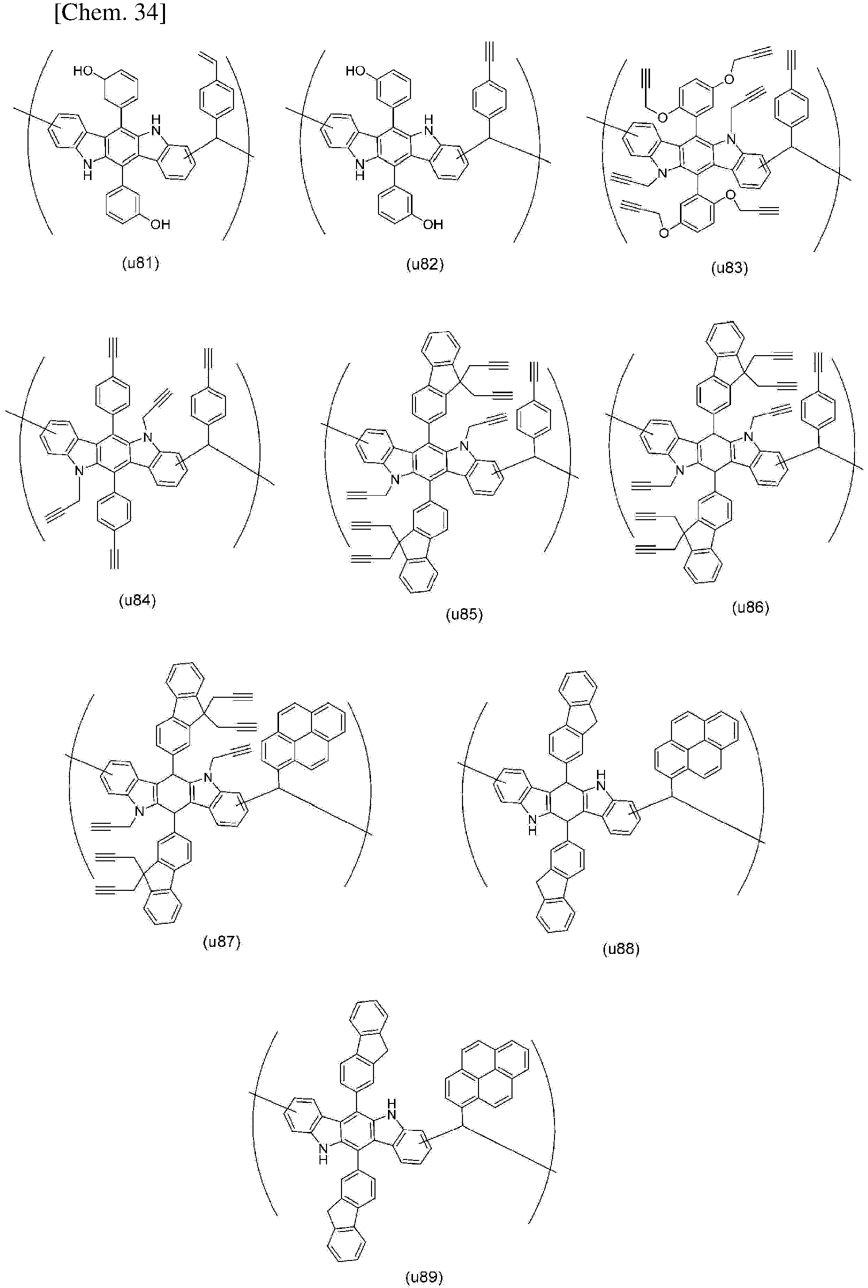

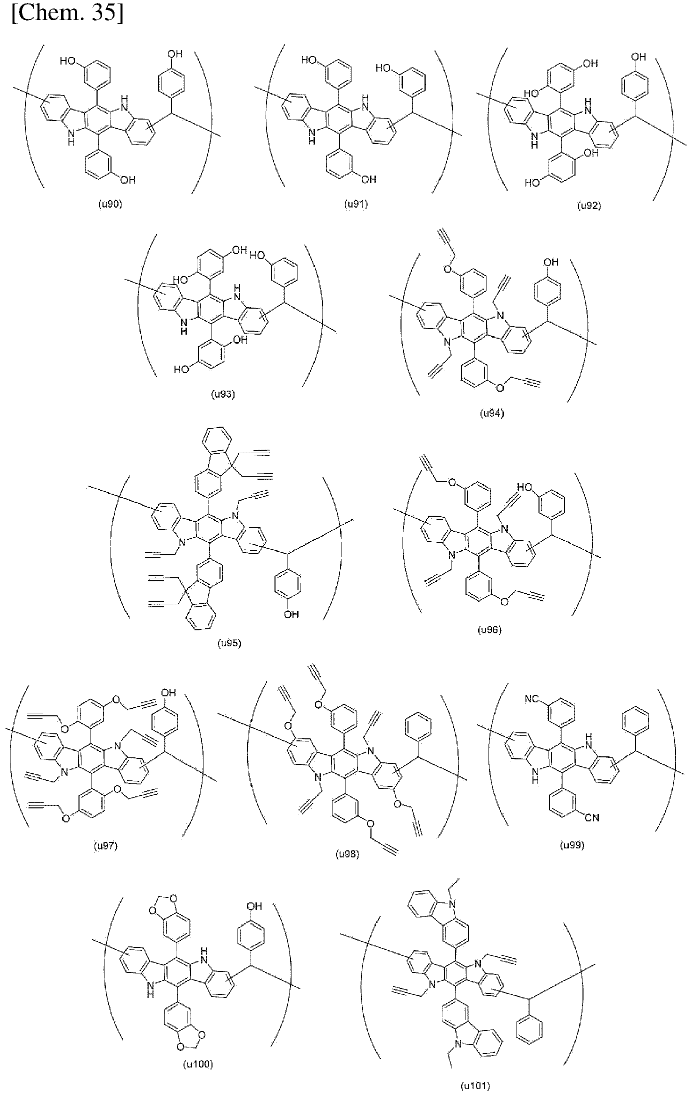

The aforementioned Polymer (II) can be produced by making the aforementioned Compound (I) and an aldehyde or a ketone undergo a condensation reaction in the presence of an acid catalyst.

Examples of the acid catalyst to be used for the condensation reaction may include: inorganic acids, such as sulfuric acid, hydrochloric acid, hydrobromic acid, phosphoric acid, heteropoly acids, etc.; organic acids, such as methanesulfonic acid, benzenesulfonic acid, p-toluenesulfonic acid, p-toluenesulfonic acid monohydrate, formic acid, oxalic acid, oxalic acid dihydrate, trifluoromethanesulfonic acid, etc.; and Lewis acids, such as aluminum trichloride, aluminum ethoxide, aluminum isopropoxide, boron trifluoride, boron trichloride, boron tribromide, tin tetrachloride, tin tetrabromide, titanium tetrachloride, titanium tetrabromide, titanium oxide, etc.

R²³ and R²⁴ in the Structural Unit (II) are groups originating from the aldehyde or ketone which is a material of the polymer. For example, using formaldehyde will give a polymer wherein R²³ and R²⁴ are a hydrogen atom. Further, using benzaldehyde will give a polymer wherein R²³ is a phenyl group and R²⁴ is a hydrogen atom. Similarly, using naphthoaldehyde, anthracenecarboxaldehyde, pyrenecarboxaldehyde, or fluorenecarboxaldehyde respectively gives polymers wherein R²³ is a naphthyl group, an anthracenyl group, a pyrenyl group, or a fluorenyl group, respectively, and R²⁴ is a hydrogen atom.

A polymer in which neither R²³ nor R²⁴ in the Structural Unit (II) is a hydrogen atom can be obtained by using a ketone. Examples of ketones may include diaryl ketones and alkyl aryl ketones. For example, using diphenyl ketone will give a polymer wherein R²³ and R²⁴ are a phenyl group, and using methyl phenyl ketone will give a polymer wherein R²³ is a methyl group and R²⁴ is a phenyl group.

A polymer wherein R²³ and R²⁴ in the general formula (I) are directly bonded to one another to form a ring can be obtained by using a cyclic ketone. For example, using fluorenone will give a polymer wherein R²³ and R²⁴ are a phenyl group, which are directly bonded to one another to form a ring.

The amount of usage of aldehyde and ketone may be adjusted such that the obtained polymer has a desired molecular weight and copolymerization ratio, and it is preferable that the amount is from 0.05 to 1.1 mol, more preferably from 0.3 to 1.0 mol, with respect to 1 mol of the Compound (I).

The condensation reaction may be conducted without a solvent, but is typically conducted using a solvent. Any solvent can be used so long as it does not inhibit the reaction, and examples may include: alcohols, such as methanol, ethanol, isopropyl alcohol, butanol, ethylene glycol, propylene glycol, diethylene glycol, ethylene glycol monomethyl ether, propylene glycol monomethyl ether, etc.; ethers, such as diethylene glycol dimethyl ether, 1,4-dioxane, tetrahydrofuran, etc.; halogen-based solvents, such as methylene chloride, chloroform, dichloroethane, etc.; hydrocarbons, such as toluene, xylene, etc.; ketones, such as ethyl methyl ketone, isobutyl methyl ketone, etc.; esters, such as ethyl acetate, butyl acetate, propylene glycol methyl ether acetate, etc.; and aprotic polar solvents, such as acetonitrile, dimethyl sulfoxide, dimethyl acetamide, N,N-dimethylformamide, N-methylpyrrolidone, etc. One type may be used alone, or two or more types may be used as a mixture.

The temperature for the condensation reaction is typically from 50°C to 200°C, and preferably from 100°C to 200°C from the viewpoint of reaction time. The reaction time may be adjusted by the amount of catalyst, reaction temperature, etc., and is typically from 30 minutes to 50 hours.

The solvent to be used in the present invention may be any solvent capable of dissolving or dispersing the Compound (I) and the Polymer (I), and examples may include: ketones, such as methyl ethyl ketone, methyl amyl ketone, diethyl ketone, acetone, methyl isopropyl ketone, methyl isobutyl ketone, cyclohexanone, 2-heptanone, etc.; ether-based solvents, such as ethyl ether, dioxane, tetrahydrofuran, 1,2-dimethoxyethane, 1,2-diethoxyethane, dipropylene glycol dimethyl ether, etc.; ester-based solvents, such as methyl acetate, ethyl acetate, n-propyl acetate, isopropyl acetate, n-butyl acetate, cyclohexyl acetate, ethyl lactate, dimethyl succinate, Texanol, etc.; cellosolve-based solvents, such as ethylene glycol monomethyl ether, ethylene glycol monoethyl ether, etc.; alcohol-based solvents, such as methanol, ethanol, iso- or n-propanol, iso- or n-butanol, amyl alcohol, etc.; ether ester-based solvents, such as ethylene glycol monomethyl ether acetate, ethylene glycol monoethyl ether acetate, propylene glycol monomethyl ether acetate, dipropylene glycol monomethyl ether acetate, 3-methoxybutyl ether acetate, ethoxyethyl ether propionate, etc.; BTX solvents, such as benzene, toluene, xylene, etc.; aliphatic hydrocarbon-based solvents, such as hexane, heptane, octane, cyclohexane, etc.; terpene hydrocarbon oils, such as turpentine oil, D-limonene, pinene, etc.; paraffinic solvents, such as mineral spirit, Swasol #310 (from Cosmo Matsuyama Oil Co., Ltd.), Solvesso #100 (from Exxon Chemical), etc.; halogenated aliphatic hydrocarbon-based solvents, such as carbon tetrachloride, chloroform, trichloroethylene, methylene chloride, 1,2-dichloroethane, etc.; halogenated aromatic hydrocarbon-based solvents, such as chlorobenzene, etc.; carbitol solvents; aniline; triethylamine, pyridine; acetic acid; acetonitrile; carbon disulfide; N,N-dimethylformamide; N,N-dimethylacetamide (DMAc); N-methylpyrrolidone; dimethyl sulfoxide; water; etc. One type of solvent may be used alone, or two or more types may be used as a mixed solvent.

Preferable among the above are, for example, ketones and ether ester-based solvents, and particularly propylene glycol monomethyl ether acetate, cyclohexanone, etc., because of excellent solubility of the Compound (I) and the Polymer (I).

The content of the solvent is preferably from 50 to 99 parts by mass, more preferably from 70 to 95 parts by mass, in 100 parts by mass of the material for forming a film for a semiconductor.

The material for forming a film for a semiconductor according to the present invention is characterized by containing the Compound (I) or the Polymer (I). By containing the Compound (I) or the Polymer (I), it is possible to obtain a film for semiconductors that has excellent heat resistance.

It is preferable that the material for forming a film for semiconductors contains, in the solid content thereof, from 20 to 100 mass% in total of the Compound (I) and the Polymer (I), because a film having excellent heat resistance can be obtained. The content thereof within the solid content is more preferably from 40 to 100 mass%, and particularly preferably from 60 to 100 mass%. Herein, "solid content" refers to components in the material for forming a film for semiconductors, excluding the solvent.

The material for forming a film for semiconductors according to the present invention may contain compound(s) other than the Compound (I). Examples may include fused ring compounds, such as fluorene compounds, bisphenol compounds, xanthene compounds, naphthalene compounds, anthracene compound, etc., and biphenyl compounds. Particularly preferable compounds among the above include the following compounds (a) to (s).

The content of the other compound(s) is preferably 20 mass% or less in the solid content.

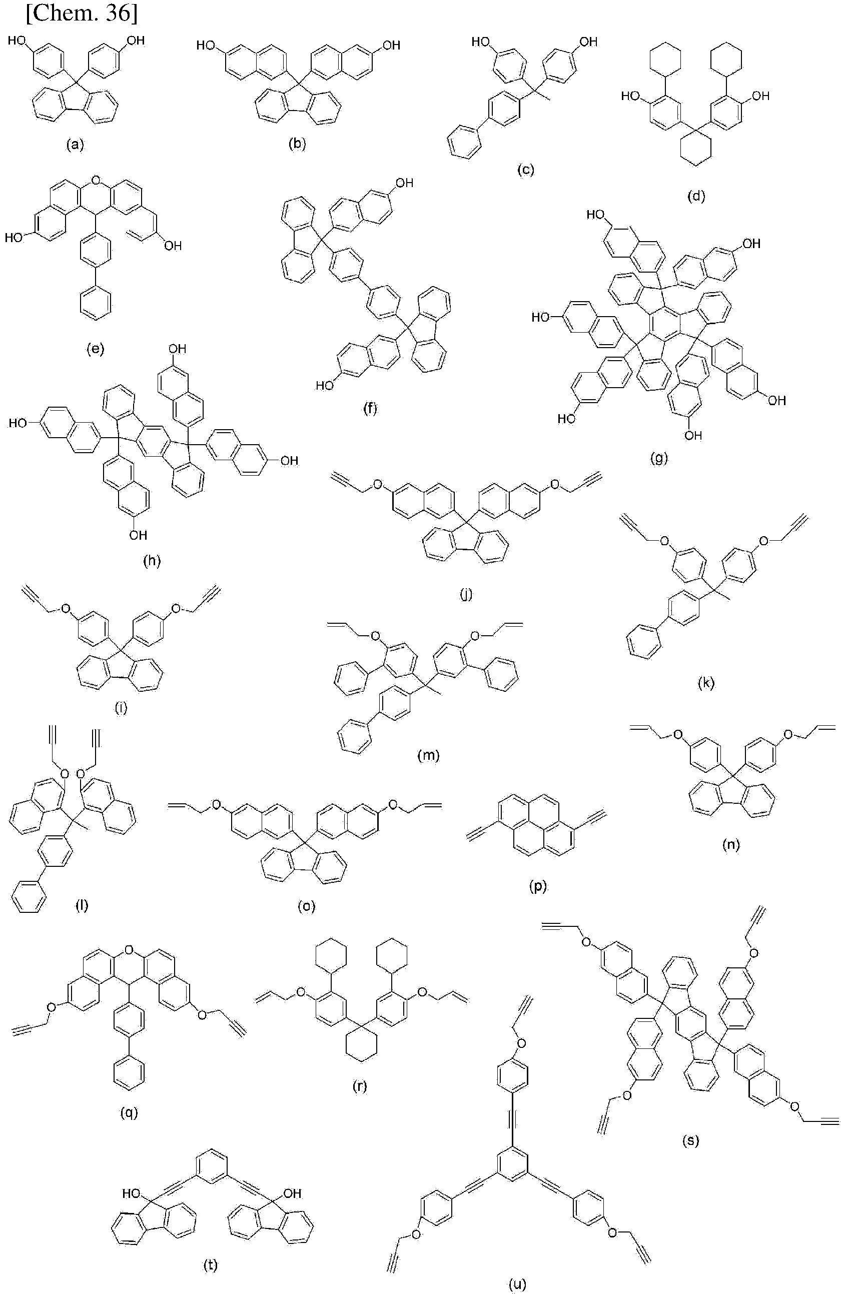

Further, the material for forming a film for semiconductors according to the present invention may contain polymer(s) other than the Polymer (I). Examples may include polymers which employ, as monomers, fused ring compounds, such as fluorene compounds, bisphenol compounds, xanthene compounds, naphthalene compounds, anthracene compound, etc., and biphenyl compounds. Particularly preferable among the above include polymers including the following structural units (u201) to (u210).

The content of the other polymer(s) is preferably 20 mass% or less in the solid content.

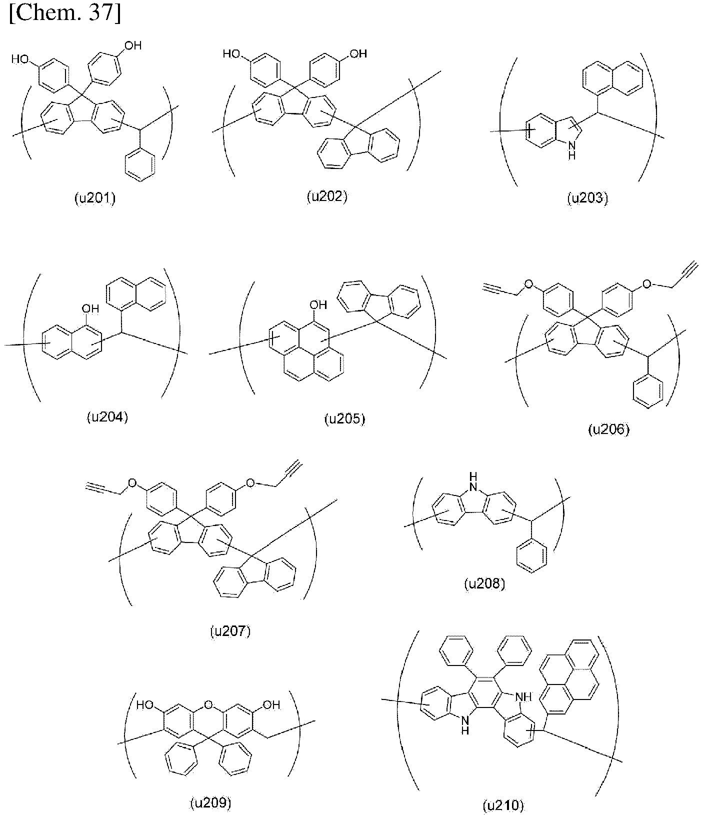

The material for forming a film for a semiconductor according to the present invention may contain a cross-linking agent. A "cross-linking agent" refers to a compound that reacts with Compound (I) and/or Polymer (I) having a reactive group, to thereby link together a plurality of molecules through chemical bonding, the compound being incorporated into the polymer after the polymerization reaction and changing the physical and/or chemical properties thereof. Containing a cross-linking agent can give a cured product having even better heat resistance. Examples of cross-linking agents may include phenol compounds, epoxy compounds, cyanate compounds, amine compounds, benzoxazine compounds, melamine compounds, guanamine compounds, glycoluril compounds, urea compounds, isocyanate compounds, azide compounds, etc.

Examples of phenol compounds may include: alkylphenols, such as phenol, cresols, xylenols, etc.; bisphenols, such as bisphenol A, bisphenol F, bis(3-methyl-4-hydroxyphenyl)propane, bis(4-hydroxyphenyl)-1-phenylethane, etc.; trisphenols, such as α,α,α'-tris(4-hydroxyphenyl)-1-ethyl-4-isopropylbenzene, etc.; phenolic resins, such as phenol novolac resin, phenol aralkyl resin, etc.; and resinous phenol derivatives, such as linear trisphenols, trisphenol methane compounds, linear tetrakisphenols, and radial hexakisphenol compounds represented by the following general formulas.

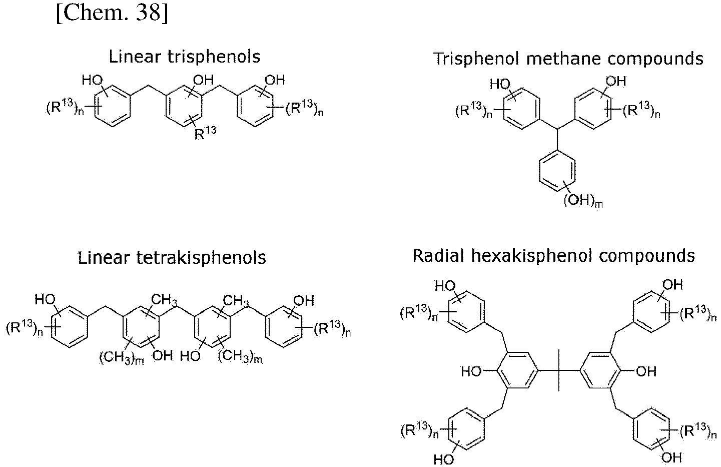

In the formulas, R¹³ represents an alkyl group having 1 to 4 carbon atoms, n represents an integer of from 0 to 2, and m represents an integer of from 0 to 1.

Examples of epoxy compounds may include glycidyl ethers of the aforementioned phenol compounds, tris(2,3-epoxypropyl)isocyanurate, trimethylolmethane triglycidyl ether, trimethylolpropane triglycidyl ether, etc.

Examples of cyanate resins may include compounds wherein a hydroxy group in the aforementioned phenol compound has been substituted by a cyanate group.

Examples of amine compounds may include: aromatic amines, such as m-phenylenediamine, p-phenylenediamine, 4,4'-diaminodiphenylmethane, 4,4'-diaminodiphenylpropane, 4,4'-diaminodiphenyl ether, 1,3-bis(4-aminophenoxy)benzene, etc.; alicyclic amines, such as diaminocyclohexane, diaminodicyclohexylmethane, diaminodicyclohexylpropane, diaminobicyclo[2.2.1]heptane, isophorone diamine, etc.; aliphatic amines, such as ethylenediamine, hexamethylenediamine, octamethylenediamine, decamethylenediamine, diethylenetriamine, triethylenetetramine, etc.

Examples of benzoxazine compounds may include P-d type benzoxazines obtained from diamine compounds and monofunctional phenol compounds, F-a type benzoxazines obtained from amine compounds and bifunctional phenol compounds.

Examples of melamine compounds may include hexamethylolmelamine, hexamethoxymethylmelamine, compounds wherein one to six of the methylol groups in hexamethylolmelamine have undergone methoxymethylation or mixtures of such compounds, hexamethoxyethylmelamine, hexaacyloxymethylmelamine, and compounds wherein one to six of the methylol groups in hexamethylolmelamine have undergone acyloxymethylation or mixtures of such compounds.

Examples of guanamine compounds may include tetramethylol guanamine, tetramethoxymethyl guanamine, compounds wherein one to four of the methylol groups in tetramethylol guanamine have undergone methoxymethylation or mixtures of such compounds, tetramethoxyethyl guanamine, tetraacyloxy guanamine, and compounds wherein one to four methylol groups in tetramethylol guanamine have undergone acyloxymethylation or mixtures of such compounds.

Examples of glycoluril compounds may include tetramethylol glycoluril, tetramethoxy glycoluril, tetramethoxymethyl glycoluril, compounds wherein one to four of the methylol groups in tetramethylol glycoluril have undergone methoxymethylation or mixtures of such compounds, and compound wherein one to four of the methylol groups in tetramethylol glycoluril have undergone acyloxymethylation or mixtures of such compounds.

Examples of urea compounds may include tetramethylol urea, tetramethoxymethyl urea, compounds wherein one to four of the methylol groups in tetramethylol urea have undergone methoxymethylation or mixtures of such compounds, tetramethoxyethyl urea, etc.

Examples of isocyanate compounds may include tolylene diisocyanate, diphenylmethane diisocyanate, hexamethylene diisocyanate, cyclohexane diisocyanate, etc. Examples of azide compounds may include 1,1'-biphenyl-4,4'-bisazide, 4,4'-methylidene bisazide, 4,4'-oxybisazide, etc.

As for the aforementioned cross-linking agents, compounds having a hydroxy group and/or a thiol group are preferable, because cured products having excellent heat resistance can be obtained. Examples may include the aforementioned phenol compounds and glycoluril compounds.

The content of the cross-linking agent is preferably from 0.1 to 20 parts by mass, more preferably from 1 to 10 parts by mass, with respect to 100 parts by mass in total of the Compound (I) and the Polymer (I). If the content is below the aforementioned range, the effect of improving heat resistance may not be achieved sufficiently.

The material for forming a film for a semiconductor according to the present invention may contain a polymerization initiator. A "polymerization initiator" refers to a compound capable of initiating polymerization by generating active species by heating, irradiation of active energy rays, etc., and various known polymerization initiators can be used. Examples of polymerization initiators may include acid generators, base generators, and radical polymerization initiators. By containing a polymerization initiator, promotion of curing of the material for forming a film for a semiconductor can be expected.

Polymerization initiators can be classified into photopolymerization initiators which generate active species by irradiation of active energy rays, and thermal polymerization initiators which generate active species by heat. Examples of photopolymerization initiators may include photo-acid generators, photo-base generators, and photo-radical polymerization initiators. Examples of thermal polymerization initiators may include thermo-acid generators, thermo-base generators, and thermal radical polymerization initiators.

To promote the curing reaction of the aforementioned cross-linking agent, the material for forming a film for a semiconductor according to the present invention may contain an acid generator as a polymerization initiator. Any compound capable of generating an acid under predetermined conditions can be employed for the acid generator, and examples may include onium salts, such as sulfonium salts, iodonium salts and ammonium salts. The acid generator may either be a thermo-acid generator which generates an acid by heat or a photo-acid generator which generates an acid by light; thermo-acid generators, which generate an acid by heat, are particularly preferable because of good curability.

Concrete examples of thermo-acid generators may include onium salts, such as bis(4-tert-butylphenyl)iodonium nonafluorobutanesulfonate, tetramethylammonium trifluoromethanesulfonate, tetramethylammonium nonafluorobutanesulfonate, triethylammonium nonafluorobutanesulfonate, pyridinium nonafluorobutanesulfonate, triethylammonium camphorsulfonate, pyridinium camphorsulfonate, tetra-n-butylammonium nonafluorobutanesulfonate, tetraphenylammonium nonafluorobutanesulfonate, tetramethylammonium p-toluenesulfonate, diphenyliodonium trifluoromethanesulfonate, (p-tert-butoxyphenyl)phenyliodonium trifluoromethanesulfonate, diphenyliodonium p-toluenesulfonate, (p-tert-butoxyphenyl)phenyliodonium p-toluenesulfonate, triphenylsulfonium trifluoromethanesulfonate, (p-tert-butoxyphenyl)diphenylsulfonium trifluoromethanesulfonate, bis(p-tert-butoxyphenyl)phenylsulfonium trifluoromethanesulfonate, tris(p-tert-butoxyphenyl)sulfonium trifluoromethanesulfonate, triphenylsulfonium p-toluenesulfonate, (p-tert-butoxyphenyl)diphenylsulfonium p-toluenesulfonate, bis(p-tert-butoxyphenyl)phenylsulfonium p-toluenesulfonate, tris(p-tert-butoxyphenyl)sulfonium p-toluenesulfonate, triphenylsulfonium nonafluorobutanesulfonate, triphenylsulfonium butanesulfonate, trimethylsulfonium trifluoromethanesulfonate, trimethylsulfonium p-toluenesulfonate, cyclohexylmethyl(2-oxocyclohexyl)sulfonium trifluoromethanesulfonate, cyclohexylmethyl(2-oxocyclohexyl)sulfonium p-toluenesulfonate, dimethylphenylsulfonium trifluoromethanesulfonate, dimethylphenylsulfonium p-toluenesulfonate, dicyclohexylphenylsulfonium trifluoromethanesulfonate, dicyclohexylphenylsulfonium p-toluenesulfonate, trinaphthylsulfonium trifluoromethanesulfonate, cyclohexylmethyl(2-oxocyclohexyl)sulfonium trifluoromethanesulfonate, (2-norbornyl)methyl(2-oxocyclohexyl)sulfonium trifluoromethanesulfonate, ethylenebis-[methyl(2-oxocyclopentyl)sulfonium trifluoromethanesulfonate], 1,2'-naphthylcarbonylmethyltetrahydrothiophenium triflate, etc.

Photo-acid generators are compounds which generate an acid by being irradiated with light. Examples of photo-acid generators include compounds which generate an acid by being exposed to radiation such as visible rays, ultraviolet rays, far-ultraviolet rays, electron beams, X rays, etc., and concrete examples may include known compounds, such as onium salt compounds, sulfone compounds, sulfonate ester compounds, quinone diazide compounds, sulfonimide compounds, diazomethane compounds, etc. Particularly, it is preferable that the photo-acid generator is at least one type selected from the group consisting of onium salt compounds, sulfonimide compounds, and diazomethane compounds, more preferably onium salt compounds, and even more preferably triarylsulfonium salts.

Examples of onium salt compounds may include diaryliodonium salts, triarylsulfonium salts, triarylphosphonium salts, etc.

Concrete examples of diaryliodonium salts may include: diphenyliodonium salts, such as diphenyliodonium tetrafluoroborate, diphenyliodonium hexafluorophosphonate, diphenyliodonium hexafluoroantimonate, diphenyliodonium hexafluoroarsenate, diphenyliodonium trifluoromethane sulfonate, diphenyliodonium trifluoroacetate, diphenyliodonium p-toluenesulfonate, etc.; 4-methoxyphenylphenyliodonium salts, such as 4-methoxyphenylphenyliodonium tetrafluoroborate, 4-methoxyphenylphenyliodonium hexafluorophosphonate, 4-methoxyphenylphenyliodonium hexafluoroantimonate, 4-methoxyphenylphenyliodonium hexafluoroarsenate, 4-methoxyphenylphenyliodonium trifluoromethane sulfonate, 4-methoxyphenylphenyliodonium trifluoroacetate, 4-methoxyphenylphenyliodonium p-toluenesulfonate, etc.; and bis(4-tert-butylphenyl)iodonium salts, such as bis(4-tert-butylphenyl)iodonium tetrafluoroborate, bis(4-tert-butylphenyl)iodonium hexafluorophosphonate, bis(4-tert-butylphenyl)iodonium hexafluoroantimonate, bis(4-tert-butylphenyl)iodonium hexafluoroarsenate, bis(4-tert-butylphenyl)iodonium trifluoromethane sulfonate, bis(4-tert-butylphenyl)iodonium trifluoroacetate, bis(4-tert-butylphenyl)iodonium p-toluenesulfonate, etc.

Examples of triarylsulfonium salts may include: triphenylsulfonium salts, such as triphenylsulfonium tetrafluoroborate, triphenylsulfonium hexafluorophosphonate, triphenylsulfonium hexafluoroantimonate, triphenylsulfonium hexafluoroarsenate, triphenylsulfonium trifluoromethane sulfonate, triphenylsulfonium trifluoroacetate, triphenylsulfonium p-toluenesulfonate, etc.; 4-methoxyphenyldiphenylsulfonium salts, such as 4-methoxyphenyldiphenylsulfonium tetrafluoroborate, 4-methoxyphenyldiphenylsulfonium hexafluorophosphonate, 4-methoxyphenyldiphenylsulfonium hexafluoroantimonate, 4-methoxyphenyldiphenylsulfonium hexafluoroarsenate, 4-methoxyphenyldiphenylsulfonium trifluoromethane sulfonate, 4-methoxyphenyldiphenylsulfonium trifluoroacetate, 4-methoxyphenyldiphenylsulfonium p-toluenesulfonate, etc.; and 4-phenylthiophenyldiphenylsulfonium salts, such as 4-phenylthiophenyldiphenylsulfonium tetrafluoroborate, 4-phenylthiophenyldiphenylsulfonium hexafluorophosphonate, 4-phenylthiophenyldiphenylsulfonium hexafluoroantimonate, 4-phenylthiophenyldiphenylsulfonium hexafluoroarsenate, 4-phenylthiophenyldiphenylsulfonium trifluoromethane sulfonate, 4-phenylthiophenyldiphenylsulfonium trifluoroacetate, 4-phenylthiophenyldiphenylsulfonium p-toluenesulfonate, etc.

Examples of triarylphosphonium salts may include: triphenylphosphonium salts, such as triphenylphosphonium tetrafluoroborate, triphenylphosphonium hexafluorophosphonate, triphenylphosphonium hexafluoroantimonate, triphenylphosphonium hexafluoroarsenate, triphenylphosphonium trifluoromethane sulfonate, triphenylphosphonium trifluoroacetate, triphenylphosphonium p-toluenesulfonate, etc.; 4-methoxyphenyldiphenylphosphonium salts, such as 4-methoxyphenyldiphenylphosphonium tetrafluoroborate, 4-methoxyphenyldiphenylphosphonium hexafluorophosphonate, 4-methoxyphenyldiphenylphosphonium hexafluoroantimonate, 4-methoxyphenyldiphenylphosphonium hexafluoroarsenate, 4-methoxyphenyldiphenylphosphonium trifluoromethane sulfonate, 4-methoxyphenyldiphenylphosphonium trifluoroacetate, 4-methoxyphenyldiphenylphosphonium p-toluenesulfonate, etc.; and tris(4-methoxyphenyl)phosphonium salts, such as tris(4-methoxyphenyl)phosphonium tetrafluoroborate, tris(4-methoxyphenyl)phosphonium hexafluorophosphonate, tris(4-methoxyphenyl)phosphonium hexafluoroantimonate, tris(4-methoxyphenyl)phosphonium hexafluoroarsenate, tris(4-methoxyphenyl)phosphonium trifluoromethane sulfonate, tris(4-methoxyphenyl)phosphonium trifluoroacetate, tris(4-methoxyphenyl)phosphonium p-toluenesulfonate, etc.

Examples of sulfonimide compounds may include: sulfonimide compounds having a N-(trifluoromethylsulfonyloxy) group, such as N-(trifluoromethylsulfonyloxy)succinimide, N-(trifluoromethylsulfonyloxy)phthalimide, N-(trifluoromethylsulfonyloxy)diphenylmaleimide, N-(trifluoromethylsulfonyloxy)bicyclo-[2,2,1]-hept-5-en-2,3-dicarboximide, N-(trifluoromethylsulfonyloxy)-7-oxabicyclo-[2,2,1]-hept-5-en-2,3-dicarboximide, N-(trifluoromethylsulfonyloxy)bicyclo-[2,2,1]-heptane-5,6-oxy-2,3-dicarboximide, N-(trifluoromethylsulfonyloxy)naphthylimide, etc.; sulfonimide compounds having a N-(camphanylsulfonyloxy) group, such as N-(camphanylsulfonyloxy)succinimide, N-(camphanylsulfonyloxy)phthalimide, N-(camphanylsulfonyloxy)diphenylmaleimide, N-(camphanylsulfonyloxy)bicyclo-[2,2,1]-hept-5-en-2,3-dicarboximide, N-(camphanylsulfonyloxy)-7-oxabicyclo-[2,2,1]-hept-5-en-2,3-dicarboximide, N-(camphanylsulfonyloxy)bicyclo-[2,2,1]-heptane-5,6-oxy-2,3-dicarboximide, N-(camphanylsulfonyloxy)naphthylimide, etc.; sulfonimide compounds having a N-(4-methylphenylsulfonyloxy) group, such as N-(4-methylphenylsulfonyloxy)succinimide, N-(4-methylphenylsulfonyloxy)phthalimide, N-(4-methylphenylsulfonyloxy)diphenylmaleimide, N-(4-methylphenylsulfonyloxy)bicyclo-[2,2,1]-hept-5-en-2,3-dicarboximide, N-(4-methylphenylsulfonyloxy)-7-oxabicyclo-[2,2,1]-hept-5-en-2,3-dicarboximide, N-(4-methylphenylsulfonyloxy)bicyclo-[2,2,1]-heptane-5,6-oxy-2,3-dicarboximide, N-(4-methylphenylsulfonyloxy)naphthylimide, etc.; and sulfonimide compounds having a N-(2-trifluoromethylphenylsulfonyloxy) group, such as N-(2-trifluoromethylphenylsulfonyloxy)succinimide, N-(2-trifluoromethylphenylsulfonyloxy)phthalimide, N-(2-trifluoromethylphenylsulfonyloxy)diphenylmaleimide, N-(2-trifluoromethylphenylsulfonyloxy)bicyclo-[2,2,1]-hept-5-en-2,3-dicarboximide, N-(2-trifluoromethylphenylsulfonyloxy)-7-oxabicyclo-[2,2,1]-hept-5-en-2,3-dicarboximide, N-(2-trifluoromethylphenylsulfonyloxy)bicyclo-[2,2,1]-heptane-5,6-oxy-2,3-dicarboximide, N-(2-trifluoromethylphenylsulfonyloxy)naphthylimide, etc.

Examples of diazomethane compounds may include bis(trifluoromethylsulfonyl)diazomethane, bis(cyclohexylsulfonyl)diazomethane, bis(phenylsulfonyl)diazomethane, bis(p-toluenesulfonyl)diazomethane, methylsulfonyl-p-toluenesulfonyl diazomethane, 1-cyclohexylsulfonyl-1-1,1-dimethylethylsulfonyl)diazomethane, bis(1,1-dimethylethylsulfonyl)diazomethane, etc.

Commercially available products may be used for the photo-acid generator. Examples of commercially available photocation polymerization initiators may include: "KAYARAD (registered trademark) PCI-220" and "KAYARAD (registered trademark) PCI-620" from Nippon Kayaku Co., Ltd.; "UVI-6990" from Dow Chemical Company; "ADEKA ARKLS (registered trademark) SP-150", "ADEKA ARKLS (registered trademark) SP-170", "ADEKA ARKLS (registered trademark) SP-500", and "ADEKA ARKLS (registered trademark) SP-606" from Adeka Corporation; "CI-5102", "CIT-1370", "CIT-1682", "CIP-1866S", "CIP-2048S", and "CIP-2064S" from Nippon Soda Co., Ltd.; "DPI-101", "DPI-102", "DPI-103", "DPI-105", "MPI-103", "MPI-105", "BBI-101", "BBI-102", "BBI-103", "BBI-105", "TPS-101", "TPS-102", "TPS-103", "TPS-105", "MDS-103", "MDS-105", "DTS-102", and "DTS-103" from Midori Kagaku Co., Ltd.; "PI-2074" from Solvay Japan, Ltd.; and CPI-100P from San-Apro Ltd.

The content of the acid generator is preferably from 0.001 to 50 parts by mass, more preferably from 0.01 to 20 parts by mass, with respect to 100 parts by mass of the total solid content. When the content is within the aforementioned range, it is possible to obtain a composition having excellent curability and to obtain a cured product having excellent heat resistance and solvent resistance.

To promote the curing reaction of the cross-linking agent, the composition of the present invention may contain a base generator as a polymerization initiator. Any compound capable of generating a base under predetermined conditions can be employed for the base generator, and it may either be a thermo-base generator which generates a base by heat or a photo-base generator which generates a base by light; thermo-base generators, which generate a base by heat, are particularly preferable because of good curability.

Examples of thermo-base generators may include: carbamate derivatives, such as 2-(4-biphenyl)-2-propyl carbamate, 1,1-dimethyl-2-cyanoethyl carbamate, etc.; urea and urea derivatives, such as N,N,N'-trimethylurea, etc.; dihydropyridine derivatives, such as 1,4-dihydronicotinamide, etc.; dicyandiamide; salts, such as organic salts and inorganic salts, consisting of an acid and a base; etc.

Examples of photo-base generators may include carbamate compounds, α-aminoketone compounds, quaternary ammonium compounds, O-acyloxime compounds, aminocyclopropenone compounds, etc.

Examples of carbamate compounds may include 1-(2-anthraquinonyl)ethyl 1-piperidine carboxylate, 1-(2-anthraquinonyl)ethyl 1H-2-ethylimidazole-1-carboxylate, 9-anthrylmethyl 1-piperidine carboxylate, 9-anthrylmethyl N,N-diethylcarbamate, 9-anthrylmethyl N-propylcarbamate, 9-anthrylmethyl N-cyclohexylcarbamate, 9-anthrylmethyl 1H-imidazole-1-carboxylate, 9-anthrylmethyl N,N-dioctylcarbamate, 9-anthrylmethyl 1-(4-hydroxypiperidine)carboxylate, 1-pyrenyl methyl 1-piperidine carboxylate, bis[1-(2-anthraquinonyl)ethyl] 1,6-hexane-diylbiscarbamate, bis(9-anthrylmethyl) 1,6-hexane-diylbiscarbamate, etc.

Examples of α-aminoketone compounds may include 1-phenyl-2-(4-morpholinobenzoyl)-2-dimethylaminobutane, 2-(4-methylthiobenzoyl)-2-morpholinopropane, etc.

Examples of quaternary ammonium compounds that may serve as a photo-base generator may include 1-(4-phenylthiophenacyl)-1-azonia-4-azabicyclo[2,2,2]octane tetraphenylborate, 5-(4-phenylthiophenacyl)-1-aza-5-azoniabicyclo[4,3,0]-5-nonene tetraphenylborate, 8-(4-phenylthiophenacyl)-1-aza-8-azoniabicyclo[5,4,0]-7-undecene tetraphenylborate, etc.

Examples of aminocyclopropenone compounds that may serve as a photo-base generator may include 2-diethylamino-3-phenylcyclopropenone, 2-diethylamino-3-(1-naphthyl)cyclopropenone, 2-pyrrolidinyl-3-phenylcyclopropenone, 2-imidazolyl-3-phenylcyclopropenone, 2-isopropylamino-3-phenylcyclopropenone, etc.

The content of the base generator is preferably from 0.001 to 50 parts by mass, more preferably from 0.01 to 20 parts by mass, with respect to 100 parts by mass of the total solid content. When the content is within the aforementioned range, it is possible to obtain a composition having excellent curability and to obtain a cured product having excellent heat resistance and solvent resistance.

In cases where the aforementioned Polymer (I) has an ethylenically unsaturated bond as a reactive group, the composition of the present invention may contain a radical polymerization initiator as a polymerization initiator. For the radical polymerization initiator, it is possible to use either a photo-radical polymerization initiator or a thermal radical polymerization initiator.

Examples of photo-radical polymerization initiators may include: benzoins, such as benzoin, benzoin methyl ether, benzoin propyl ether, benzoin butyl ether, etc.; benzyl ketals, such as benzyl dimethyl ketal, etc.; acetophenones, such as acetophenone, 2,2-dimethoxy-2-phenylacetophenone, 2,2-diethoxy-2-phenylacetophenone, 1-benzyl-1-dimethylamino-1-(4'-morpholinobenzoyl)propane, 2-morpholyl-2-(4'-methylmercapto)benzoylpropane, 2-methyl- 1-[4-(methylthio)phenyl]-2-morpholino-propan- 1-one, 1-hydroxycyclohexyl phenyl ketone, 1-hydroxy-1-benzoylcyclohexane, 2-hydroxy-2-benzoylpropane, 2-hydroxy-2-(4'-isopropyl)benzoylpropane, N,N-dimethylaminoacetophenone, 1,1-dichloroacetophenone, 4-butylbenzoyltrichloromethane, 4-phenoxybenzoyldichloromethane, etc.; anthraquinones, such as 2-methylanthraquinone, 1-chloroanthraquinone, 2-amylanthraquinone, etc.; thioxanthones, such as 2,4-dimethylthioxanthone, 2,4-diethylthioxanthone, 2-chlorothioxanthone, 2,4-diisopropylthioxanthone, etc.; ketals, such as acetophenone dimethyl ketal, benzyl dimethyl ketal, etc.; benzophenones, such as benzophenone, methylbenzophenone, 4,4'-dichlorobenzophenone, 4,4'-bisdiethylaminobenzophenone, Michler's ketone, 4-benzoyl-4'-methyldiphenylsulfide, etc.; oxides, such as 2,4,6-trimethylbenzoyldiphenylphosphine oxide, bis(2,4,6-trimethylbenzoyl)-phenylphosphine oxide, etc.; carbazoles, such as 3-(2-methyl-2-morpholinopropionyl)-9-methylcarbazole, etc.; α-dicarbonyls, such as benzil, methyl benzoylformate, etc.; oxime esters, such as compounds disclosed in JP 2000-80068A, JP 2001-233842A, JP 2005-97141A, JP 2006-516246T, Japanese Patent No. 3860170, Japanese Patent No. 3798008, WO2006/018973, JP 2011-132215A, and WO2015/152153; triazines, such as p-methoxyphenyl-2,4-bis(trichloromethyl)-s-triazine, 2-methyl-4,6-bis(trichloromethyl)-s-triazine, 2-phenyl-4,6-bis(trichloromethyl)-s-triazine, 2-naphthyl-4,6-bis(trichloromethyl)-s-triazine, 2-(p-butoxystyryl)-s-triazine, etc.; and benzoyl peroxide, 2,2'-azobis-isobutyronitrile, ethylanthraquinone, 1,7-bis(9'-acridinyl)heptane, thioxanthone, 1-chloro-4-propoxythioxanthone, isopropylthioxanthone, diethylthioxanthone, benzophenone, phenyl biphenyl ketone, 4-benzoyl-4'-methyldiphenyl sulfide, 2-(p-butoxystyryl)-5-trichloromethyl-1,3,4-oxadiazole, 9-phenylacridine, 9,10-dimethylbenzphenazine, benzophenone/Michler's ketone, hexaarylbiimidazole/mercaptobenzimidazole, thioxanthone/amine, etc.

Examples of thermal radical polymerization initiators may include: peroxides, such as benzoyl peroxide, 2,4-dichlorobenzoyl peroxide, 1,1-di(t-butylperoxy)-3,3,5-trimethylcyclohexane, 4,4-di(t-butylperoxy)butyl valerate, dicumyl peroxide, etc.; azocompounds, such as 2,2'-azobis-isobutyronitrile, etc.; and tetramethylthiuram disulfide, etc.

The content of the radical polymerization initiator is preferably from 0.1 to 20 parts by mass, more preferably from 0.5 to 15 parts by mass, even more preferably from 1 to 10 parts by mass, with respect to 100 parts by mass in total of the Polymer (I) and Compound (I) having a carbon-carbon double bond, because it is possible to obtain a composition having excellent curability and capable of providing a cured product having excellent heat resistance and solvent resistance.

The material for forming a film for a semiconductor according to the present invention may contain, as necessary, other components in addition to the aforementioned components.

Examples of other components may include various additives, such as inorganic fillers, organic fillers, silane coupling agents, coloring agents, photosensitizers, antifoaming agents, thickeners, thixotropic agents, surfactants, leveling agents, flame retardants, plasticizers, stabilizers, polymerization inhibitors, UV absorbers, antioxidants, antistatic agents, flow modifiers, adhesion accelerators, etc.

The material for forming a film for a semiconductor according to the present invention can be cured by being heated with, for example, a heated plate such as a hot plate etc., an atmospheric oven, an inert gas oven, a vacuum oven, a convection oven, etc.

The heating temperature at the time of thermal curing can be selected as appropriate depending on the type of reactive group. For example, in cases where the reactive functional group is a carbon-carbon triple bond, the temperature is preferably from 200°C to 400°C, more preferably from 250°C to 350°C. The curing time is not particularly limited, but is preferably from 1 to 60 minutes, more preferably from 1 to 30 minutes, from the viewpoint of improving productivity.

The material for forming a film for a semiconductor according to the present invention is capable of providing a film having excellent heat resistance and solvent resistance. Hence, the present material is useful because it is possible to form a film for semiconductors, which is required to have high heat resistance and solvent resistance, and the material is particularly useful as an underlayer film forming material.

### Examples

The present invention will be described in further detail below according to Examples and Comparative Examples. The invention, however, is not to be limited thereto.

### {Production Example 1}

A reflux condenser-equipped reaction flask was charged with 29.3 g (250 mmol) of indole, 30.5 g (250 mmol) of 3-hydroxybenzaldehyde and 166 g of acetonitrile, and while stirring, the solute was dissolved completely. The mixture was water-cooled, and then 4.2 g (25 mmol) of 48% hydrobromic acid was dropped slowly. After confirming that generation of heat has subsided, the mixture was returned to room temperature and was stirred as-is for 2 hours. The reaction liquid was cooled to 10°C, and the precipitate was filtered and washed with 70 g of acetonitrile, and then the filtered residue was dried at 40°C under reduced pressure, to obtain Compound (M1) in the form of a pale-yellow powder.

Yield amount: 28.5 g (yield rate: 54%).

¹H-NMR (DMSO-d₆): δ (ppm) = 5.57 (s, 2H), 6.59-7.25 (m, 16H), 9.16 (s, 2H), 10.66 (s, 2H).

A reflux condenser-equipped reaction flask was charged with 26.6 g (60 mmol) of Compound (M1), 18.3 g (72 mmol) of iodine and 80 g of acetonitrile. The temperature was raised, and reaction was conducted at 80°C for 14 hours. The reaction liquid was cooled to room temperature, and 25 g of 10% sodium thiosulfate aqueous solution was dropped, and the mixture was cooled to 10°C while being stirred. The precipitate was filtered and washed with 50 g of acetonitrile, and then the filtered residue was dried at 40°C under reduced pressure, to obtain Compound (A1) in the form of a pale-yellow powder.

Yield amount: 20.7 g (yield rate: 78%).

¹H-NMR (DMSO-d₆): δ (ppm) = 6.83-7.49 (m, 16H), 9.68 (s, 2H), 10.49 (s, 2H).

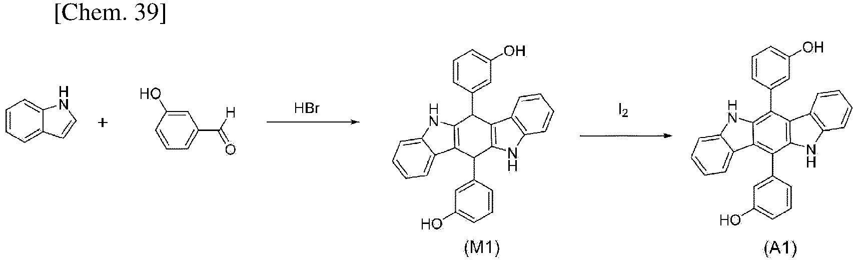

### {Production Example 2}

A reflux condenser-equipped reaction flask was charged with 40.0 g (341 mmol) of indole, 47.2 g (341 mmol) of 2,5-dihydroxybenzaldehyde and 243 g of acetonitrile, and while stirring, the solute was dissolved completely. The mixture was water-cooled, and then 5.7 g (34 mmol) of 48% hydrobromic acid was dropped slowly. After confirming that generation of heat has subsided, the mixture was returned to room temperature and was stirred as-is for 2 hours. The reaction liquid was cooled to 10°C, and the precipitate was filtered and washed with 100 g of acetonitrile, and then the filtered residue was dried at 40°C under reduced pressure, to obtain Compound (M2) in the form of a gray powder.

Yield amount: 33.2 g (yield rate: 41%).

¹H-NMR (DMSO-d₆): δ (ppm) = 6.00 (s, 2H), 6.04-7.33 (m, 14H), 8.27 (s, 2H), 9.25 (s, 2H), 10.42 (s, 2H).

A reflux condenser-equipped reaction flask was charged with 30.0 g (63.2 mmol) of Compound (M2), 19.3 g (75.9 mmol) of iodine and 90 g of acetonitrile. The temperature was raised, and reaction was conducted at 80°C for 14 hours. The reaction liquid was cooled to room temperature, 30 g of 10% sodium thiosulfate aqueous solution was dropped, and the mixture was cooled to 10°C while being stirred. The precipitate was filtered and washed with 50 g of acetonitrile, and then the filtered residue was dried at 40°C under reduced pressure, to obtain Compound (A2) in the form of a pale-brown powder.

Yield amount: 29.7 g (yield rate: 99%).

¹H-NMR (DMSO-d₆): δ (ppm) = 6.80-7.43 (m, 14H), 8.60 (s, 2H), 8.89 (s, 2H), 10.27 (s, 2H).

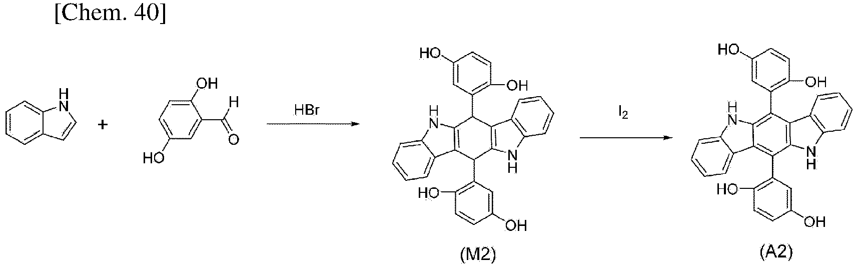

### {Production Example 3}

A reflux condenser-equipped reaction flask was charged with 6.61 g (15 mmol) of Compound (A1) and 60 g of dimethylsulfoxide (DMSO), and while stirring, 5.29 g (66 mmol) of 48% sodium hydroxide aqueous solution was dropped at room temperature under a flow of nitrogen gas. Next, 7.55 g (63 mmol) of 3-bromo-1-propyne was dropped, and stirring was conducted as-is at room temperature for 2 hours. The reaction liquid was placed into 350 g of water and stirred for 30 minutes, and then the precipitate was filtered. The filtered residue collected was dissolved in 50 g of ethyl acetate, and then 50 g of water was added thereto and stirred for 30 minutes, which was followed by oil-water separation. The organic layer was subjected to solvent removal, and the residue was dried at 40°C under reduced pressure, to obtain Compound (A3) in the form of a pale-brown powder.

Yield amount: 3.8 g (yield rate: 42%).

¹H-NMR (DMSO-d₆): δ (ppm) = 3.10 (t, 2H), 3.56 (t, 2H), 4.65 (d, 4H), 4.92 (d, 4H), 6.61-7.69 (m, 16H).

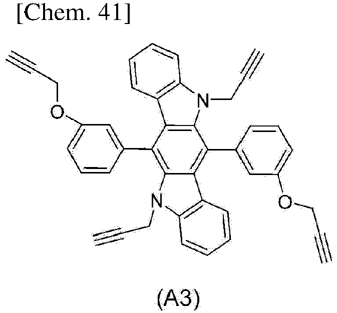

### {Production Example 4}

A reflux condenser-equipped reaction flask was charged with 5.20 g (11 mmol) of Compound (A2) and 50 g of tetrahydrofuran (THF), and while stirring, 3.67 g (44 mmol) of 48% sodium hydroxide aqueous solution was dropped at room temperature under a flow of nitrogen gas. Next, 5.00 g (42 mmol) of 3-bromo-1-propyne was dropped, and stirring was conducted as-is at room temperature for 1 hour. The reaction liquid was placed into 300 g of water and stirred for 30 minutes, and then the precipitate was filtered. Then, 50 g of isopropanol was added to the filtered residue collected and stirred for 30 minutes, which was followed by filtering. The residue was dried at 40°C under reduced pressure, to obtain Compound (A4) in the form of a pale-brown powder.

Yield amount: 1.3 g (yield rate: 20%).

¹H-NMR (DMSO-d₆): δ (ppm) = 3.39 (t, 2H), 3.53 (t, 2H), 4.61 (d, 4H), 6.82 (t, 2H), 7.09-7.43 (m, 12H), 10.41 (s, 2H).

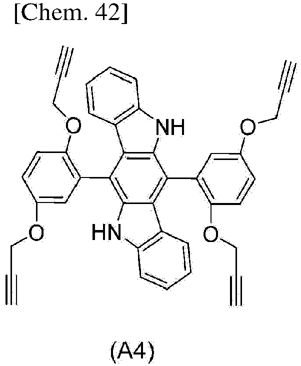

### {Production Example 5}

A reflux condenser-equipped reaction flask was charged with 5.20 g (11 mmol) of Compound (A2) and 50 g of dimethylsulfoxide (DMSO), and while stirring, 5.50 g (66 mmol) of 48% sodium hydroxide aqueous solution was dropped at room temperature under a flow of nitrogen gas. Next, 7.50 g (63 mmol) of 3-bromo-1-propyne was dropped, and stirring was conducted as-is at room temperature for 2 hours. The reaction liquid was placed into 300 g of water and stirred for 30 minutes, and then the precipitate was filtered. The filtered residue collected was dissolved in 50 g of ethyl acetate, and then 50 g of water was added thereto and stirred for 30 minutes, which was followed by oil-water separation. The organic layer was subjected to solvent removal, and the residue was dried at 40°C under reduced pressure, to obtain Compound (A5) in the form of a pale-brown powder.

Yield amount: 4.90 g (yield rate: 65%).

¹H-NMR (DMSO-d₆): δ (ppm) = 3.09 (t, 2H), 3.44 (t, 2H), 3.52 (t, 2H), 4.65-4.85 (m, 12H), 6.72-7.49 (m, 14H).

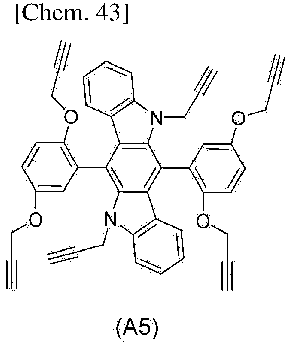

### {Production Example 6}

A reflux condenser-equipped reaction flask was charged with 30.0 g (256 mmol) of indole, 52.0 g (256 mmol) of 4-(trimethylsilyl)ethynylbenzaldehyde and 230 g of acetonitrile, and while stirring under water cooling, 9.0 g (52.0 mmol) of 48% hydrobromic acid was dropped slowly. The reaction liquid was returned to room temperature and was stirred as-is for 2 hours. The reaction liquid was cooled to 10°C, and the precipitate was filtered and washed with 100 g of acetonitrile, and then the filtered residue was dried at 40°C under reduced pressure, to obtain Compound (M3) in the form of a pale-yellow powder.

Yield amount: 25.0 g (yield rate: 33%).

A reflux condenser-equipped reaction flask was charged with 22.0 g (36 mmol) of Compound (M3), 10.0 g (40 mmol) of chloranil and 880 g of o-xylene. The temperature was raised, and reaction was conducted at 100°C for 30 hours. The reaction liquid was cooled to room temperature, and after solvent removal, 200 g of methanol was added to the residue, and stirring was conducted at room temperature for 30 minutes. The precipitate was filtered and washed twice with 200 g of methanol, and the filtered residue was dried at 40°C under reduced pressure, to obtain Compound (M4) in the form of a pale-yellow powder.

Yield amount: 10.9 g (yield rate: 50%).

A reflux condenser-equipped reaction flask was charged with 9.0 g (15 mmol) of Compound (M4), 80 g of tetrahydrofuran and 80 g of methanol, and stirring was conducted at room temperature for 30 minutes. Then, 2.3 g (16 mmol) of potassium carbonate was added, and reaction was conducted at room temperature for 3 hours. To the reaction liquid, 20 g of ion-exchanged water was added, and the precipitate was filtered. The filtered residue was washed three times with 20 g of methanol, and the filtered residue was dried at 40°C under reduced pressure, to obtain Compound (A6) in the form of a pale-yellow powder.

Yield amount: 6.1 g (yield rate: 88%).

¹H-NMR (DMSO-d₆): δ (ppm) = 4.37 (s, 2H), 6.88-7.83 (m, 16H), 10.64 (s, 2H).

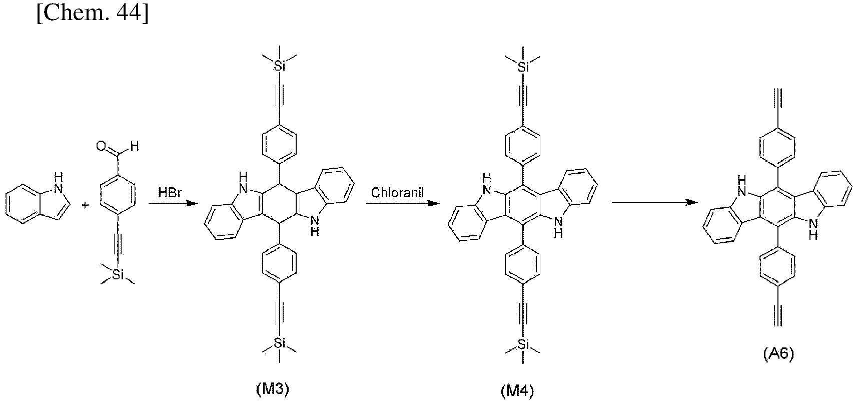

### {Production Example 7}

A reflux condenser-equipped reaction flask was charged with 11.7 g (100 mmol) of indole, 13.1 g (100 mmol) of 3-formylbenzonitrile and 50 g of acetonitrile, and while stirring, the solute was dissolved completely. The mixture was water-cooled, and then 1.7 g (10 mmol) of 48% hydrobromic acid was dropped slowly. The reaction liquid was returned to room temperature and was stirred as-is for 2 hours. The reaction liquid was cooled to 10°C, and the precipitate was filtered and washed with 20 g of acetonitrile, and then the filtered residue was dried at 40°C under reduced pressure, to obtain Compound (M5) in the form of a gray powder.

Yield amount: 3.8 g (yield rate: 16.5%).

¹H-NMR (DMSO-d₆): δ (ppm) = 5.89 (s, 2H), 6.83-8.08 (m, 16H), 10.91 (s, 2H).

A reflux condenser-equipped flask was charged with 2.12 g (4.6 mmol) of Compound (M5) and 20 g of dimethylsulfoxide (DMSO), and while stirring, 1.00 g (12.0 mmol) of 48% sodium hydroxide aqueous solution was dropped at room temperature under a flow of nitrogen gas. Next, 1.41 g (12.0 mmol) of 3-bromo-1-propyne was dropped, and stirring was conducted as-is at room temperature for 2 hours. The reaction liquid was placed into 120 g of water and stirred for 30 minutes, and then the precipitate was filtered. To the filtered residue collected, 40 g of methanol was added and stirred for 30 minutes. The suspension was filtered, and the filtered residue was dried at 40°C under reduced pressure, to obtain Compound (A7) in the form of a pale-orange powder.

Yield amount: 1.5 g (yield rate: 61%).

¹H-NMR (DMSO-d₆): δ (ppm) = 2.89 (t, 2H), 4.81 (d, 2H), 5.03 (d, 2H), 6.19 (s, 2H), 7.00-7.94 (m, 16H).

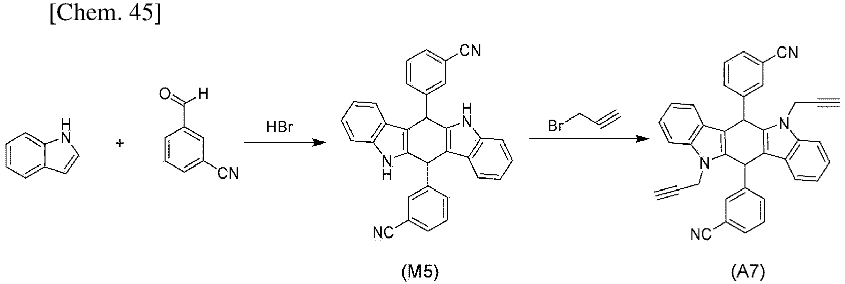

### {Production Example 8}

A reflux condenser-equipped reaction flask was charged with 11.7 g (100 mmol) of indole, 19.4 g (100 mmol) of 2-fluorene carboxaldehyde and 90 g of acetonitrile, and while stirring, the solute was dissolved completely. The mixture was water-cooled, and then 1.7 g (10 mmol) of 48% hydrobromic acid was dropped slowly. The reaction liquid was returned to room temperature and was stirred as-is for 2 hours. The reaction liquid was cooled to 10°C, and the precipitate was filtered and washed with 20 g of acetonitrile, and then the filtered residue was dried at 40°C under reduced pressure, to obtain Compound (M6) in the form of a brown powder.

Yield amount: 27.0 g (yield rate: 92.1%).

¹H-NMR (DMSO-d₆): δ (ppm) = 3.84 (s, 4H), 5.85 (s, 2H), 6.77-7.87 (m, 22H), 10.76 (s, 2H).

A reflux condenser-equipped reaction flask was charged with 2.35 g (4.0 mmol) of Compound (M6) and 30 g of dimethylsulfoxide (DMSO), and while stirring, 3.0 g (36.0 mmol) of 48% sodium hydroxide aqueous solution was dropped at room temperature under a flow of nitrogen gas. Next, 4.25 g (36.0 mmol) of 3-bromo-1-propyne was dropped, and stirring was conducted as-is at room temperature for 3 hours. The reaction liquid was placed into 120 g of water and stirred for 30 minutes, and then the precipitate was filtered. To the filtered residue collected, 20 g of methanol was added and stirred for 30 minutes, which was then filtered. Thereafter, the filtered residue was dried at 40°C under reduced pressure, to obtain Compound (A8) in the form of a pale-orange powder.

Yield amount: 1.6 g (yield rate: 49%).

¹H-NMR (DMSO-d₆): δ (ppm) = 2.47 (d, 8H), 2.94 (t, 4H), 3.05 (t, 2H), 4.72 (d, 2H), 4.92 (d, 2H), 6.10 (s, 2H), 6.90-8.34 (m, 22H).

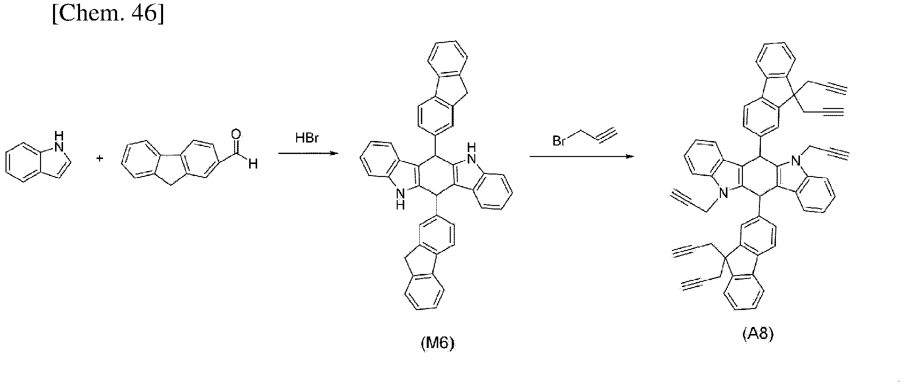

### {Production Example 9}

A reflux condenser-equipped flask was charged with 3.10 g (7.0 mmol) of Compound (M1) and 20 g of dimethylsulfoxide (DMSO), and while stirring, 2.92 g (35.0 mmol) of 48% sodium hydroxide aqueous solution was dropped at room temperature under a flow of nitrogen gas. Next, 4.23 g (35.0 mmol) of 3-bromo-1-propene was dropped, and stirring was conducted as-is at room temperature for 3 hours. The reaction liquid was placed into 100 g of water and stirred for 30 minutes, and then the precipitate was filtered. To the filtered residue collected, 30 g of methanol was added and stirred for 30 minutes. The suspension was filtered, and the filtered residue was dried at 40°C under reduced pressure, to obtain Compound (A9) in the form of a pale-yellow powder.

Yield amount: 3.0 g (yield rate: 71%).

¹H-NMR (DMSO-d₆): δ (ppm) = 4.52 (m, 4H), 4.89 (m, 4H), 5.17-5.34 (m, 10H), 5.89 (s, 2H), 5.96 (m, 2H), 6.72-7.46 (m, 16H).

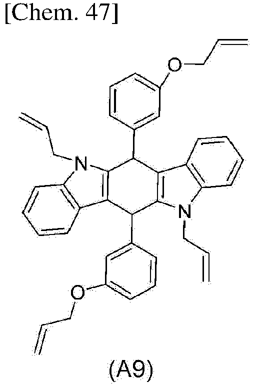

### {Production Example 10}

A reflux condenser-equipped reaction flask was charged with 2.20 g of Compound (M2), 0.54 g of benzaldehyde and 24 g of PGMEA, and 0.14 g of methanesulfonic acid was added to the mixture being stirred. The temperature was raised under a flow of nitrogen gas, and stirring was conducted at 140°C for 20 hours. After cooling the mixture to room temperature and removing insoluble matter, the reaction liquid was dropped into 120 g of a mixed solvent of n-hexane and 2-propanol (2:1 in ratio) and was stirred for 30 minutes. The precipitate was filtered, and the filtered residue was dried at 60°C under reduced pressure, to obtain 2.2 g of a brown powder of Polymer (A10). As regards the molecular weight (in terms of polystyrene) measured by GPC, the weight-average molecular weight was 1,450 and the dispersity was 1.25.

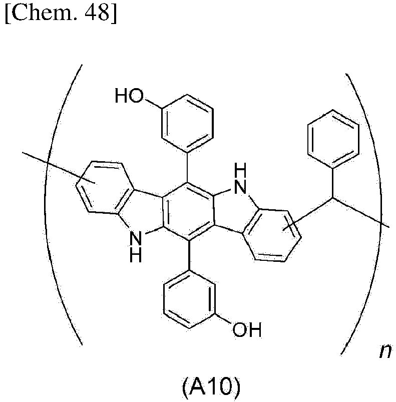

### {Production Example 11}

A reflux condenser-equipped reaction flask was charged with 2.20 g of Compound (M2), 0.90 g of fluorenone and 24 g of N-methylpyrrolidone, and 0.32 g of methanesulfonic acid was added to the mixture being stirred. The temperature was raised under a flow of nitrogen gas, and stirring was conducted at 140°C for 20 hours. After cooling the mixture to room temperature and removing insoluble matter, the reaction liquid was dropped into 120 g of a mixed solvent of n-hexane and 2-propanol (2:1 in ratio) and was stirred for 30 minutes. The precipitate was filtered, and the filtered residue was dried at 60°C under reduced pressure, to obtain 2.3 g of a black powder of Polymer (A11). As regards the molecular weight (in terms of polystyrene) measured by GPC, the weight-average molecular weight was 1,990 and the dispersity was 1.07.

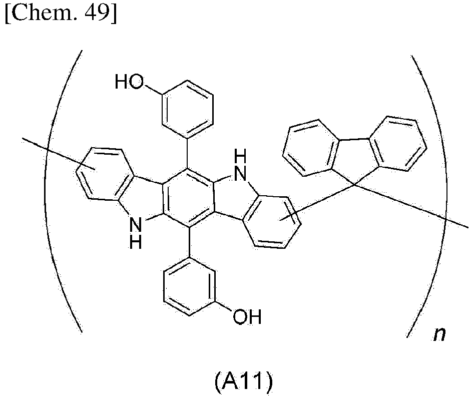

### {Production Example 12}

A reflux condenser-equipped reaction flask was charged with 2.10 g of Compound (A5), 0.32 g of benzaldehyde and 24 g of PGMEA, and 0.20 g of methanesulfonic acid was added to the mixture being stirred. The temperature was raised under a flow of nitrogen gas, and stirring was conducted at 100°C for 20 hours. After cooling the mixture to room temperature and removing insoluble matter, the reaction liquid was dropped into 100 g of a mixed solvent of n-hexane and 2-propanol (2:1 in ratio) and was stirred for 30 minutes. The precipitate was filtered, and the filtered residue was dried at 60°C under reduced pressure, to obtain 1.7 g of a blackish brown powder of Polymer (A12). As regards the molecular weight (in terms of polystyrene) measured by GPC, the weight-average molecular weight was 2,640 and the dispersity was 1.17.

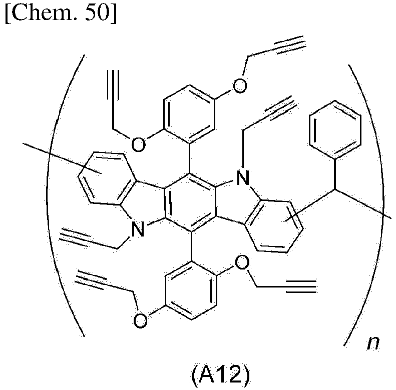

### {Production Example 13}

A reflux condenser-equipped reaction flask was charged with 2.85 g of Compound (M6), 0.37 g of benzaldehyde and 30 g of propylene glycol monomethyl ether acetate (PGMEA), and 0.22 g of methanesulfonic acid was added to the mixture being stirred. The temperature was raised under a flow of nitrogen gas, and stirring was conducted at 100°C for 20 hours. After cooling the mixture to room temperature and removing insoluble matter, the reaction liquid was dropped into 150 g of a mixed solvent of n-hexane and 2-propanol (2:1 in ratio) and was stirred for 30 minutes. The precipitate was filtered, and the filtered residue was dried at 60°C under reduced pressure, to obtain 1.5 g of a brown powder of Polymer (A13). As regards the molecular weight (in terms of polystyrene) measured by GPC, the weight-average molecular weight was 2,350 and the dispersity was 1.17.

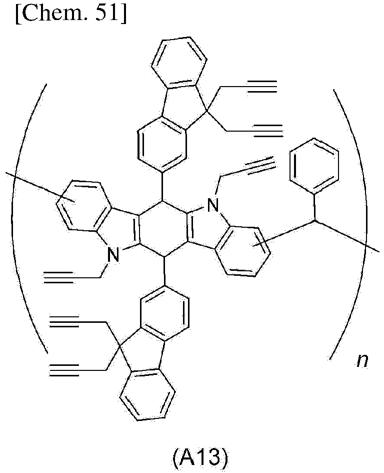

### {Examples 1 to 23 and Comparative Examples 1 to 6}

According to the respective makeup (parts by mass) shown in Tables 1 to 3, components were measured and mixed, and were then stirred to dissolve. After confirming that the solid had dissolved completely, the mixture was filtered with a fluorocarbon resin-made filter (pore size: 0.2 µm), to prepare a composition for evaluation.

The components in the Tables are as follows.
A1: Compound (A1); Compound (I).
A2: Compound (A2); Compound (I).
A3: Compound (A3); Compound (I).
A4: Compound (A4); Compound (I).
A5: Compound (A5); Compound (I).
A6: Compound (A6); Compound (I).
A7: Compound (A7); Compound (I).
A8: Compound (A8); Compound (I).
A9: Compound (A9); Compound (I).
A10: Polymer (A10); Polymer (I) and Polymer (II).
A11: Polymer (A11); Polymer (I) and Polymer (II).
A12: Polymer (A12); Polymer (I) and Polymer (II).
A13: Polymer (A13); Polymer (I) and Polymer (II).
A14: Compound (A14); Compound other than Compound (I).
A15: Compound (A15); Compound other than Compound (I).
A16: Compound (A16); Compound other than Compound (I).
A17: Compound (A17); Compound other than Compound (I).
A18: Polymer (A18); Polymer including, as monomer, compound other than Compound (I).
B1: Compound (B1) below; Cross-linking agent (glycoluril compound).
B2: Compound (B2) below; Cross-linking agent (phenol compound).
C1: Bis(4-tert-butylphenyl)iodonium nonafluorobutanesulfonate (polymerization initiator: thermo-acid generator)
D1: Propylene glycol monomethyl ether acetate (PGMEA); solvent.
D2: Cyclohexanone; solvent.

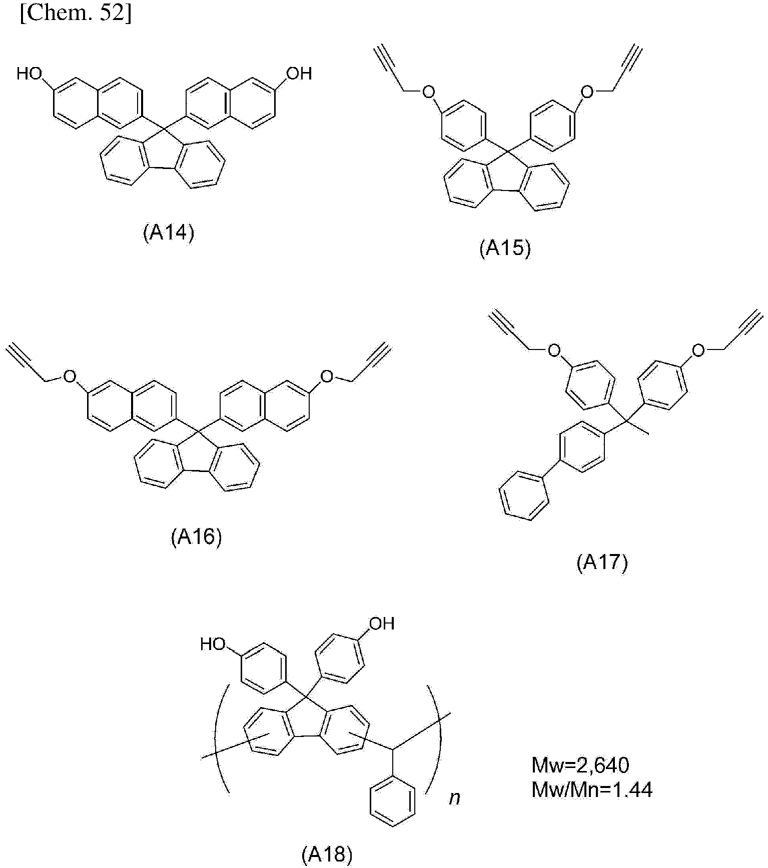

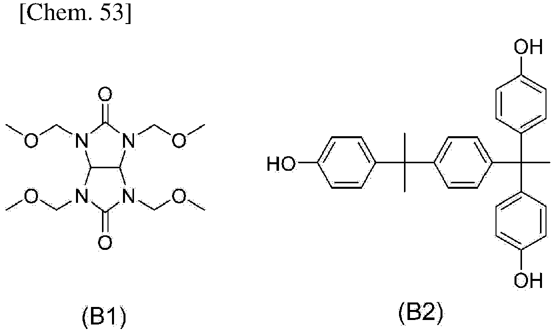

### {Preparation of Evaluation Substrate}

Using a spin coater, each prepared evaluation composition was applied to a silicon wafer substrate such that the film thickness after being heated would become 200 nm. The coated substrate was heated for 60 seconds on a hot plate set to 170°C, and was further heated for 60 seconds on a hot plate set to 300°C, to obtain an evaluation substrate.

This evaluation substrate was used to conduct the various evaluations described below. The evaluation results are collectively shown in Tables 1 to 3.

### {Heat Resistance Evaluation}

Using a spectroscopic ellipsometer SE-2000 from Semilab Inc., the film thickness at five points on the evaluation substrate was measured, and the average value thereof was found as the pre-test film thickness.

The evaluation substrate was heated at 300°C for 60 seconds, and the film thickness after being heated was measured in the same manner as above, to thereby find the post-test film thickness.

Cases in which the rate of change in film thickness before and after the test was below 5% were rated as "A", cases in which the rate of change was 5% or higher and below 10% were rated as "B", and cases in which the rate of change was 10% or higher were rated as "C".

A material having a smaller change in film thickness before and after the test can be used more preferably as a material for forming a film for semiconductors.

### {Solvent Resistance Evaluation}

Using a spectroscopic ellipsometer SE-2000 from Semilab Inc., the film thickness at five points on the evaluation substrate was measured, and the average value thereof was found as the pre-test film thickness.

The evaluation substrate was immersed in PGMEA at 25°C for 60 seconds and was then heated at 170°C for 60 seconds, and then the film thickness after evaporation of PGMEA was measured in the same manner as above, to thereby find the post-test film thickness.

Cases in which the rate of change in film thickness before and after the test was below 1% were rated as "A", cases in which the rate of change was 1% or higher and below 3% were rated as "B", and cases in which the rate of change was 3% or higher were rated as "C".

A material having a smaller change in film thickness before and after the test can be used more preferably as a material for forming a film for semiconductors.

### {Dry-Etching Test}

Using a spectroscopic ellipsometer SE-2000 from Semilab Inc., the film thickness at five points on the evaluation substrate was measured, and the average value thereof was found as the pre-test film thickness.

An etching device (CE-300I from ULVAC, Inc.) was used, and the film thickness after etching was measured in the same manner as above. Etching was conducted according to the following conditions. The film thickness after being etched for 15 seconds, 30 seconds, 60 seconds, and 120 seconds was measured respectively, to calculate the film thickness etched per second (etching rate). The etching conditions were as follows.
Ar gas flow rate: 44 ml/minute.
CF₄ gas flow rate: 11 ml/minute.
Chamber pressure: 4.0 Pa.
RF power: 100 W.

Defining the etching rate of Comparative Example 2 (Compound (A15)) as 100, cases in which the etching rate was below 90 were rated as "A", cases in which the etching rate was 90 or higher and below 100 were rated as "B", and cases in which the etching rate was 100 or higher were rated as "C".

A material having a smaller etching rate has higher etching resistance and can thus be used more preferably as a material for forming a film for semiconductors, and can be used particularly preferably as an underlayer film-forming material.

### {Embeddability; Planarizing Properties}

Each composition was applied with a spin coater onto a SiO₂ stepped substrate (with walls 500 nm wide and 100 nm high, and trenches 500 nm wide). The substrate was heated at 170°C for 60 seconds and at 300°C for 60 seconds, to produce a film. The spin coating conditions were adjusted such that the film thickness would become 200 nm from the trench.

Evaluation of embeddability: A cutout segment of each substrate was produced. Segments in which the steps were completely filled, with no void, when observed with an SEM (S-4800 from Hitachi High-Tech Corporation) were rated as "A", and segments in which voids were observed were rated as "B".

Evaluation of planarizing properties: Planarizing properties were evaluated from the difference (film thickness difference) between the thickest portion of the film produced on the wall on the substrate and the thinnest portion of the film produced on the trench. Cases in which the difference in film thickness was less than 10 nm were rated as "A", cases in which the difference was 10 nm or greater and less than 30 nm were rated as "B", and cases in which the difference was 30 nm or greater were rated as "C".

A material having a smaller difference in film thickness has better planarizing properties and can thus be used more preferably as a material for forming a film for semiconductors, and can be used particularly preferably as an underlayer film-forming material.

**[Table 1]**

| | | Examp le | | | | | | | | | | | | |
|---|---|---|---|---|---|---|---|---|---|---|---|---|---|---|
| | | 1 | 2 | 3 | 4 | 5 | 6 | 7 | 8 | 9 | 10 | 11 | 12 | 13 |
| Composition | A1 | 5 | | | - | | | - | - | - | - | - | - | - |
| | A2 | - | 5 | - | - | - | - | - | - | - | - | - | - | - |
| | A3 | - | - | 5 | - | - | - | - | - | - | - | - | - | - |
| | A4 | - | - | - | 5 | - | - | - | - | - | - | - | - | - |
| | A5 | - | - | - | - | 5 | - | - | - | - | - | - | - | - |
| | A6 | - | - | - | - | - | 5 | - | - | - | - | - | - | - |
| | A7 | - | - | - | - | - | - | 5 | - | - | - | - | - | - |
| | A8 | - | - | - | - | - | - | - | 5 | - | - | - | - | - |
| | A9 | - | - | - | - | - | - | - | - | 5 | - | - | - | - |
| | A10 | - | - | - | - | - | - | - | - | - | 5 | - | - | - |
| | A11 | - | - | - | - | - | - | - | - | - | - | 5 | - | - |
| | A12 | - | - | - | - | - | - | - | - | - | - | - | 5 | - |
| | A13 | - | - | - | - | - | - | - | - | - | - | - | - | 5 |
| | A14 | - | - | - | - | - | - | - | - | - | - | - | - | - |
| | A15 | - | - | - | - | - | - | - | - | - | - | - | - | - |
| | A16 | - | - | - | - | - | - | - | - | - | - | - | - | - |
| | A17 | - | - | - | - | - | - | - | - | - | - | - | - | - |
| | A18 | - | - | - | - | - | - | - | - | - | - | - | - | - |
| | B1 | - | - | - | - | - | - | - | - | - | - | - | - | - |
| | B2 | - | - | - | - | - | - | - | - | - | - | - | - | - |
| | C1 | - | - | - | - | - | - | - | - | - | - | - | - | - |
| | D1 | 47.5 | 47.5 | 47.5 | 47.5 | 47.5 | 47.5 | 47.5 | 47.5 | 47.5 | 47.5 | 47.5 | 47.5 | 47.5 |
| | D2 | 47.5 | 47.5 | 47.5 | 47.5 | 47.5 | 47.5 | 47.5 | 47.5 | 47.5 | 47.5 | 47.5 | 47.5 | 47.5 |
| | | | | | | | | | | | | | | |
| Heat resistance | | B | B | A | A | A | A | A | A | A | B | B | A | A |
| Solvent resistance | | A | A | A | A | A | A | A | A | A | A | A | A | A |
| Dry-etchingtest | | A | A | A | A | A | A | A | A | A | A | A | A | A |
| Embeddability | | A | A | A | A | A | A | A | A | A | A | A | A | A |
| Planarizing properties | | A | A | A | A | A | A | A | A | A | A | A | A | A |

**Table 2]**

| | | Example | | | | | | | | | |
|---|---|---|---|---|---|---|---|---|---|---|---|
| | | 14 | 15 | 16 | 17 | 18 | 19 | 20 | 21 | 22 | 23 |
| Composition | A1 | 4.5 | - | - | - | - | - | - | - | - | - |
| | A2 | - | 4.5 | 4.5 | - | - | - | - | - | - | - |
| | A3 | - | - | - | - | - | 4.5 | - | - | - | 3 |
| | A4 | - | - | - | - | - | - | 3 | - | - | - |
| | A5 | - | - | - | - | - | - | - | 1.5 | - | - |
| | A6 | - | - | - | - | - | - | - | - | - | - |
| | A7 | - | - | - | - | - | - | - | - | - | - |
| | A8 | - | - | - | - | - | - | - | - | - | - |
| | A9 | - | - | - | - | - | - | - | - | - | - |
| | A10 | - | - | - | 4.5 | - | - | - | - | - | - |
| | A11 | - | - | - | - | 4.5 | - | - | - | - | - |
| | A12 | - | - | - | - | - | - | - | 3 | 3 | - |
| | A13 | - | - | - | - | - | - | - | - | - | - |
| | A14 | - | - | - | - | - | - | - | - | - | |
| | A15 | - | - | - | - | - | - | - | - | - | - |
| | A16 | - | - | - | - | - | - | 1.5 | - | 1.5 | - |
| | A17 | - | - | - | - | - | - | - | - | - | - |
| | A18 | - | - | - | - | - | - | - | - | - | 1.5 |
| | B1 | 0.4 | 0.4 | - | 0.4 | 0.4 | 0.4 | - | - | - | - |
| | B2 | - | - | 0.4 | - | - | - | - | - | - | - |
| | Cl | 0.1 | 0.1 | 0.1 | 0.1 | 0.1 | 0.1 | - | - | - | - |
| | D1 | 47.5 | 47.5 | 47.5 | 47.5 | 47.5 | 47.5 | 47.5 | 47.5 | 47.5 | 47.5 |
| | D2 | 47.5 | 47.5 | 47.5 | 47.5 | 47.5 | 47.5 | 47.5 | 47.5 | 47.5 | 47.5 |
| | | | | | | | | | | | |
| Heat resistance | | A | A | A | A | A | A | A | A | A | A |
| Solvent resistance | | A | A | A | A | A | A | A | A | A | A |
| Dry-etching test | | A | A | A | A | A | A | A | A | A | A |
| Embeddability | | A | A | A | A | A | A | A | A | A | A |
| Planarizing properties | | A | A | A | A | A | A | A | A | A | A |

**Table 3]**

| | | Comparative Example | | | | | |
|---|---|---|---|---|---|---|---|
| | | 1 | 2 | 3 | 4 | 5 | 6 |
| Composition | A1 | - | - | - | - | - | - |
| | A2 | - | - | - | - | - | - |
| | A3 | - | - | - | - | - | - |
| | A4 | - | - | - | - | - | - |
| | A5 | - | - | - | - | - | - |
| | A6 | - | - | - | - | - | - |
| | A7 | - | - | - | - | - | - |
| | A8 | - | - | - | - | - | - |
| | A9 | - | - | - | - | - | - |
| | A10 | - | - | - | - | - | - |
| | A11 | - | - | - | - | - | - |
| | A12 | - | - | - | - | - | - |
| | A13 | - | - | - | - | - | - |
| | A14 | 5 | - | - | - | - | 4.5 |
| | A15 | - | 5 | - | - | - | - |
| | A16 | - | - | 5 | - | - | - |
| | A17 | - | - | - | 5 | - | - |
| | A18 | - | - | - | - | 5 | - |
| | B1 | - | - | - | - | - | 0.4 |
| | B2 | - | - | - | - | - | - |
| | Cl | - | - | - | - | - | 0.1 |
| | D1 | 47.5 | 47.5 | 47.5 | 47.5 | 47.5 | 47.5 |
| | D2 | 47.5 | 47.5 | 47.5 | 47.5 | 47.5 | 47.5 |
| | | | | | | | |
| Heat resistance | | C | B | B | C | C | B |
| Solvent resistance | | B | C | B | A | C | A |
| Dry-etching test | | C | C | C | C | C | C |
| Embeddability | | A | A | A | A | A | A |
| Planarizing properties | | B | A | A | C | B | B |

### {Production Example 14}

A reflux condenser-equipped flask was charged with 15.0 g (26 mmol) of Compound (M6) and 450 g of o-xylene, and while stirring, 7.8 g (31 mmol) of iodine was added, and reaction was conducted at 140°C for 5 hours.

The reaction liquid was cooled to room temperature, and 120 g of 10% sodium thiosulfate aqueous solution was dropped, and the mixture was stirred for 30 minutes. The precipitate was filtered, and then the filtered residue was washed with 50 g of o-xylene, and further washed twice with 100 g of a mixed solvent of water and methanol (1:2 in weight ratio). The filtered residue was dried at 40°C under reduced pressure, to obtain Compound (M7) in the form of a gray powder.

Yield amount: 13.3 g (yield rate: 89%).

¹H-NMR (THF-d₈): δ (ppm) = 4.12 (s, 4H), 6.77-8.19 (m, 22H), 9.72 (s, 2H).

A reflux condenser-equipped reaction flask was charged with 5.0 g (9.0 mmol) of Compound (M7) and 250 g of dimethylsulfoxide (DMSO), and while stirring, 9.0 g (77 mmol) of 48% potassium hydroxide aqueous solution was dropped at room temperature under a flow of nitrogen gas. Next, 9.2 g (77 mmol) of 3-bromo-1-propyne was dropped, and stirring was conducted as-is at room temperature for 1 hour. The reaction liquid was placed into 250 g of water and stirred for 30 minutes, which was then filtered. To the filtrate, 250 g of water was added and stirred for 30 minutes, which was then filtered. Thereafter, the filtered residue was washed twice with 100 g of a mixed solvent of water and methanol (1:2 in weight ratio). The residue was dried at 40°C under reduced pressure, to obtain Compound (A19) in the form of a yellow powder.

Yield amount: 2.2 g (yield rate: 44%).

¹H-NMR (THF-d₈): δ (ppm) = 2.51 (d, 8H), 2.63 (t, 4H), 3.05 (t, 2H), 4.67 (d, 4H), 6.76-8.19 (m, 22H).

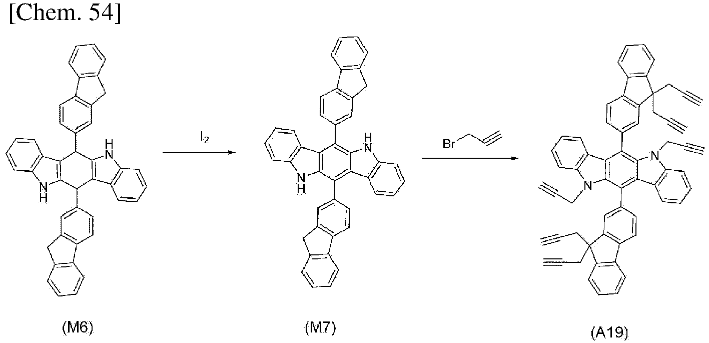

### {Production Example 15}

A reflux condenser-equipped flask was charged with 4.0 g (30 mmol) of 5-hydroxyindole, 5.8 g (30 mmol) of 2-fluorene carboxaldehyde and 30 g of acetonitrile. While stirring, 0.51 g (3 mmol) of 48% hydrobromic acid was dropped at room temperature, and stirring was conducted for 2 hours. To the reaction liquid, 10 g of water was added and stirred at room temperature for 30 minutes, which was followed by filtering. The filtered residue collected was washed three times with 10 g of methanol, and the residue was dried at 40°C under reduced pressure, to obtain Compound (M8) in the form of a greenish gray powder.

Yield amount: 5.2 g (yield rate: 56%).

¹H-NMR (DMSO-d₆): δ (ppm) = 4.12 (s, 4H), 5.66 (s, 2H), 6.13-8.29 (m, 20H), 10.09 (s, 2H), 10.33 (s, 2H).

A reflux condenser-equipped flask was charged with 4.5 g (7 mmol) of Compound (M8) and 90 g of acetonitrile, and while stirring, 2.2 g (9 mmol) of chloranil was added at room temperature. After stirring at room temperature for 30 minutes, the temperature was raised to 70°C, and heating and stirring were conducted as-is for 7 hours. The reaction liquid was cooled to room temperature and was then filtered. The filtered residue collected was washed six times with 30 g of o-xylene, and then the residue was dried at 40°C under reduced pressure, to obtain Compound (M9) in the form of a gray powder.

Yield amount: 4.5 g (yield rate: 100%).

¹H-NMR (DMSO-d₆): δ (ppm) = 4.12 (s, 4H), 6.43-8.24 (m, 20H), 10.09 (s, 2H), 10.33 (s, 2H).

A reflux condenser-equipped flask was charged with 4.0 g (6 mmol) of Compound (M9) and 60 g of dimethylsulfoxide (DMSO), and while stirring, 4.3 g (52 mmol) of 48% potassium hydroxide aqueous solution was added at room temperature under a flow of nitrogen gas. Next, 6.2 g (52 mmol) of 3-bromo-1-propene was added, and stirring was conducted as-is at room temperature for 1 hour. To the reaction liquid, 80 g of water was dropped, and stirring was conducted for 30 minutes, and then the precipitate was filtered. The filtered residue collected was washed with 10 g of a mixed solvent of methanol and water (1:1 in ratio), and the residue was dried at 40°C under reduced pressure, to obtain Compound (A20) in the form of a reddish brown powder.

Yield amount: 3.7 g (yield rate: 61%).

¹H-NMR (DMSO-d₆): δ (ppm) = 2.55 (d, 8H), 2.73 (t, 4H), 3.05 (t, 2H), 3.38 (t, 2H), 4.65 (d, 4H), 4.74 (d, 4H), 6.74-8.26 (m, 20H).

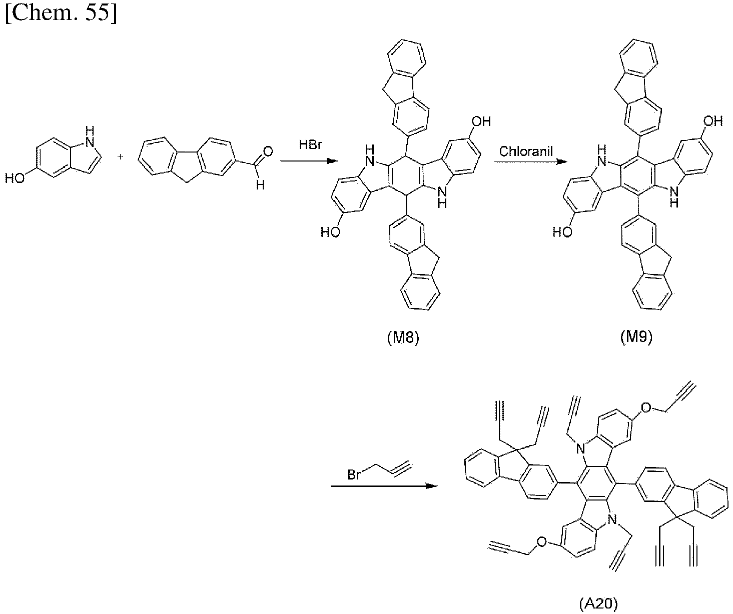

### {Production Example 16}

A reflux condenser-equipped flask was charged with 75.0 g (510 mmol) of 5-methoxyindole, 69.3 g (510 mmol) of 3-methoxybenzaldehyde and 270 g of acetonitrile. While stirring, 8.59 g (51 mmol) of 48% hydrobromic acid was dropped at room temperature, and stirring was conducted for 2 hours. The reaction liquid was cooled and stirred for 30 minutes, and was then filtered. The filtered residue collected was washed three times with 20 g of acetonitrile, and the residue was dried at 40°C under reduced pressure, to obtain Compound (M10) in the form of a gray powder.

Yield amount: 33.1 g (yield rate: 25%).

A reflux condenser-equipped flask was charged with 45.0 g (85 mmol) of Compound (M10) and 134 g of dimethylformamide, and while stirring, 21.9 g (89 mmol) of chloranil was added at room temperature. After stirring at room temperature for 30 minutes, the temperature was raised to 100°C, and heating and stirring were conducted as-is for 2 hours. The reaction liquid was cooled to room temperature, and 202 g of methanol was added thereto and stirred for 30 minutes, which was then filtered. The filtered residue collected was washed three times with 20 g of acetonitrile, followed by washing with 120 g of a mixed solvent of tetrahydrofuran, methanol, and water (1:1:1 in ratio), and then the residue was dried at 40°C under reduced pressure, to obtain Compound (M11) in the form of a greenish brown powder.

Yield amount: 32.1 g (yield rate: 72%).

A reflux condenser-equipped flask was charged with 25.0 g (47 mmol) of Compound (M11), 224 g of acetic acid and 95.7 g (568 mmol) of 48% hydrobromic acid. Stirring was conducted at room temperature for 30 minutes, and then the temperature was raised to 120°C, and heating and stirring were conducted as-is for 48 hours. The reaction liquid was cooled to room temperature, and 202 g of water was added and stirred for 30 minutes, which was then filtered. The filtered residue collected was dissolved in 56 g of acetonitrile, and after removing unnecessary matter, the filtrate was dried at 40°C under reduced pressure, to obtain a solid. The solid was washed with 116 g of ethyl acetate and then washed with 17 g of a mixed solvent of methanol and water (1:1 in ratio), and thereafter, the solid was dried at 40°C under reduced pressure, to obtain Compound (M12) in the form of a greenish brown powder.

Yield amount: 4.3 g (yield rate: 20%).

A reflux condenser-equipped flask was charged with 2.0 g (4.2 mmol) of Compound (M12) and 15 g of dimethylformamide, and while stirring, 2.2 g (26 mmol) of 48% sodium hydroxide aqueous solution was added at room temperature under a flow of nitrogen gas. Next, 3.2 g (26 mmol) of 3-bromo-1-propene was added, and stirring was conducted as-is at room temperature for 1 hour. Then, a mixed solvent including 15 g of methanol and 15 g of water was dropped to the reaction liquid and was stirred for 30 minutes, and then the precipitate was filtered. The filtered residue collected was washed with 12 g of a mixed solvent of methanol and water (1:1 in ratio), and then the residue was dried at 40°C under reduced pressure, to obtain Compound (A21) in the form of a yellowish brown powder.

Yield amount: 2.0 g (yield rate: 69%).

¹H-NMR (DMSO-d₆): δ (ppm) = 3.07 (t, 2H), 3.43 (t, 2H), 3.53 (t, 2H), 4.50 (d, 4H), 4.62 (d, 4H), 4.90 (d, 4H), 6.16 (d, 2H), 7.09-7.69 (m, 12H).

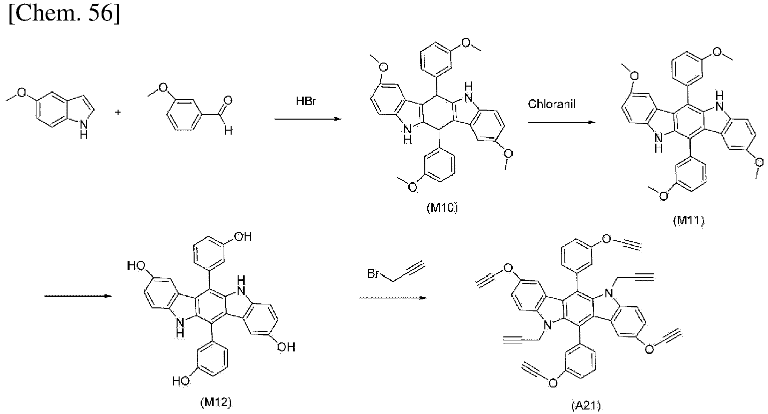

### {Production Example 17}

A reflux condenser-equipped reaction flask was charged with 2.28 g of Compound (A19), 0.30 g of benzaldehyde and 30 g of N-methylpyrrolidone, and the temperature was raised to 50°C under a flow of nitrogen gas. Then, 0.18 g of methanesulfonic acid was dropped slowly and the temperature was further raised, and stirring was conducted at 80°C for 8 hours. After cooling the mixture to room temperature and removing insoluble matter, the reaction liquid was dropped into 150 g of a mixed solvent of n-hexane and 2-propanol (2:1 in ratio) and was stirred for 30 minutes. The precipitate was filtered and dried at 60°C under reduced pressure, to obtain 1.3 g of a brown powder of Polymer (A22). As regards the molecular weight (in terms of polystyrene) of the obtained powder as measured by gel permeation chromatography (GPC), the weight-average molecular weight (Mw) was 2,490 and the dispersity was 1.17.

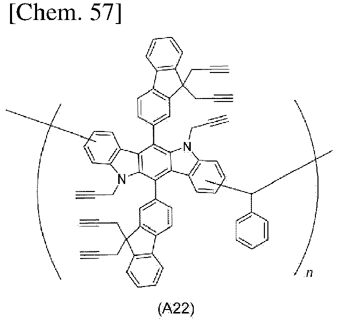

### {Examples 24 to 27}

According to the respective makeup (parts by mass) shown in Table 4, components were measured and mixed, and were then stirred to dissolve. After confirming that the solid had dissolved completely, the mixture was filtered with a fluorocarbon resin-made filter (pore size: 0.2 µm), to prepare a composition for evaluation.

The components in the Table are as follows.
A19: Compound (A19); Compound (I).
A20: Compound (A20); Compound (I).
A21: Compound (A21); Compound (I).
A22: Polymer (A22); Polymer (I) and Polymer (II).
D1: Propylene glycol monomethyl ether acetate (PGMEA); solvent.
D2: Cyclohexanone; solvent.

Using the prepared evaluation compositions, various evaluations were conducted as in Example 1. The results are collectively shown in Table 4.

**Table 4**

| | | Example | | | |
|---|---|---|---|---|---|
| | | 24 | 25 | 26 | 27 |
| Composition | A19 | 5 | - | - | - |
| | A20 | - | 5 | - | - |
| | A21 | - | - | 5 | - |
| | A22 | - | - | - | 5 |
| | D1 | 47.5 | 47.5 | 47.5 | 47.5 |
| | D2 | 47.5 | 47.5 | 47.5 | 47.5 |
| | | | | | |
| Heat resistance | | A | A | A | A |
| Solvent resistance | | A | A | A | A |
| Dry-etching test | | A | A | A | A |
| Embeddability | | A | A | A | A |
| Planarizing properties | | A | A | A | A |

As shown in Tables 1 to 4, the materials for forming films for semiconductors according to the present invention, which employed Compound (I) or Polymer (I), were capable of obtaining films having excellent heat resistance and solvent resistance, compared to materials of the Comparative Examples which used neither Compound (I) nor Polymer (I). Further, the materials for forming films for semiconductors according to the present invention were capable of obtaining films having excellent dry-etching properties, and also had excellent embeddability and planarizing properties at the time of film formation.

## Claims

1. A material for forming a film for a semiconductor, the material comprising:
a compound represented by general formula (I) below and having in its molecule at least one reactive group, or a polymer including, as a monomer, a compound represented by general formula (I) below and having in its molecule at least one reactive group; and
a solvent: 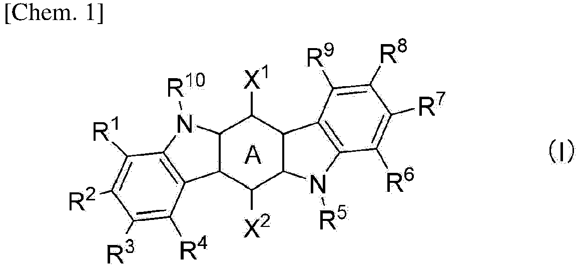
wherein, in the formula, A represents a hydrocarbon ring having 6 carbon atoms;
X¹ and X² each independently represent an aryl group having 6 to 30 carbon atoms and optionally substituted by a reactive group or a group having a reactive group, a heterocyclic group having 2 to 30 carbon atoms and optionally substituted by a reactive group or a group having a reactive group, or a heterocycle-containing group having 3 to 30 carbon atoms and optionally substituted by a reactive group or a group having a reactive group;
R¹, R², R³, R⁴, R⁶, R⁷, R⁸, and R⁹ each independently represent a hydrogen atom, a halogen atom, a reactive group, a nitro group, a hydrocarbon group having 1 to 20 carbon atoms and optionally substituted by a reactive group, a heterocyclic group having 2 to 10 carbon atoms and optionally substituted by a reactive group, a heterocycle-containing group having 3 to 30 carbon atoms and optionally substituted by a reactive group, a group in which at least one methylene group in said hydrocarbon group having 1 to 20 carbon atoms has been substituted by a divalent group selected from {Group A} below, or a group in which at least one methylene group in said heterocycle-containing group having 3 to 30 carbon atoms has been substituted by a divalent group selected from {Group A} below; and
R⁵ and R¹⁰ each independently represent a hydrogen atom, a hydrocarbon group having 1 to 20 carbon atoms and optionally substituted by a reactive group, a heterocyclic group having 2 to 10 carbon atoms and optionally substituted by a reactive group, a heterocycle-containing group having 3 to 30 carbon atoms and optionally substituted by a reactive group, a group in which at least one methylene group in said hydrocarbon group having 1 to 20 carbon atoms has been substituted by a divalent group selected from {Group A} below, or a group in which at least one methylene group in said heterocycle-containing group having 3 to 30 carbon atoms has been substituted by a divalent group selected from {Group A} below:
{Group A}: -O-, -CO-, -COO-, -OCO-, -NR¹¹-, -NR¹²CO-, -S-,
wherein R¹¹ and R¹² each independently represent a hydrogen atom or a hydrocarbon group having 1 to 20 carbon atoms.

2. The material for forming a film for a semiconductor according to claim 1, wherein
X¹ and X² in general formula (I) are each
a phenyl group optionally substituted by a reactive group or a group having a reactive group,
a hydrocarbon-type aromatic fused ring group having 7 to 30 carbon atoms and optionally substituted by a reactive group or a group having a reactive group, or a heterocycle-containing fused ring group having 3 to 30 carbon atoms and optionally substituted by a reactive group or a group having a reactive group.

3. The material for forming a film for a semiconductor according to claim 1, wherein R⁵ and R¹⁰ in general formula (I) are each a hydrocarbon group having 1 to 20 carbon atoms and substituted by a reactive group.

4. The material for forming a film for a semiconductor according to claim 1, wherein the reactive group is a carbon-carbon triple bond or a phenolic hydroxy group.

5. The material for forming a film for a semiconductor according to claim 1, wherein the compound represented by general formula (I) has at least two reactive groups in its molecule.

6. The material for forming a film for a semiconductor according to claim 1, wherein the compound represented by general formula (I) is a compound represented by general formula (Ia), (Ib), or (Ic) below: 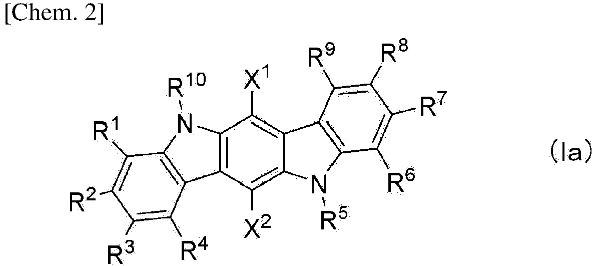 the symbols in the formula are the same as those in said general formula (I); 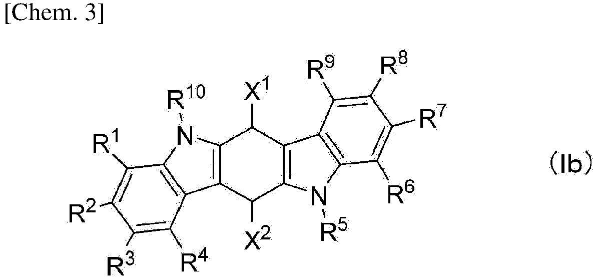 the symbols in the formula are the same as those in said general formula (I); 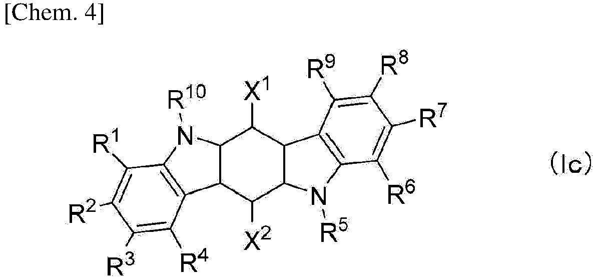 the symbols in the formula are the same as those in said general formula (I).

7. The material for forming a film for a semiconductor according to claim 1, comprising a cross-linking agent.

8. The material for forming a film for a semiconductor according to claim 1, comprising a polymerization initiator.

9. A material for forming a member for a semiconductor, comprising the material for forming a film for a semiconductor according to any one of claims 1 to 8.

10. A material for forming a member for a semiconductor process, comprising the material for forming a film for a semiconductor according to any one of claims 1 to 8.

11. An underlayer film-forming material comprising the material for forming a film for a semiconductor according to any one of claims 1 to 8.

12. An underlayer film formed by using the underlayer film-forming material according to claim 11.

13. A semiconductor device manufactured by using the material for forming a film for a semiconductor according to any one of claims 1 to 8.
